# EUROPÄISCHE PATENTSCHRIFT

(11) **EP 1 891 035 B1**
(45) Veröffentlichungstag und Bekanntmachung des Hinweises auf die Patenterteilung: **27.04.2011**
(21) Anmeldenummer: 06753681.3
(22) Anmeldetag: 17.05.2006
(51) Int. Cl.: C07D 337/08, C07C 271/24, C07D 313/08, A61K 31/27, A61K 31/335, A61K 31/38, A61P 25/04

(54) **SUBSTITUIERTE BENZOKONDENSIERTE CYCLOHEPTANON-DERIVATE UND DEREN VERWENDUNG ZUR HERSTELLUNG VON ARZNEIMITTELN**
SUBSTITUTED BENZO-CONDENSED CYCLOHEPTANONE DERIVATIVES AND THEIR USE FOR PRODUCING DRUGS
DERIVES DE CYCLOHEPTANONE BENZOCONDENSES SUBSTITUES, ET LEUR UTILISATION POUR PRODUIRE DES MEDICAMENTS

(30) Priorität: 20.05.2005 DE 102005023944
(43) Veröffentlichungstag der Anmeldung: 27.02.2008
(73) Patentinhaber: Grünenthal GmbH, 52078 Aachen (DE)
(72) Erfinder: HINZE, Claudia, 52066 Aachen (DE); FRANK, Robert, 52070 Aachen (DE); JOSTOCK, Ruth, 52223 Stolberg (DE); SCHIENE, Klaus, 40227 Düsseldorf (DE); HAURAND, Michael, 52078 Aachen (DE); HENNIES, Hagen-Heinrich, 52152 Simmerath (DE)
(74) Vertreter: Brosch, Oliver
(86) Internationale Anmeldenummer: PCT/EP2006/004665
(87) Internationale Veröffentlichungsnummer: WO 2006/122776

(56) Entgegenhaltungen:
- WO-A-2004/052845
- WO-A-2005/044802
- US-B1- 6 384 072
- B. EISTERT ET. AL.: "Weitere Umsetzungen von Phenanthrenchinon mit aliphatichen Diazoverbindungen." CHEMISCHE BERICHTE, Bd. 101, 1968, Seiten 84-93, XP009071826
- B. D. ASTILL ET. AL.: "The Synthesis of 1-Benzazepine Derivatives as Model Compounds Related to Apo-beta-Erythriodine." JOURNAL OF THE AMERICAN CHEMICAL SOCIETY, Bd. 77, 1955, Seiten 4079-4084, XP002397577
- C. S. J. WALPOLE ET. AL.: "The Discovery of Capsazepine, the First Competitive Antagonist of the Sensory Neuron Excitants Capsaicin and Resiniferatoxin." JOURNAL OF MEDICINAL CHEMISTRY, Bd. 37, 1994, Seiten 1942-1954, XP002397578

## Beschreibung

Die vorliegende Erfindung betrifft substituierte benzokondensierte Cycloheptanon-Derivate, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung- Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufrieden stellende Schmerzbehandlung für den Patienten zu verstehen ist, dokumentiert sich auch in der großen Anzahl von wissenschäftlichen Arbeiten, die auf dem Gebiet der angewandten Analgetik bzw. der Grundlagenforschung zur Nociception in letzter Zeit erschienen sind.

Einen geeigneten Ansatzpunkt zur Behandlung von Schmerz, insbesondere von neuropathischem Schmerz, stellt der Vanilloid-Rezeptor vom Subtyp 1 (VR1/TRPV1) dar, der häufig auch als Capsaicin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen;-Angstzuständen; Epilepsie; Husten; Diarrhöe; Pruritus: Störungen des kardiovaskulären Systems; Störungen der Nahrungsaufnahme; Medikamentenabhängigkeit; Medikamentenmißbrauch und insbesondere Harninkontinenz.

Aus der Veröffentlichung von Klein et al. in Chemische Berichte, Band 101, Seiten 84-93 (1968) sind Umsetzungen von Phenanthrenchinon mit aliphatischen Diazoverbindungen bekannt. Die Veröffentlichung von D. Astill und V. Boekelheide offenbart die Synthese von 1-Benzazepin-Derivaten (Journal of the American Chemical Society, Seiten 4079-84, 1955). US 6,384,072 B1 offenbart Benzocyclohepten-Derivate, W02004/052845 Tetrahydro-naphthalin-Derivate als Vanilloid Rezeptor Antagonisten und WO 2005/044802 Tetrahydrochinolinhamstoff-Derivate. Aus der Veröffentlichung von Walpole et al. sind ferner Capsaicin-Analoga bekannt (J. Med. Chem. 1994, 37, 1942-1954).

Eine Aufgabe der vorliegenden Erfindung bestand daher darin, neue Verbindungen zur Verfügung zu stellen die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass sich substituierte benzokondensierte Cycloheptanon-Derivate der nachstehend angegebenen allgemeinen Formel I zur Bekämpfung von Schmerzen eignen und eine ausgezeichnete Affinität zum Vanilloid-Rezeptor vom Subtyp 1 (VR1/TRPV1-Rezeptor) aufweisen. Darüber hinaus zeigen diese erfindungsgemäßen benzokondensierten Cycloheptanon-Derivate auch eine hohe Affinität für Cannabinoid-Rezeptoren CB1 (CB1-Rezeptoren) und/oder Cannabinoid-Rezeptoren CB2(CB2-Rezeptoren) aufweisen. Die erfindungsgemäßen benzokondensierten Cylcoheptanon-Verbindungen eignen sich daher insbesondere zur Prophylaxe und/oder Behandlung von Störungen oder Krankheiten, die zumindest teilweise durch Vanilloid-Rezeptoren 1 (VR1/TRPV1) und/oder Cannabinoid-Rezeptoren CB1 (CB1-Rezeptoren) und/oder Cannabinoid-Rezeptoren CB2 (CB2-Rezeptoren) vermittelt werden.

Ein Gegenstand der vorliegenden Erfindung sind daher substituierte benzokondensierte Cycloheptanon-Derivate der allgemeinen Formel I. 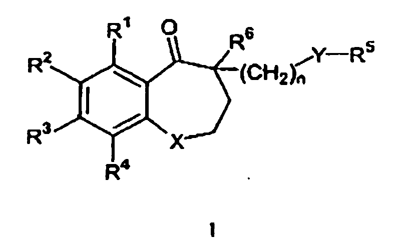 worin
- n: gleich 1, 2 oder 3 ist;
- X: für CH₂, O, S, S(=O), S(=O)₂, N(H). N(R⁷), N[C(=O)-R⁸] oder N[C(=O)-O-R⁹] steht;
- Y: für O, O-C(=O), O-C(=O)-O, O-S(=O)₂, O-C(=O)-N(H), O-C(=S)-N(H), N(H)- C(=O)-N(H) oder N(H)-C(=S)-N(H) steht; wobei das Atom, das an den Rest R⁵ bindet, immer zuletzt angegeben ist;
- R¹, R², R³ und R⁴,: unabhängig voneinander, jeweils
für H, F, Cl, Br, I, -SF₅, -CN, -NC, -NO₂, -SO₃H, -NH₂, -OH, -SH, -OR¹⁰, -SR¹¹, -NR¹²R¹³, -NH-R¹⁴, -NH-C(=O)-R¹⁵, -NR¹⁶-C(=O)-R¹⁷, C(=O)-NH₂, -C(=O)-NH-R¹⁸, -C(=O)-NR¹⁹R²⁰, -C(=O)-H, -C(=O)-R²¹, - C(=O)-OH, -C(=O)-OR²², -O-C(=O)-R²³ oder für einen linearen oder verzweigten, gesättigten oder ungesättigten, ggf. substituierten C₁₋₁₀ aliphatischen Rest stehen;
- R⁵: für eine -C(=O)-R²⁴-Gruppe steht;
für eine -S(=O)₂R²⁵-Gruppe steht;
für einen ungesättigten oder gesättigten, ggf. substituierten 3-, 4-, 5-, 6-, 7-, 8- oder 9-gliedrigen cycloaliphatischen Rest;
für einen linearen oder verzweigten, gesättigten oder ungesättigten, ggf. substituierten C₁₋₁₀ aliphatischen Rest;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine -(CH₂)-, -(CH₂)-(CH₂)- oder -(CH₂)-(CH₂)-(CH₂)- Gruppe gebunden sein kann, steht;
- R⁶: für einen Wasserstoff-Rest;
für -(CH₂)ₚ-Z-R²⁶ mit p = 1, 2 oder 3;
oder für -(CH₂)_{q}-OR²⁷ mit q = 1,2 oder 3 steht;
- R⁷, R⁸ und R⁹,: unabhängig voneinander, jeweils
für einen linearen oder verzweigten, gesättigten oder ungesättigten, ggf. substituierten C₁₋₁₀ aliphatischen Rest;
oder für einen ggf. substituierten 5- bis 14-gliedrigen Aryl- oder Heteroaryl-Rest, der über eine -(CH₂)-, -(CH₂)-(CH₂)- oder -CH₂)- (CH₂)-(CH₂)-Gruppe ist, stehen;
- R¹⁰, R¹¹, R¹² R¹³, R¹⁴, R¹⁵, R¹⁶,:
- R¹⁷, R¹⁸, R¹⁹, R²⁰, R²¹, R²² und R²³,: unabhängig voneinander, jeweils
für einen linearen oder verzweigten, gesättigten oder ungesättigten; ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine -(OH₂)-, -(CH₂)-(CH₂)- oder -(CH₂)-(CH₂)-(CH₂)- Gruppe gebunden sein kann, stehen;
- Z: für O, O-C(=O), O-C(=O)-O, O-S(=O)₂, O-C(=O)-N(H), O-C(=S)-N(H), N(H), N(H)-C(=O)-N(H) oder N(H)-C(=S)-N(H) steht; wobei das Atom, das an den Rest R²⁶ bindet, immer zuletzt angegeben ist;
- R²⁴, R²⁵, R²⁸ und R²⁹,: unabhängig voneinander, jeweils für für einen ungesättigten oder gesättigten, ggf. substituierten 3-, 4-, 5-, 6-, 7-, 8- oder 9-gliedrigen cycloaliphatischen Rest;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
- R²⁶: für eine -C(=O)-R²⁸-Gruppe steht;
für eine -S(=O)₂-R²⁹-Gruppe steht;
für einen ungesättigten oder gesättigten, ggf. substituierten 3-, 4-, 5-, 6-, 7-, 8- oder 9-gliedrigen cycloaliphatischen Rest;
für einen linearen oder verzweigten, gesättigten oder ungesättigten, ggf. substituierten C₁₋₁₀ aliphatischen Rest;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine -(CH₂)-, -(CH₂)-(CH₂)- oder -(CH₂)-(CH₂)-(CH₂)- Gruppe gebunden sein kann, steht;
und
- R²⁷: für einen Wasserstoff-Rest steht;
wobei
die vorstehend genannten C₁₋₁₀ aliphatischen Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -SH und -NH₂ substituiert sein können;
die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₁₀-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann,
und die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
die vorstehend genannten cycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, - O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, - C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Senzo[b]furanyl und Benzyl mit 1, 2, 3, 4 oder 5 Substituenten unabhängig voneinander ausgewählt aus der Gruppe bestehend aus F, Cl, Br, -OH, - CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt können benzokondensierte Cycloheptanon-Derivate der allgemeinen Formel I ausgenommen sein, in denen X für CH₂ steht; R⁶ für einen Wasserstoff-Rest oder für --(CH₂)ₚ-Z-R²⁶ mit p = 1, 2 oder 3 steht; Z für O oder O-C(=O) steht; R²⁶ für einen linearen oder verzweigten, gesättigten oder ungesättigten, unsubstituierten oder wenigstens einfach substituierten aliphatischen Rest steht; n gleich 1, 2 oder 3 ist; Y für O oder O-C(=O) steht; R⁵ für einen linearen oder verzweigten, gesättigten oder ungesättigten, unsubstituierten oder wenigstens einfach substituierten aliphatischen Rest steht und R¹, R², R³ und R⁴ jeweils für einen beliebigen der vorstehend genannten Substituenten stehen.

Aliphatische Reste umfassen im Sinne dieser Erfindung azyklische gesättigte oder ungesättigte Kohlenwasserstoffreste, die verzweigt oder geradkettig sowie unsubstituiert oder einfach substituiert oder mehrfach gleich oder verschieden substituiert sein können, mit vorzugsweise 1 bis 6 (d. h. 1, 2, 3, 4, 5 oder 6) Kohlenstoffatomen, d.h C₁₋₆-Alkyle, C₂₋₆-Alkenyle und C₂₋₆-Alkinyle. Dabei weisen Alkenyle mindestens eine C-C-Doppelbindung und Alkinyle mindestens eine C-C-Dreifachbindung auf. Vorteilhafterweise können aliphatische Reste ausgewählt werden aus der Gruppe, die Methyl, Ethyl, n-Propyl, Isopropyl, n-Butyl, Isobutyl, sec-Butyl, tert-Butyl, n-Pentyl, Isopentyl, neo-Pentyl, n-Hexyl, 2-Hexyl, n-Heptyl, n-Octyl, n-Nonyl, n-Decyl. Ethenyl (Vinyl), Ethinyl, Propenyl (-CH₂CH=CH₂, -CH=CH-CH₃, -C(=CH₂)-CH₃), 2-Methyl-propenyl, Propinyl (-CH₂-C≡CH, -C≡C-CH₃), Butenyl, Butinyl, Pentenyl, Pentinyl, Hexenyl, Hexinyl, Octenyl und Octinyl umfaßt.

Im Zusammenhang mit aliphatischen Resten versteht man unter dem Begriff "substituiert" - soweit nicht anders definiert - im Sinne dieser Erfindung die einfache oder mehrfache Substitution, bevorzugt die ein-, zwei-, drei-, vier-, fünf-, sechs-, sieben-, acht- oder neunfache Substitution, von einem oder mehreren Wasserstoffatomen durch F, Cl, Br, I, -CN, -NO₂, -OH, -SH und -NH₂, wobei die mehrfache Substitution entweder an verschiedenen oder an gleichen Atomen mehrfach, z.B. zwei- oder dreifach, erfolgt, beispielsweise dreifach am gleichen C-Atom wie im Falle von -CF₃ oder -CH₂CF₃ oder an verschiedenen Stellen wie im Falle von -CH(OH)-CH=CCl-CH₂Cl. Die Mehrfachsubstitution kann mit dem gleichen oder mit verschiedenen Substituenten erfolgen. Bevorzugte substituierte aliphatische Reste sind -CH₂-Cl, -CH₂-Br, -CH₂-CH₂-Cl, -CH₂-CH₂-Br, -CH₂-CH₂-CH₂-Br, -CH₂-CH₂-CH₂-Cl, -CF₃, -CHF₂, -CH₂F, -CCl₃, -CBr₃, -CH₂-CN, -CH₂-NO₂, -CF₂-CF₃, -CH₂-CF₃, -CCl₂-CCl₃, -CF₂CH₃, -CH₂-CH₂-CN, -CH₂-CH₂-NO₂, -CF₂-CF₂-CF₃, - CH₂-CH₂-CH₂CN und -CH₂-CH₂-CH₂-NO₂.

Unter dem Ausdruck Aryl-Rest ist für die Zwecke der vorliegenden Erfindung vorzugsweise ein Rest zu verstehen, der aus der Gruppe, die Phenyl, Naphthyl, Phenanthrenyl und Anthracenyl umfaßt, ausgewählt ist und unsubstituiert oder einfach oder mehrfach gleich oder verschieden substituiert ist. Bevorzugt ist Aryl ein unsubstituiertes oder einfach substituiertes oder mehrfach, beispielsweise zwei-, drei- vier oder fünffach, gleich oder verschieden substituiertes Phenyl, 1-Naphthyl oder 2-Naphthyl.

Heteroaryl-Reste im Sinne der vorliegende Erfindung sind solche Heterozyklen, die heteroaromatisch sind. Heteroaryl-Reste sind 5- bis 14-gliedrig, d. h. 5-, 6-, 7-, 8-, 9-, 10-, 11-, 12-, 13- oder 14-gliedrig und weisen 1, 2, 3, 4 oder 5 Heteroatome unabhängig voneinander ausgewählt aus der Gruppe umfassend Sauerstoff, Stickstoff und Schwefel auf. Jeder Heteroaryl-Rest kann unsubstituiert oder einfach substituiert oder mehrfach, beispielsweise zwei-, drei-, vier- oder fünffach, gleich oder verschieden substituiert vorliegen.

Beispielhaft für Heteroaryl-Rest im Sinne der vorliegenden Erfindung seien Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl, Benzothiazolyl, Benzo[2,1,3]thiadiazolyl, [1,2,3]-Benzothiadiazolyl, [2,1,3]-Benzoxadiazolyl und [1,2,3]-Benzoxadiazolyl genannt.

In Bezug auf Aryl- und Heteroaryl-Reste versteht man im Sinne dieser Erfindung unter "substituiert" die ein- oder mehrfache, z.B. ein-, zwei-, drei-, vier- oder fünffache, Substitution eines oder mehrerer Wasserstoffatome des Ringsystems durch geeignete Substituenten. Soweit die Bedeutung dieser geeigneten Substituenten im Zusammenhang mit Aryl- oder Heteroaryl-Resten nicht an anderer Stelle der Beschreibung oder in den Ansprüchen definiert ist, sind geeignete Substituenten F, Cl, Br, I, -CN, -CF₃, -SF₅, -OH, -O-C₁₋₁₀-Alkyl, -NH₂, -NO₂, -O-CF₃, - S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, - S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Behzo[b]furanyl, -O-Phenyl, -O-Benzyl, Phenyl und Benzyl, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann.
Die Mehrfachsubstitution erfolgt dabei mit dem gleichen oder mit unterschiedlichen Substituenten.

Cycloaliphatische Reste im Sinne dieser Erfindung sind zyklische gesättigte oder ungesättigte Kohlenwasserstoffreste mit 3, 4, 5, 6, 7, 8, oder 9, bevorzugt 3, 4, 5, 6, 7 oder 8 Kohlenstoffatomen, wobei jeder Rest unsubstituiert oder einfach substituiert oder mehrfach gleich oder verschieden substituiert sein kann. Cycloaliphatische Reste können 1, 2, 3, 4 oder 5 Heteroatome unabhängig voneinander ausgewählt aus der Gruppe bestehend aus Sauerstoff, Stickstoff (NH) und Schwefel aufweisen.

Beispielhaft für cycloaliphatische Rest seien Cyclopropyl, Cyclobutyl, Cyclopentyl, Cyclohexyl. Cyclooctyl, Cyclononyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl genannt.

Unter einem mono- oder polyzyklischen Ringsystem werden im Sinne der vorliegenden Erfindung mono- oder polyzyklische Kohlenwasserstoffreste verstanden, die gesättigt oder ungesättigt sein und ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind. Ein solches mono- bzw. polyzyklisches Ringsystem kann beispielsweise mit einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien [1,3]-Benzodioxolyl, [1,4]-Benzodioxanyl, [1,2,3,4]-Tetrahydronaphthyl, [1,2,3,4]-Tetrahydrochinolinyl, [1,2,3,4]-Tetrahydrochinazolinyl und [3,4]-Dihydro-2H-1,4-benzoxazinyl genannt.

Im Zusammenhang mit cycloaliphatischen Resten und mono- oder polyzyklischen Ringsysteme versteht man unter dem Begriff "substituiert" - soweit nicht anders definiert - im Sinne dieser Erfindung die einfache oder mehrfache Substitution, bevorzugt die ein-, zwei-, drei-, vier-, fünf-, sechs-, sieben-, acht- oder neunfache Substitution, von einem oder mehreren Wasserstoffatomen durch beispielsweise Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, - C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann. Die mehrfache Substitution kann entweder an verschiedenen oder an gleichen Atomen mehrfach, z.B. zwei- oder dreifach, erfolgen. Die Mehrfachsubstitution kann mit dem gleichen oder mit verschiedenen Substituenten erfolgen.

Besonders bevorzugt sind substituierte benzokondensierte Cycloheptanon-Derivate der vorstehend angegebenen allgemeinen Formel I, worin
- n: gleich 1 ist;
- X: für CH₂, O, S, S(=O), S(=O)₂, N(H), N(R⁷), N[C(=O)-R⁸] oder N[C(=O)-O-R⁹] steht;
- Y: für O, O-C(=O), O-C(=O)-O, O-S(=O)₂, O-C(=S)-N(H) oder O-C(=O)-N(H) steht;
- R¹, R², R³ und R⁴,: unabhängig voneinander, jeweils
für H, F, Cl, Br, I, -SF₅ -CN, -NC, -NO₂, -OH, -SH, -OR¹⁰, -SR¹¹, -NR¹²R¹³,
oder für einen Rest ausgewählt aus der Gruppe bestehend aus Methyl, -CF₃, - CHF₂, -CH₂F, -CCl₃, -CBr₃, -CH₂-CN, -CH₂-NO₂, Ethyl, -CF₂-CF₃, -CH₂CF₃, - CCl₂-CCl₃, -CF₂-CH₃, -CH₂-CH₂-CN, -CH₂-CH₂-NO₂, n-Propyl, -CF₂-CF₂-CF₃, - CH₂-CH₂-CH₂-CN, -CH₂-CH₂-CH₂-NO₂, Isopropyl, n-Butyl, sec-Butyl, Isobutyl, tert-Birtyl, (1,1)-Dimethyl-propyl, n-Pentyl, sec-Pentyl, n-Hexyl und n-Heptyl stehen;
- R⁵: für eine -C(=O)-R²⁴-Gruppe steht;
für eine -S(=O)₂-R²⁵-Gruppe steht;
für einen Rest ausgewählt aus der Gruppe bestehend aus Methyl, Ethyl, n- Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl,
Tetrahydrothiophenyl, Pyrrolidinyl, Piperidinyl, Morpholinyl, Piperazinyl und Thiomorpholinyl steht;
oder für einen Rest ausgewählt aus der Gruppe bestehend aus Phenyl, Benzyl, Phenethyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest über eine -(CH₂)-, -(CH₂)-(CH₂)- oder- (CH₂)-(CH₂)-(Ch₂)-Gruppe gebunden und ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O- C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O- C₂H₅, -NH-C(=O)-O-C(CH₃)₃, -C(=O)-H, -C(=O)-CH₃, -C(=O)-C₂H₅, -C(=O)- C(CH₃)₃, -C(=O)-NH₂, -C(=O)-NH-CH₃, =C(=O)-NH-C₂H₅, -C(=O)-N-(CH₃)₂, - C(=O)-N-(C₂H₅)₂, -S(=O)₂-CH₃, -S(=O)-C₂H₅, -NH-S(=O)₂-CH₃, -NH-S(=O)₂- C₂H₅, -S(=O)₂-NH-CH₃ und -S(=O)₂-NH-C₂H₅ substituiert sein kann;
- R⁶: für einen Wasserstoff-Rest;
für -(CH₂)-Z-R²⁶,
oder für -(CH₂)-OR²⁷ steht;
- R⁷, R⁸ und R⁹,: unabhängig voneinander, jeweils
für einen Rest ausgewählt aus der Gruppe bestehend aus Methyl, Ethyl, n- Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen; oder für einen Rest ausgewählt aus der Gruppe bestehend aus Benzyl und Phenethyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl und tert-Butyl substituiert sein kann;
- R¹⁰, R¹¹, R¹² und R¹³,: unabhängig voneinander, jeweils für einen Rest ausgewählt aus der Gruppe bestehend aus Methyl, -CF₃, - CHF₂, -CH₂F, -CCl₃, -CBr₃, -CH₂-CN, -CH₂-NO₂, Ethyl, -CF₂-CF₃, -CH₂-CF₃, - CCl₂-CCl₃, -CF₂-CH₃, -CH₂-CH₂-CN, -CH₂-CH₂-NO₂, n-Propyl, Isopropyl, n- Butyl, sec-Butyl, Isobutyl, tert-Butyl, (1,1)-Dimethyl-propyl, n-Pentyl, sec- Pentyl, n-Hexyl und n-Heptyl stehen; oder für einen Rest ausgewählt aus der Gruppe bestehend aus Phenyl, Benzyl und Phenethyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl und tert-Butyl substituiert sein kann;
- Z: für O, O-C(=O), O-C(=O)-O, O-S(=O)₂, O-C(=S)-N(H) oder O-C(=O)-N(H) steht;
- R²⁴ und R²⁵,: unabhängig voneinander, jeweils für einen Rest ausgewählt aus der Gruppe bestehend aus Phenyl, Naphthyl, Thiophenyl, Furanyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -N(CH₃)₂, -N(C₂H₅)₂, -NH-CH₃ und -NH-C₂H₅ substituiert sein kann;
- R²⁶: für eine -C(=O)-R²⁸-Gruppe steht;
für eine -S(=O)₂-R²⁹-Gruppe steht;
für einen Rest ausgewählt aus der Gruppe bestehend aus Methyl, Ethyl, n- Propyl, Isopropyl, n-Butyl; sec-Butyl, Isobutyl, tert-Butyl, (1,1)-Dimethyl-propyl, n-Pentyl, sec-Pentyl, n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht;
oder für einen Rest ausgewählt aus der Gruppe bestehend aus Phenyl, Benzyl, Phenethyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest über eine -(CH₂)-, -(CH₂)-(CH₂)- oder - (CH₂)-(CH₂)-(CH₂)-Gruppe gebunden und ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=OFO-CH₃, -C(=O)-O- C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O- C₂H₅, -NH-C(=O)-O-C(CH₃)₃, -C(=O)-H, -C(=O)-CH₃, -C(=O)-C₂H₅, -C(=O)- C(CH₃)₃, -C(=O)-NH₂, -C(=O)-NH-CH₃, -C(=O)NH-C₂H₅, -C(=O)-N-(CH₃)₂, - C(=O)N-(C₂H₅)₂, -S(=O)₂-CH₃, -S(=O)₂-C₂H₅, -NH-S(=O)₂-CH₃, -NH-S(=O)₂- C₂H₅, -S(=O)₂-NH-CH₃ und -S(=O)₂-NH-C₂H₅ substituiert sein kann;
- R²⁷: für einen Wasserstoff-Rest steht;
und
- R²⁸ und R²⁹,: unabhängig voneinander, jeweils
für einen Rest ausgewählt aus der Gruppe bestehend aus Phenyl, Naphthyl, Thiophenyl, Furanyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -N(CH₃)₂, -N(C₂H₅)₂, -NH-CH₃ und -NH-C₂H₅ substituiert sein kann;
wobei benzokondensierte Cycloheptanon-Derivate der allgemeinen Formel I ausgenommen sind, worin X für CH₂ steht; R⁶ für einen Wasserstoff-Rest oder für - (CH₂)-Z-R²⁶ steht; Z für O oder O-C(=O) steht R²⁶ für einen aliphatischen Rest steht; n gleich 1 ist; Y für O oder O-C(=O) steht; R⁵ für einen aliphatischen Rest steht und R¹, R², R³ und R⁴ jeweils für einen beliebigen der vorstehend genannten Substituenten;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte benzokondensierte Cycloheptanon-Derivate der vorstehend angegebenen allgemeinen Formel I, worin
- n: gleich 1 ist;
- X: für CH₂, O, S, S(=O) oder S(=O)₂ steht;
- Y: für O, O-C(=O), O-C(=O)-O, O-S(=O)₂, O-C(=S)-N(H) oder O-C(=O)-N(H) steht;
- R¹, R², R³ und R⁴,: unabhängig voneinander, jeweils
für H, F, Cl, Br, -SF₅, -OH, -OR¹⁰, -SR¹¹, -NR¹²R¹³,
oder für einen Rest ausgewählt aus der Gruppe bestehend aus Methyl, -CF₃, - CHF₂, -CH₂F, -CCl₃, -CBr₃, -CH₂-CN, -CH₂-NO₂, Ethyl, -CF₂-CF₃, -CH₂-CF₃, n- Propyl, Isopropyl, n-Butyl, sec-Butyl, Isobutyl, tert-Butyl, (1,1)-Dimethyl-propyl und n-Pentyl stehen;
- R⁵: für eine -C(=O)-R²⁴-Gruppe steht;
für eine -S(=O)₂-R²⁵-Gruppe steht;
für einen Rest ausgewählt aus der Gruppe bestehend aus Methyl, Ethyl, n- Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl und Cycloheptenyl steht;
oder für einen Rest ausgewählt aus der Gruppe bestehend aus Phenyl, Naphthyl, Thiophenyl, Furanyl, Pyridinyl, Indolyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, -SF₅, -CF₃, -O-CH₃, -O-C₂H₅, -SCH₃, -SC₂H₅, -O-CF₃, -S-CF₃, Methyl, Ethyl, n-Propyl, Isopropyl, n-Butyl, sec-Butyl, Isobutyl und tert-Butyl substituiert sein kann;
- R⁶: für einen Wasserstoff-Rest;
für -(CH₂)-Z-R²⁶; oder für -(CH₂)-OR²⁷ steht;
- R¹⁰, R¹¹, R¹² und R¹³,: unabhängig voneinander, jeweils
für einen Rest ausgewählt aus der Gruppe bestehend aus Methyl, -CF₃, - CH₂F, -CF₂H, Ethyl, -C₂F₅, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
- Z: für O, O-C(=O), O-C(=O)-O, O-S(=O)₂, O-C(=S)-N(H) oder O-C(=O)-N(H) steht;
- R²⁴ und R²⁵,: unabhängig voneinander, jeweils
für einen Rest ausgewählt aus der Gruppe bestehend aus Phenyl, Naphthyl, Thiophenyl, Furanyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CF₃, -SF₅, -O-CH₃, -O-C₂H₅, -S-CF₃, Methyl, Ethyl, n-Propyl, Isopropyl, n-Butyl, sec-Butyl, Isobutyl und tert-Butyl substituiert sein kann;
- R²⁶: für eine -C(=O)-R²⁸-Gruppe steht;
für eine -S(=O)₂-R²⁹-Gruppe steht;
für einen Rest ausgewählt aus der Gruppe bestehend aus Methyl, Ethyl, n- Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl und Cycloheptenyl steht;
oder für einen Rest ausgewählt aus der Gruppe bestehend aus Phenyl, Naphthyl, Thiophenyl, Furanyl, Pyridinyl, Indolyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, -SF₅, -CF₃, -O-CH₃, -O-C₂H₅, -SCH₃, -SC₂H₅, -O-CF₃, -S-CF₃, Methyl, Ethyl, n-Propyl, Isopropyl, n-Butyl, sec-Butyl, Isobutyl und tert-Butyl substituiert sein kann;
- R²⁷: für einen Wasserstoff-Rest steht;
und R²⁸ und R²⁹, unabhängig voneinander, jeweils
für einen Rest ausgewählt aus der Gruppe bestehend aus Phenyl, Naphthyl, Thiophenyl, Furanyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CF₃, -SF₅, -O-CH₃, -O-C₂H₅, -S-CF₃, Methyl, Ethyl, n-Propyl, Isopropyl, n-Butyl, sec-Butyl, Isobutyl und tert-Butyl substituiert sein kann;
wobei benzokondensierte Cycloheptanon-Derivate der allgemeinen Formel I ausgenommen sind, worin X für CH₂ steht; R⁶ für einen Wasserstoff-Rest oder für - (CH₂)-Z-R²⁶ steht; Z für O oder O-C(=O) steht; R²⁶ für einen aliphatischen Rest steht; n gleich 1 ist; Y für O oder O-C(=O) steht; R⁵ für einen aliphatischen Rest steht und R¹, R², R³ und R⁴ jeweils für einen beliebigen der vorstehend genannten Substituenten;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Noch weiter bevorzugt sind substituierte benzokondensierte Cycloheptanon-Derivate der vorstehend angegebenen allgemeinen Formel I ausgewählt aus der Gruppe bestehend aus
- [1]: Pentyl-carbaminsäure-5-oxo-2,3,4,5-tetrahydro-benzo[b]thiepin-4-ylmethyl- ester
- [2]: Phenyl-carbaminsäure-4-(phenyl-carbamoyloxymethyl)-5-oxo-2,3,4,5- tetrahydro-benzo[b]thiepin-4-ylmethyl-ester
- [3]: Phenyl-carbaminsäure-5-oxo-2,3,4,5-tetrahydro-benzo[b]thiepin-4-ylmethyl- ester
- [4]: (3-Trifluormethyl-phenyl)-carbaminsäure-5-oxo-2,3,4,5-tetrahydro- benzo[b]thiepin-4-ylmethyl-ester
- [5]: (4-Brom-phenyl)-carbaminsäure-5-oxo-2,3,4,5-tetrahydro-benzo[b]thiepin-4- ylmethyl-ester
- [6]: Cyclohexyl-carbaminsäure-4-hydroxymethyl-5-oxo-2,3,4,5-tetrahydro- benzo[b]thiepin-4-ylmethyl-ester
- [7]: Cyclohexyl-carbaminsäure-5-oxo-2,3,4,5-tetrahydro-benzo[b]thiepin-4- ylmethyl-ester
- [8]: Cyclohexyl-carbaminsäure-6-(cyclohexyl-carbamoyloxymethyl)-5-oxo-6,7,8,9- tetrahydro-5H-benzocyclohepten-6-ylmethyl-ester
- [9]: Phenyl-thiocarbaminsäure-O-(4-hydroxymethyl-5-oxo-2,3,4,5-tetrahydro- benzo[b]thiepin-4-ylmethyl)-ester
- [10]: N-(4-methyl-phenylsulfonyl)-carbaminsäure-5-oxo-2,3,4,5-tetrahydro- benzo[b]thiepin-4-ylmethyl-ester
- [11]: Naphthalin-1-yl-thiocarbaminsäure-O-(4-hydroxymethyl-5-oxo-2,3,4,5- tetrahydro-benzo[b]thiepin-4-ylmethyl)-ester
- [12]: Pentyl-carbaminsäure-4-hydroxymethyl-5-oxo-2,3,4,5-tetrahydro- benzo[b]oxepin-4-ylmethyl-ester
- [13]: Pentyl-carbaminsäure-4-hydroxymethyl-5-oxo-2,3,4,5-tetrahydro- benzo[b]thiepin-4-ylmethyl-ester
- [14]: Pentyl-carbaminsäure-4-(pentylcarbamoyloxymethyl)-5-oxo-2,3,4,5- tetrahydro-benzo[b]thiepin-4-ylmethyl-ester
- [15]: Phenyl-thiocarbaminsäure-O-(4-hydroxymethyl-5-oxo-2,3,4,5-tetrahydro- benzo[b]oxepin-4-ylmethyl)-ester
- [16]: (2,4-Difluor-phenyl-carbaminsäure)-4-(2,4-difluor-phenylcarbamoyloxymethyl)- 5-oxo-2,3,4,5-tetrahydro-benzo[b]oxepin-4-ylmethyl-ester
- [17]: (3-Trifluormethyl-phenyl)-thiocarbaminsäure-O-(4-hydroxymethyl-5-oxo- . 2,3,4,5-tetrahydro-benzo[b]thiepin-4-ylmethyl)-ester
- [18]: (3-Trifluormethyl-phenyl)-thiocarbaminsäure-O-(4-hydroxymethyl-5-oxo- 2,3,4,5-tetrahydro-benzo[b]oxepin-4-ylmethyl)-ester
- [19]: Benzoyl-carbaminsäure-4-benzoyl-carbamoyloxymethyl-5-oxo-2,3,4,5- tetrahydro-benzo[b]oxepin-4-ylmethyl-ester
- [20]: (2,4-Difluor-phenyl)-carbaminsäure-4-hydroxymethyl-5-oxo-2,3,4,5-tetrahydro- benzo[b]thiepin-4-ylmethyl-ester
- [21]: (2,4-Difluor-phenyl)-thiocarbaminsäure-O-(4-hydroxymethyl-5-oxo-2,3,4,5- tetrahydro-benzo[b]thiepin-4-ylmethyl)-ester
- [22]: (3-Trifluormethyl-phenyl)-thiocarbaminsäure-O-(6-hydroxymethyl-5-oxo- 6,7,8,9-tetrahydro-5H-benzocyclohepten-6-ylmethyl)-ester
- [23]: (2,4-Difluor-phenyl)-thiocarbaminsäure-O-(4-hydroxymethyl-5-oxo-2,3,4,5- tetrahydro-benzo[b]oxepin-4-ylmethyl)-ester
- [24]: (2,4-Difluoro-phenyl)-thiocarbaminsäure-O-(6-hydroxymethyl-5-oxo-6,7,8,9- tetrahydro-5H-benzocyclohepten-6-ylmethyl)-ester
- [25]: (3-Trifluormethyl-phenyl)-carbaminsäure-4-(3-trifluormethyl-phenyl)- carbamoyloxymethyl-5-oxo-2,3,4,5-tetrahydro-benzo[b]oxepin-4-ylmethyl-ester
- [26]: Butyl-carbaminsäure-4-hydroxymethyl-5-oxo-2,3,4,5-tetrahydro- benzo[b]thiepin-4-ylmethyl-ester
- [27]: Butyl-carbaminsäure-4-(butylcarbamoyloxymethyl)-5-oxo-2,3,4,5-tetrahydro- benzo[b]thiepin-4-ylmethyl-ester
und
- [28]: (4-Trifluoromethoxy-phenyl)-carbaminsäure-4-hydroxymethyl-5-oxo-2,3,4,5- tetrahydro-benzo[b]thiepin-4-ylmethyl-ester;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls können erfindungsgemäße Verbindungen bevorzugt sein, die im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %; besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM aufweisen.

Dabei wird Im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist ein Verfahren zur Herstellung von erfindungsgemäßen Verbindungen der vorstehend angegebenen allgemeinen Formel I Gemäß dem wenigstens eine Verbindung der allgemeinen Formel II, 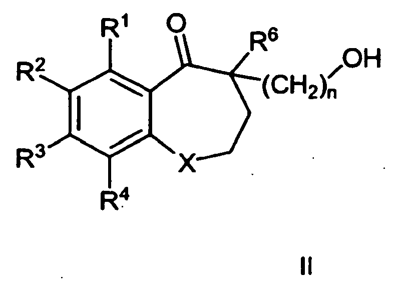 worin X, n und R¹ bis R⁴ die vorstehend genannte Bedeutung haben und R⁶ für einen Wasserstoff-Rest, -(CH₂)_{q}-NH₂ oder -(CH₂)_{q}-OR²⁷ steht, wobei q und R²⁷ die vorstehend genannte Bedeutung haben, in einem Reaktionsmedium, ggf. in Gegenwart wenigstens einer Base, bevorzugt in Gegenwart wenigstens einer organischen Base, mit wenigstens einer Verbindung der allgemeinen Formel R⁵-N=C=O und ggf. wenigstens einer Verbindung der allgemeinen Formel R²⁶-N=C=O, wobei R⁵ und R²⁶ ggf. die identische, vorstehend genannte Bedeutung haben, zu wenigstens einer Verbindung der allgemeinen Formel Ia, 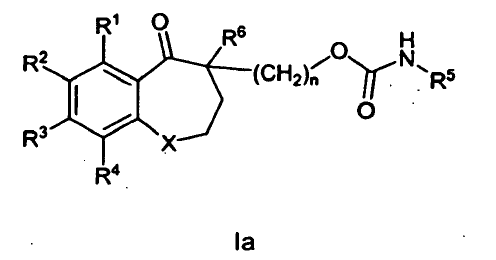 worin X, n und R¹ bis R⁵ die vorstehend genannte Bedeutung haben und R⁶ für einen Wasserstoff-Rest, für -(CH₂)ₚ-O-C(=O)-N(H)-R²⁶, für -(CH₂)ₚ-N(H)-C(=O)-N(H)-R²⁶ oder für -(CH₂)_{q}-OR²⁷ steht; wobei p, q, R²⁶ und R²⁷ die vorstehend genannte Bedeutung haben; umgesetzt wird und diese ggf. gereinigt und/oder isoliert wird;
oder
wenigstens eine Verbindung der allgemeinen Formel II; worin X, n und R¹ bis R⁴ die vorstehend genannte Bedeutung haben und R⁶ für einen Wasserstoff-Rest oder-(CH₂)_{q}-OR²⁷ steht, wobei q und R²⁷ die vorstehend genannte Bedeutung haben; in einem Reaktionsmedium, ggf. in Gegenwart wenigstens einer Base, bevorzugt in Gegenwart wenigstens einer organischen Base, mit wenigstens einer Verbindung der allgemeinen Formel R⁵-S(=O)₂-LG und ggf. wenigstens einer Verbindung der allgemeinen Formel R²⁶-S(=O)₂-LG, wobei R⁵ und R²⁶ ggf. die identische, vorstehend genannte Bedeutung haben und LG für eine Abgangsgruppe, bevorzugt für ein Halogenatom, besonders bevorzugt für ein Chloratom steht, zu wenigstens einer Verbindung der allgemeinen Formel Ib, 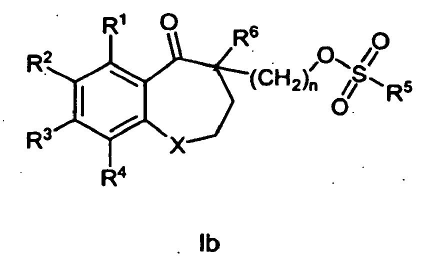 worin X, n und R¹ bis R⁵ die vorstehend genannte Bedeutung haben und R⁶ für einen Wasserstoff-Rest, für -(CH₂)ₚ-O-S(=O)₂-R²⁶ oder für -(CH₂)_{q}-OR²⁷ steht; wobei p, q, R²⁶ und R²⁷ die vorstehend genannte Bedeutung haben; umgesetzt wird und diese ggf. gereinigt und/oder isoliert wird;
oder
wenigstens eine Verbindung der allgemeinen Formel **II;** worin X, n und R¹ bis R⁴ die vorstehend genannte Bedeutung haben und R⁶ für einen Wasserstoff-Rest oder-(CH₂)_{q}-OR²⁷ steht, wobei q und R²⁷ die vorstehend genannte Bedeutung haben; in einem Reaktionsmedium, ggf. in Gegenwart wenigstens einer Base, bevorzugt in Gegenwart wenigstens einer organischen Base, mit wenigstens einer Verbindung der allgemeinen Formel R⁵-C(=O)-LG und ggf. wenigstens einer Verbindung der allgemeinen Formel R²⁶-C(=O)-LG, wobei R⁵ und R²⁶ ggf. die identische, vorstehend genannte Bedeutung haben und LG für eine Abgangsgruppe, bevorzugt für ein Halogenatom, besonders bevorzugt für ein Chloratom steht, zu wenigstens einer Verbindung der allgemeinen Formel Ic, 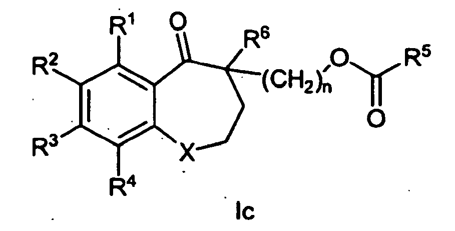 worin X, n und R¹ bis R⁵ die vorstehend genannte Bedeutung haben und R⁶ für einen Wasserstoff-Rest, für -(CH₂)ₚ-O-C(=O)-R²⁶ oder für -(CH₂)_{q}-OR²⁷ steht; wobei p, q, R²⁶ und R²⁷ die vorstehend genannte Bedeutung haben; umgesetzt wird und diese ggf. gereinigt und/oder isoliert wird;
oder
wenigstens eine Verbindung der allgemeinen Formel II; worin X, n und R¹ bis R⁴ die vorstehend genannte Bedeutung haben und R⁶ für einen Wasserstoff-Rest oder - (CH₂)_{q}-OR²⁷ steht, wobei q und R²⁷ die vorstehend genannte Bedeutung haben; in einem Reaktionsmedium, ggf. in Gegenwart wenigstens einer Base, bevorzugt in Gegenwart wenigstens einer organischen Base, mit wenigstens einer Verbindung der allgemeinen Formel R⁵-O-C(=O)-LG und ggf. wenigstens einer Verbindung der allgemeinen Formel R²⁶-O-C(=O)-LG, wobei R⁵ und R²⁶ ggf. die identische, vorstehend genannte Bedeutung haben und LG für eine Abgangsgruppe, bevorzugt für ein Halogenatom, besonders bevorzugt für ein Chloratom steht, zu wenigstens einer Verbindung der allgemeinen Formel Id, 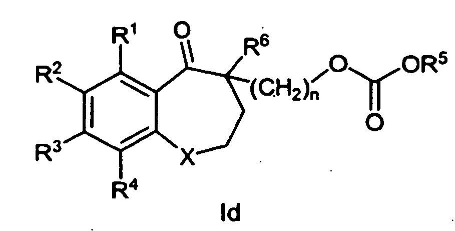 worin X, n und R¹ bis R⁵ die vorstehend genannte Bedeutung haben und R⁶ für einen Wasserstoff-Rest, für -(CH₂)ₚ-O-C(=O)-O-R²⁶ oder für -(CH₂)_{q}-OR²⁷ steht; wobei p, q, R²⁶ und R²⁷ die vorstehend genannte Bedeutung haben; umgesetzt wird und diese ggf. gereinigt und/oder isoliert wird;
oder
wenigstens eine Verbindung der allgemeinen Formel II; worin X, n und R¹ bis R⁴ die vorstehend genannte Bedeutung haben und R⁶ für einen Wasserstoff-Rest, -(CH₂)_{q}-NH₂ oder -(CH₂)_{q}-OR²⁷ steht, wobei q und R²⁷ die vorstehend genannte Bedeutung haben; in einem Reaktionsmedium, in Gegenwart wenigstens einer Base, bevorzugt in Gegenwart wenigstens eines Metallhydridsalzes, besonders bevorzugt in Gegenwart von Kalium- und/oder Natriumhydrid mit wenigstens einer Verbindung der allgemeinen Formel R⁵-LG und ggf. wenigstens einer Verbindung der allgemeinen Formel R²⁶-LG, wobei R⁵ und R²⁶ ggf. die identische, vorstehend genannte Bedeutung haben und LG für eine Abgangsgruppe, bevorzugt für ein Halogenatom, besonders bevorzugt für ein Chloratom steht, zu wenigstens einer Verbindung der allgemeinen Formel Ie, 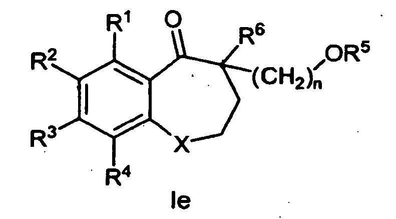 worin X, n und R¹ bis R⁵ die vorstehend genannte Bedeutung haben und R⁶ für einen Wasserstoff-Rest, für -(CH₂)ₚ-OR²⁶, für -(CH₂)ₚ-NHR²⁶ oder für -(CH₂)_{q}-OR²⁷ steht; wobei p, q, R²⁶ und R²⁷ die vorstehend genannte Bedeutung haben; umgesetzt wird und diese ggf. gereinigt und/oder isoliert wird;
oder
wenigstens eine Verbindung der allgemeinen Formel II, worin X, n und R¹ bis R⁴ die vorstehend genannte Bedeutung haben und R⁶ für einen Wasserstoff-Rest, -(CH₂)_{q}-NH₂ oder -(CH₂)_{q}-OR²⁷ steht, wobei q und R²⁷ die vorstehend genannte Bedeutung haben, in einem Reaktionsmedium, ggf. in Gegenwart wenigstens einer Base, bevorzugt in Gegenwart wenigstens einer organischen Base, mit wenigstens einer Verbindung der allgemeinen Formel R⁵-N=C=S und ggf. wenigstens einer Verbindung der allgemeinen Formel R²⁶-N=C=S, wobei R⁵ und R²⁶ ggf. die identische, vorstehend genannte Bedeutung haben, zu wenigstens einer Verbindung der allgemeinen Formel If, 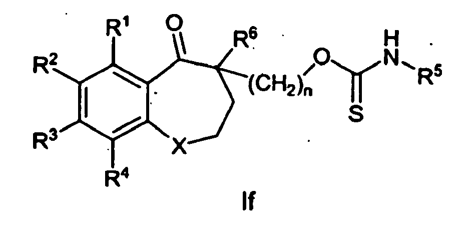 worin X, n und R¹ bis R⁵ die vorstehend genannte Bedeutung haben und R⁶ für einen Wasserstoff-Rest, für -(CH₂)ₚ-O-C(=S)-N(H)-R²⁶, für -(CH₂)ₚ-N(H)-C(=S)-N(H)-R²⁶ oder für -(CH₂)_{q}-OR²⁷ steht; wobei p, q, R²⁶ und R²⁷ die vorstehend genannte Bedeutung haben; umgesetzt wird und diese ggf. gereinigt und/oder isoliert wird;
und
ggf. wenigstens eine Verbindung der allgemeinen Formeln Ia, Ib, Ic, Id, Ie oder If, worin X, n und R¹ bis R⁵ die vorstehend genannte Bedeutung haben und Y für O, 0-C(=O), O-C(=O)-O, O-S(=O)₂, O-C(=S)-N(H) oder O-C(=O)-N(H) steht und R⁶ für - (CH₂)_{q}-OR²⁷ steht; wobei R²⁷ für einen Wasserstoff-Rest steht; in einem Reaktionsmedium ggf. in Gegenwart wenigstens einer Base, bevorzugt in Gegenwart wenigstens einer organischen Base, mit wenigstens einer Verbindung der allgemeinen Formel R²⁶-N=C=O zu wenigstens einer Verbindung der allgemeinen Formel Ia, Ib, Ic, Id oder Ie; worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für -(CH₂)ₚ-O-C(=O)-N(H)-R²⁶ steht; umgesetzt wird und diese ggf. gereinigt und/oder isoliert wird;
oder ggf. wenigstens eine Verbindung der allgemeinen Formeln Ia, Ib, Ic, Id, Ie oder If, worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für-(CH₂)_{q}-OR²⁷ steht; wobei R²⁷ für einen Wasserstoff-Rest steht; in einem Reaktionsmedium ggf. in Gegenwart wenigstens einer Base, bevorzugt in Gegenwart wenigstens einer organischen Base, mit wenigstens einer Verbindung der allgemeinen Formel R²⁶-S(=O)₂-LG zu wenigstens einer Verbindung der allgemeinen Formel Ia, Ib, Ic, Id oder Ie; worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für -(CH₂)ₚ-O-S(=O)₂-R²⁶ steht; umgesetzt wird und diese ggf. gereinigt und/oder isoliert wird;
oder ggf. wenigstens eine Verbindung der allgemeinen Formeln Ia, Ib, Ic, Id, Ie oder If, worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für - (CH₂)_{q}-OR²⁷ steht; wobei R²⁷ für einen Wasserstoff-Rest steht; in einem Reaktionsmedium ggf. in Gegenwart wenigstens einer Base, bevorzugt in Gegenwart wenigstens einer organischen Base, mit wenigstens einer Verbindung der allgemeinen Formel R²⁶-C(=O)-LG zu wenigstens einer Verbindung der allgemeinen Formel Ia, Ib, Ic, Id oder Ie; worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für -(CH₂)ₚ-O-C(=O)-R²⁶ steht; umgesetzt wird und diese ggf. gereinigt und/oder isoliert wird;
oder ggf. wenigstens eine Verbindung der allgemeinen Formeln Ia, Ib, Ic, Id, Ie oder If, worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für - (CH₂)_{q}-OR²⁷ steht; wobei R²⁷ für einen Wasserstoff-Rest steht; in einem Reaktionsmedium ggf. in Gegenwart wenigstens einer Base, bevorzugt in Gegenwart wenigstens einer organischen Base, mit wenigstens einer Verbindung der allgemeinen Formel R²⁶-O-C(=O)-LG zu wenigstens einer Verbindung der allgemeinen Formel Ia, Ib, Ic, Id oder Ie; worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für -(CH₂)ₚ-O-C(=O)-O-R²⁶ steht; umgesetzt wird und diese ggf. gereinigt und/oder isoliert wird;
oder ggf. wenigstens eine Verbindung der allgemeinen Formeln Ia, Ib, Ic, Id, Ie oder If, worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für-(CH₂)_{q}-OR²⁷ steht; wobei R²⁷ für einen Wasserstoff-Rest steht: in einem Reaktionsmedium in Gegenwart wenigstens einer Base, bevorzugt in Gegenwart wenigstens eines Metallhydridsalzes, besonders bevorzugt in Gegenwart von Kalzium- und/oder Natriumhydrid mit wenigstens einer Verbindung der allgemeinen Formel R²⁶-LG zu wenigstens einer Verbindung der allgemeinen Formel Ia, Ib, Ic, Id oder Ie; worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für -(CH₂)ₚ-O-R²⁶ steht; umgesetzt wird und diese ggf. gereinigt und/oder isoliert wird;
oder ggf. wenigstens eine Verbindung der allgemeinen Formeln Ia, Ib, Ic, Id, Ie oder If, worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für - (CH₂)_{q}-OR²⁷ steht; wobei R²⁷ für einen Wasserstoff-Rest steht; in einem Reaktionsmedium ggf. in Gegenwart wenigstens einer Base, bevorzugt in Gegenwart wenigstens einer organischen Base, mit wenigstens einer Verbindung der allgemeinen Formel -R²⁶-N=C=S zu wenigstens einer Verbindung der allgemeinen Formel Ia, Ib, Ic, Id oder Ie; worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für -(CH₂)ₚ-O-C(=S)-N(H)-R²⁶ steht; umgesetzt wird und diese ggf. gereinigt und/oder isoliert wird;
oder ggf. wenigstens eine Verbindung der allgemeinen Formeln Ia, Ib, Ic, Id, Ie oder If, worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für-(CH₂)ₚ-NH₂ steht; in einem Reaktionsmedium ggf. in Gegenwart wenigstens einer Base, bevorzugt in Gegenwart wenigstens einer organischen Base, mit wenigstens einer Verbindung der allgemeinen Formel R²⁶-N=C=S zu wenigstens einer Verbindung der allgemeinen Formel Ia, Ib, Ic, Id oder Ie; worin X, n und R¹ bis R⁵ die vorstehend genannte Bedeutung haben und Y für O; O-C(=O), O-C(=O)-O, 0-S(=O)₂, O-C(=S)-N(H) oder O-C(=O)-N(H) steht und R⁶ für-(CH₂)ₚ-N-C(=S)-N(H)-R²⁶ steht; umgesetzt wird und diese ggf. gereinigt und/oder isoliert wird;
oder ggf. wenigstens eine Verbindung der allgemeinen Formeln Ia, Ib, Ic, Id, Ie oder If, worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für - (CH₂)ₚ-NH₂ steht; in einem Reaktionsmedium ggf. in Gegenwart wenigstens einer Base, bevorzugt in Gegenwart wenigstens einer organischen Base, mit wenigstens einer Verbindung der allgemeinen Formel R²⁶-N=C=O zu wenigstens einer Verbindung der allgemeinen Formel Ia, Ib, Ic, Id oder Ie; worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für-(CH₂)ₚ-N-C(=O)-N(H)-R²⁶ steht; umgesetzt wird und diese ggf. gereinigt und/oder isoliert wird;
oder ggf. wenigstens eine Verbindung der allgemeinen Formeln Ia, Ib, Ic, Id, Ie oder If, worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für - (CH₂)ₚ-NH₂ steht; in einem Reaktionsmedium in Gegenwart wenigstens einer Base, bevorzugt in Gegenwart wenigstens eines Metallhydridsalzes, besonders bevorzugt in Gegenwart von Kalium- und/oder Natriumhydrid mit wenigstens einer Verbindung der allgemeinen Formel R²⁶-LG zu wenigstens einer Verbindung der allgemeinen Formel Ia, Ib, Ic, Id oder Ie; worin X, n und R¹ bis R⁵ die vorstehend genannte Bedeutung haben und Y für O, O-C(=O), O-C(=O)-O, O-S(=O)₂, O-C(=S)-N(H) oder O-C(=O)-N(H) steht und R⁶ für -(CH₂)ₚ-N(H)-R²⁶ steht; umgesetzt wird und diese ggf. gereinigt und/oder isoliert wird;
und ggf. wenigstens eine Verbindung der allgemeinen Formeln Ia, Ib, Ic, Id, Ie oder If, worin n, R¹ bis R⁶ und Y die vorstehend genannte Bedeutung haben und X für S steht in einem Reaktionsmedium in Gegenwart von Natriummetaperiodat zu wenigstens einer Verbindung der allgemeinen Formel Ia, Ib, Ic, Id oder Ie; worin n, R¹ bis R⁶ und Y die vorstehend genannte Bedeutung haben und X für S(=O) steht; umgesetzt wird und diese ggf. gereinigt und/oder isoliert wird;
und ggf. wenigstens eine Verbindung der allgemeinen Formeln Ia, Ib, Ic, Id, Ie oder If, worin n, R¹ bis R⁶ und Y die vorstehend genannte Bedeutung haben und X für S steht in einem Reaktionsmedium in Gegenwart von Wasserstoffperoxid und Essigsäure zu wenigstens einer Verbindung der allgemeinen Formel Ia, Ib, Ic, Id oder Ie; worin n, R¹ bis R⁶ und Y die vorstehend genannte Bedeutung haben und X für S(=O)₂ steht; umgesetzt wird und diese ggf. gereinigt und/oder isoliert wird.

Ein weiterer Gegenstand der vorliegenden Erfindung ist ein Verfahren zur Herstellung von erfindungsgemäßen Verbindungen der vorstehend angegebenen allgemeinen Formel I Gemäß dem wenigstens eine Verbindung der allgemeinen Formel II, 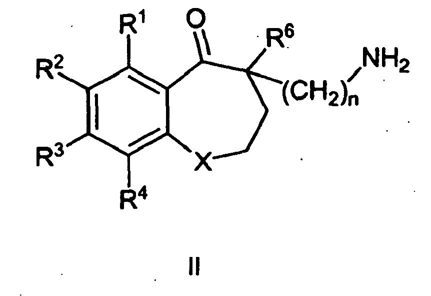 worin X, n und R¹ bis R⁴ die vorstehend genannte Bedeutung haben und R⁶ für einen Wasserstoff-Rest oder für -(CH₂)ₚ-NH₂ steht, wobei q die vorstehend genannte Bedeutung hat, in einem Reaktionsmedium, ggf. in Gegenwart wenigstens einer Base, bevorzugt in Gegenwart wenigstens einer organischen Base, mit wenigstens einer Verbindung der allgemeinen Formel R⁵-N=C=O und ggf. wenigstens einer Verbindung der allgemeinen Formel R²⁶-N=C=O, wobei R⁵ und R²⁶ ggf. die identische, vorstehend genannte Bedeutung haben, zu wenigstens einer Verbindung der allgemeinen Formel Ig, 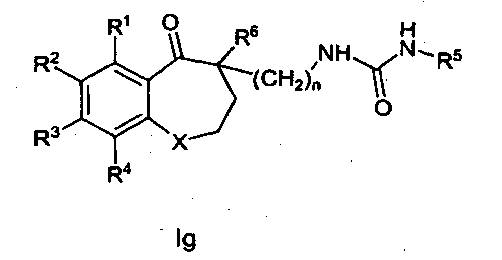 worin X, n und R¹ bis R⁵ die vorstehend genannte Bedeutung haben und R⁶ für einen Wasserstoff-Rest oder für -(CH₂)ₚ-N(H)-C(=O)-N(H)-R²⁶ steht; wobei p und R²⁶ die vorstehend genannte Bedeutung haben; umgesetzt wird und diese ggf. gereinigt und/oder isoliert wird;
oder wenigstens eine Verbindung der allgemeinen Formel III in einem Reaktionsmedium, ggf. in Gegenwart wenigstens einer Base, bevorzugt in Gegenwart wenigstens einer organischen Base, mit wenigstens einer Verbindung der allgemeinen Formel R⁵-N=C=S und ggf. wenigstens einer Verbindung der allgemeinen Formel R²⁶-N=C=S, wobei R⁵ und R²⁶ ggf. die identische, vorstehend genannte Bedeutung haben, zu wenigstens einer Verbindung der allgemeinen Formel Ih, 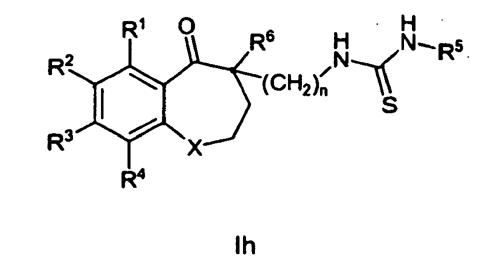 worin X, n und R¹ bis R⁵ die vorstehend genannte Bedeutung haben und R⁶ für einen Wasserstoff-Rest oder für-(CH₂)ₚ-N(H)-C(=S)-N(H)-R²⁶ steht; wobei p und R²⁶ die vorstehend genannte Bedeutung haben; umgesetzt wird und diese ggf. gereinigt und/oder isoliert wird;
oder wenigstens eine Verbindung der allgemeinen Formel III in einem Reaktionsmedium, in Gegenwart wenigstens einer Base, bevorzugt in Gegenwart wenigstens eines Metallhydridsalzes, besonders bevorzugt in Gegenwart von Kalium- und/oder Natriumhydrid mit wenigstens einer Verbindung der allgemeinen Formel R⁵-LG und ggf. wenigstens einer Verbindung der allgemeinen Formel R²⁶-LG, wobei R⁵ und R²⁶ ggf. die identische, vorstehend genannte Bedeutung haben und LG für eine Abgangsgruppe, bevorzugt für ein Halogenatom, besonders bevorzugt für ein Chloratom steht, zu wenigstens einer Verbindung der allgemeinen Formel Ik, 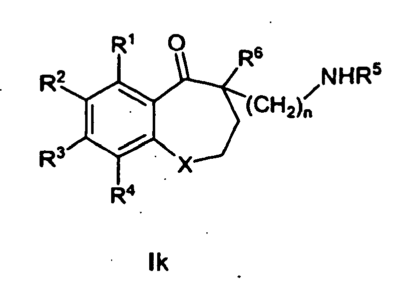 worin X, n und R¹ bis R⁵ die vorstehend genannte Bedeutung haben und R⁶ für einen Wasserstoff-Rest oder für-(CH₂)ₚ-R²⁶ steht; wobei p und R²⁶ die vorstehend genannte Bedeutung haben; umgesetzt wird und diese ggf. gereinigt und/oder isoliert wird;
oder ggf. wenigstens eine Verbindung der allgemeinen Formeln Ig, Ih oder Ik, worin X, n und R¹ bis R⁵ die vorstehend genannte Bedeutung haben und Y für N(H)-C(=O)-N(H) oder N(H)-C(=S)-N(H) steht und R⁶ für -(CH₂)ₚ-NH₂ steht; in einem Reaktionsmedium ggf. in Gegenwart wenigstens einer Blase, bevorzugt in Gegenwart wenigstens einer organischen Base, mit wenigstens einer Verbindung der allgemeinen Formel R²⁶-N=C=O zu wenigstens einer Verbindung der allgemeinen Formel Ig, Ih oder Ik; worin X, n und R¹ bis R⁵ die vorstehend genannte Bedeutung haben und Y für N(H)-C(=O)-N(H) oder N(H)-C(=S)-N(H) steht und R⁶ für -(CH₂)ₚ-N(H)-C(=O)-N(H)-R²⁶ steht; umgesetzt wird und diese ggf. gereinigt und/oder isoliert wird;
oder ggf. wenigstens eine Verbindung der allgemeinen Formeln Ig, Ih oder Ik, worin X, n und R¹ bis R⁵ die vorstehend genannte Bedeutung haben und Y für N(H)-C(=O)-N(H) oder N(H)-C(=S)-N(H) steht und R⁶ für -(CH₂)ₚ-NH₂ steht; in einem Reaktionsmedium ggf. in Gegenwart wenigstens einer Base, bevorzugt in Gegenwart wenigstens einer organischen Base, mit wenigstens einer Verbindung der allgemeinen Formel R²⁶-N=C=S zu wenigstens einer Verbindung der allgemeinen Formel Ig, Ih oder Ik; worin X, n und R¹ bis R⁵ die vorstehend genannte Bedeutung haben und Y für N(H)-C(=O)-N(H) oder N(H)-C(=S)-N(H) steht und R⁶ für -(CH₂)ₚ-N(H)-C(=S)-N(H)-R²⁶ steht; umgesetzt wird und diese ggf. gereinigt und/oder isoliert wird;
oder ggf. wenigstens eine Verbindung der allgemeinen Formeln Ig, Ih oder Ik, worin X, n und R¹ bis R⁵ die vorstehend genannte Bedeutung haben und Y für N(H)-C(=O)-N(H) oder N(H)-C(=S)-N(H) steht und R⁶ für -(CH₂)ₚ-NH₂ steht; in einem Reaktionsmedium, in Gegenwart wenigstens einer Base, bevorzugt in Gegenwart wenigstens eines Metallhydridsalzes, besonders bevorzugt in Gegenwart von Kalium- und/oder Natriumhydrid mit wenigstens einer Verbindung der allgemeinen Formel R²⁶-LG zu wenigstens einer Verbindung der allgemeinen Formel Ig, Ih oder Ik; worin X, n und R¹ bis R⁵ die vorstehend genannte Bedeutung haben und Y für N(H)-C(=O)-N(H) oder N(H)-C(=S)-N(H) steht und R⁶ für-(CH₂)ₚ-N(H)-R²⁶ steht; umgesetzt wird und diese ggf. gereinigt und/oder isoliert wird;
und ggf. wenigstens eine Verbindung der allgemeinen Formeln Ig, Ih oder Ik, worin n, R¹ bis R⁶ und Y die vorstehend genannte Bedeutung haben und X für S steht in einem Reaktionsmedium in Gegenwart von Natriummetaperiodat zu wenigstens einer Verbindung der allgemeinen Formel Ia, Ib, Ic, Id oder Ie; worin n, R¹ bis R⁶ und Y die vorstehend genannte Bedeutung haben und X für S(=O) steht; umgesetzt wird und diese ggf. gereinigt und/oder isoliert wird;
und ggf. wenigstens eine Verbindung der allgemeinen Formeln Ig, Ih oder Ik, worin n, R¹ bis R⁶ und Y die vorstehend genannte Bedeutung haben und X für S steht in einem Reaktionsmedium in Gegenwart von Wasserstoffperoxid und Essigsäure zu wenigstens einer Verbindung der allgemeinen Formel Ia, Ib, Ic, Id oder Ie; worin n, R¹ bis R⁶ und Y die vorstehend genannte Bedeutung haben und X für S(=O)₂ steht; umgesetzt wird und diese ggf. gereinigt und/oder isoliert wird.

Die Umsetzung von Verbindungen der allgemeinen Formeln II, III, la, Ib, Ic, Id, Ie, If, Ig, Ih und Ik mit Isocyanaten oder Isothiocyanaten der allgemeinen Formeln R⁵-N=C=O, R⁵-N=C=S, R²⁶-N=C=O und R²⁶-N=C=S erfolgt in einem Reaktionsmedium, vorzugsweise ausgewählt aus der Gruppe bestehend aus Acetonitril, Dichlormethan, Chloroform, Tetrahydrofuran, Diethylether, Toluol, Benzol, Ethanol, Methanol, Wasser und entsprechenden Mischungen, ggf. in Gegenwart wenigstens einer Base, bevorzugt in Gegenwart wenigstens einer Base ausgewählt aus der Gruppe bestehend aus Triethylamin, N-Methylmorpholin, Pyridin, 4,4-Dimethylaminopyridin und Diisopropylethylamin, bei Temperaturen zwischen 0 °C und 100 °C. Bevorzugt erfolgt die Umsetzung in einem Reaktionsmedium ausgewählt aus der Gruppe bestehend aus Diethylether, Tetrahydrofuran, Dichlormethan und Chloroform unter Einstrahlung von Mikrowellen.

Die Umsetzung von Verbindungen der allgemeinen Formeln II, III, Ia, Ib, Ic, Id, Ie, If, Ig, Ih und Ik mit Carbonsäurederivaten, Kohlensäurederivaten bzw. Sulfonsäurederivaten der allgemeinen Formeln R⁵-C(=O)-LG, R²⁶-C(=O)-LG, R⁵-O-C(=O)-LG, R²⁶-O-C(=O)-LG, R⁵-S(=O)₂-LG und R²⁶-S(=O)₂-LG erfolgt in einem Reaktionsmedium, vorzugsweise ausgewählt aus der Gruppe bestehend aus Diethylether, Pyridin, Tetrahydrofuran, Acetonitril, Methanol, Ethanol, Dimethylformamid, Dichlormethan und entsprechenden Mischungen, ggf. in Gegenwart einer organischen Base, vorzugsweise ausgewählt aus der Gruppe bestehend aus Triethylamin, 4,4-Dimethylaminopyridin, Pyridin und Diisopropylethylamin, oder einer anorganischen Base, bei Temperaturen von vorzugsweise -70°C bis 100°C.

Die Umsetzung von Verbindungen der allgemeinen Formeln II, III, Ia, Ib, Ic, Id, Ie, If, Ig, Ih und Ik mit Verbindungen der allgemeinen Formeln R⁵-LG und R²⁶-LG erfolgt in einem Reaktionsmedium, vorzugsweise ausgewählt aus der Gruppe bestehend aus Dichlormethan, Toluol, Tetrahydrofuran, Acetonitril, Diethylether, Dioxan und entsprechenden Mischungen ggf. in Gegenwart wenigstens einer Base, bevorzugt in Gegenwart wenigstens eines Metallhydridsalzes, besonders bevorzugt in Gegenwart von Natrium- und/oder Kaliumhydrid.

Die Verbindungen der allgemeinen Formel II lassen sich wie in Schema 1. beschrieben erhalten.

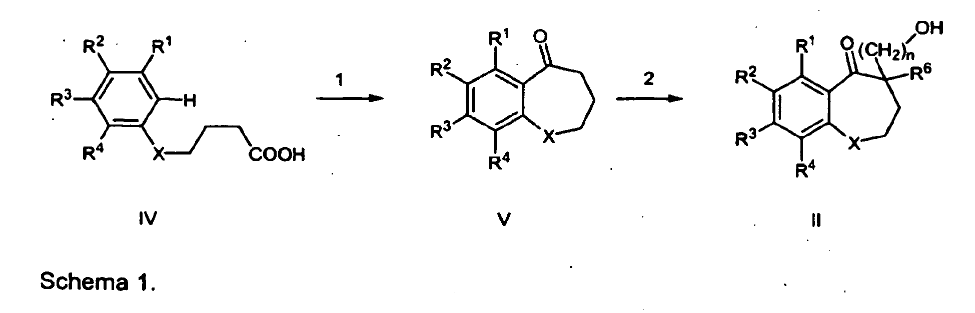

In Stufe 1 werden Verbindungen der allgemeinen Formel IV, worin R¹ bis R⁴ die vorstehend genannte Bedeutung haben und X für O, S, N(H), N(R⁷), N[C(=O)-R⁸] oder N[C(=O)-O-R⁹] steht, in einem sauren Reaktionsmedium, vorzugsweise in einem sauren Reaktionsmedium ausgewählt aus der Gruppe bestehend aus Schwefelsäure und Polyphosphorsäure, besonders bevorzugt in Polyphosphorsäure, bei Temperaturen zwischen 20°C und 100 °C zu Verbindungen der allgemeinen Formel VI, worin R¹ bis R⁴ die vorstehend genannte Bedeutung haben und X für O, S, N(H), N(R⁷), N[C(=O)-R⁸] oder N[C(=O)-O-R⁹] steht, umgesetzt.

Die Synthese von 3,4-Dihydro-2H-benzo[b]thiepin-5-on (allgemeine Formel V, R¹ bis R⁴ jeweils gleich H und X gleich S) ist von V. J. Traynelis et al. in Journal of Organic Chemistry 1961, 26, 2728-2733 beschrieben. Die Synthese von 3,4-Dihydro-2H-benzo[b]oxepin (allgemeine Formel V, R¹ bis R⁴ jeweils gleich H und X gleich O) ist von G. Fontaine et al. in Annales de Chimie 1968, 3, 179-191 beschrieben. Die entsprechenden Beschreibungen werden hiermit als Referenz eingeführt und gelten als Teil der vorliegenden Offenbarung.

In Stufe 2 werden Verbindungen der allgemeinen Formel VI, worin R¹ bis R⁴ die vorstehend genannte Bedeutung haben und X für CH₂, O, S, N(H), N(R⁷), N[C(=O)-R⁸] oder N[C(=O)-O-R⁹] steht, aus Tetrahydrofuran, Methanol, Ethanol, Isopropanol, Wasser, Dimethylformamid, Dichlormethan, Toluol, Diethylether und entsprechenden Mischungen in Gegenwart wenigstens einer Base, vorzugsweise in Gegenwart wenigstens einer anorganischen Base, besonders bevorzugt in Gegenwart von Kaliumcarbonat, Natriumcarbonat, Lithiumcarbonat und Magnesiumcarbonat, mit Formaldehyd, einem Formaldehyd-Äquivalent oder wässriger Formalin-Lösung bei Temperaturen zwischen 20 °C und 80 °C zu Verbindungen der allgemeinen Formel II, worin R¹ bis R⁴ und R⁶ die vorstehend genannte Bedeutung haben, n für 1 steht und X für CH₂, O, S, N(H), N(R⁷), N[C(=O)-R⁸] oder N[C(=O)-O-R⁹] steht, umgesetzt.

Ggf. werde Verbindungen der allgemeinen Formel II, worin R¹ bis R⁴ und R⁶ die vorstehend genannte Bedeutung haben, n für 1 steht und X für S steht, in einem Reaktionsmedium, vorzugsweise in einem Reaktionsmedium ausgewählt aus der Gruppe bestehend aus Methanol, Ethanol, Isopropanol, Wasser und entsprechenden Mischungen, bei Temperaturen zwischen 0 °C und 50 °C mit Natriummetaperiodat zur Verbindungen der allgemeinen Formel II, worin R¹ bis R⁴ und R⁶ die vorstehend genannte Bedeutung haben, n für 1 steht und X für S(=O) steht, umgesetzt.

Ggf. werde Verbindungen der allgemeinen Formel II, worin R¹ bis R⁴ und R⁶ die vorstehend genannte Bedeutung haben, n für 1 steht und X für S steht, in einem Reaktionsmedium, vorzugsweise in einem Reaktionsmedium ausgewählt aus der Gruppe bestehend aus Methanol, Ethanol, Isopropanol, Wasser und entsprechenden Mischungen, bei Temperaturen zwischen 0 °C und 100 °C mit Essigsäure und mit wässriger Wasserstoffperoxid-Lösung zur Verbindungen der allgemeinen Formel II, worin R¹ bis R⁴ und R⁶ die vorstehend genannte Bedeutung haben, n für 1 steht und X für S(=O)₂ steht, umgesetzt.

Die Verbindungen der vorstehend angegebenen Formeln IV, V, R⁵-N=C=O, R⁵-N=C=S, R²⁶-N=C=O, R²⁶-N=C=S, R⁵-C(=O)-LG, R²⁶-C(=O)-LG, R⁵-O-C(=O)-LG, R²⁶-O-C(=O)-LG, R⁵-S(=O)₂-LG, R²⁶-S(=O)₂-LG, R⁵-LG und R²⁶-LG sind ggf. jeweils am Markt käuflich erhältlich und können auch nach üblichen, dem Fachmann bekannten Verfahren hergestellt werden.

Die vorstehend beschriebenen Umsetzungen können jeweils unter den üblichen dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche der vorstehend genannten Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Die erfindungsgemäßen substituierten benzokondensierten Cycloheptanon-Derivate der vorstehend genannten allgemeinen Formeln I, Ia Ib, Ic, Id, Ie, If, Ig, Ih und Ik, im Folgenden nur als benzokondensierte Cycloheptanon-Derivate der allgemeinen Formel I bezeichnet, sowie entsprechende Stereoisomere können sowohl in Form ihrer freien Basen, ihrer freien Säuren wie auch in Form entsprechender Salze, insbesondere physiologisch verträglicher Salze, isoliert werden. Die freien Basen der jeweiligen erfindungsgemäßen substituierten benzokondensierten Cycloheptanon-Derivate der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden. Die freien Basen der jeweiligen benzokondensierten Cycloheptanon-Derivate der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten benzokondensierten Cycloheptanon-Derivate der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-AlkylRest steht, genannt.

Die erfindungsgemäßen substituierten benzokondensierten Cycloheptanon-Derivate der vorstehend genannten allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erkalten werden.

Sofern die erfindungsgemäßen substituierten benzokondensierten Cycloheptanon-Derivate der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten benzokondensierten Cycloheptanon-Derivate der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens ein substituiertes benzokondensiertes Cycloheptanon-Derivat der vorstehend angegebenen allgemeinen Formel 1, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, insbesondere zur Vanilloid-Rezeptor 1-(VR1/TRPV1)-Hemmung und/oder zur Vanilloid-Rezeptor 1 (VR1/TRPV1)-Stimulation. Ferner eignen sich die erfindungsgemäßen Arzneimittel zur CB1-Rezeptor-Regulation und/oder zur CB2-Rezeptor-Regulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1, CB1-Rezeptoren und/oder CB2-Rezeptoren vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens eines substituierten benzokondensierten Cycloheptanon-Derivates sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1 (VR1/TRPV1)-Stimulation, zur CB1-Rezeptor-Regulation und/oder zur CB2-Rezeptor-Regulation.

Bevorzugt ist die Verwendung wenigstens eines substituierten benzokondensierten Cycloheptanon-Derivates sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1, CB1-Rezeptoren und/oder CB2-Rezeptoren vermittelt werden.

Besonders bevorzugt ist die Verwendung wenigstens eines substituierten benzokondensierten Cycloheptanon-Derivates sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Ganz besonders bevorzugt ist die Verwendung wenigstens eines substituierten benzokondensierten Cycloheptanon-Derivates sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit;
Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens eines substituierten benzokondensierten Cycloheptanon-Derivates sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmerz, neuropathischem Schmerz und visceralem Schmerz, und/oder Harninkontinenz.

Das erfindungsgemäße Arzneimittel eignet sich zur Verabreichung an Erwachsene und Kinder einschließlich Kleinkindern und Säuglingen. Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens eines substituierten benzokondensierten Cycloheptanon-Derivats der vorstehend angegebenen allgemeinen Formel I, ggf. in Form eines seiner reinen Stereoisomeren, insbesondere Enantiomeren oder Diastereomeren, seiner Racemate oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Die in dem erfindungsgemäßen Arzneimittel zum Einsatz kommenden erfindungsgemäßen substituierten benzokondensierten Cycloheptanon-Derivate können, in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können das jeweilige erfindungsgemäße substituierte benzokondensierte Cycloheptanon-Derivat auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung. Die an den Patienten zu verabreichende Menge der jeweiligen erfindungsgemäßen benzokondensierten Cycloheptanon-Derivate der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,001 bis 100 mg/kg, vorzugsweise 0,05 bis 75 mg/kg, besonders bevorzugt 0,05 bis 50 mg/kg, Körpergewicht des Patienten wenigstens einer solchen erfindungsgemäßen Verbindung appliziert.

### Pharmakologische Methoden:

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit
10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in kalten, d. h. in einem Eisbad befindlichen, HBSS-Puffer (Hank's buffered saline solution, Gibco Invitrogen GmbH, Karisruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vor-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die zurückgebliebenen DRGs werden jeweils mit 500 µL Komplett-Medium versetzt. Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die **Messung der höchsten Fluoreszenzintensität (FC, Fluorescence Counts) über die Zeit.**

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

### II. Funktionelle Untersuchungen am Vanilloid Rezeptor (VR1)

Die agonistische bzw. antagonistische Wirkung der zu untersuchenden Substanzen auf Vanilloid Rezeptor (VR1) kann auch mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden, Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37 °C und 5 % CO₂ in einem Kulturmedium (Nutrient Mixture Ham's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0,01 Vol-%, Molecular Probes in HBSS (Hank's buffered saline solution), Gibco Invitrogen GmbH, Karlsruhe, Deutschland) für 30 Minuten bei 37°C inkubiert. Anschließend werden die Platten 3 mal mit HBSS-Puffer gewaschen und nach einer weiteren Inkubation von 15 Minuten bei Raumtemperatur zur Ca²⁺⁻-Messung im FLIPR eingesetzt. Die Ca²⁺-abhängige Fluoreszenz wird dabei vor und nach Zugabe der zu untersuchenden Substanzen gemessen (Wellenlänge λₑₓ=488 nm, λₑₘ=540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺⁻-Signal nach Zugabe von 10 µM Capsaicin). Nach 5 Minuten Inkubation werden 100 nM Capsaicin appliziert und ebenfalls der Einstrom von Ca²⁺ ermittelt.

Desensitisierende Agonisten und Antagonisten führten zu einer Unterdrückung des Ca²⁺-Einstroms. Es werden % Inhibierung im Vergleich zu der maximal erreichbaren Inhibierung mit 10 µM Capsazepin berechnet.

### III. Formalin-Test an der Maus

Die Untersuchung zur Bestimmung der antinociceptiven Wirkung der erfindungsgemäßen Verbindungen wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen Verbindungen werden in der zweiten Phase des Formatin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen Verbindungen vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wässrige Lösung) in die dorsale Seite der rechten Hinterpfote, so dass bei freibeweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfasst. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wässrige Natriumchlorid-Lösung) vor Formalinapplikation erhalten. Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formaün-Test antinociceptiv wirksam sind, werden die beschriebenen Verhaltensweisen der Tiere, d. h. Lecken und Beißen, reduziert bzw. aufgehoben.

### IV. Untersuchung auf analgetische Wirksamkeit im Writhing-Test

Die Untersuchung der erfindungsgemäßen Verbindungen der allgemeinen Formel I auf analgetische Wirksamkeit wurde im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Verbindungsdosis erhielten 10 Minuten nach intravenöser Gabe der zu untersuchenden Verbindungen 0,3 ml/Maus einer 0,02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere wurden einzeln in Beobachtungskäfige gesetzt. Mit Hilfe eines Drucktastenzählers wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten hatten. Alle Verbindungen wurden in der Standarddosierung von 10 mg/kg getestet.

### V. Funktionelle Untersuchung am humanen CB1-Rezeptor

### Inkubations- und Waschpuffer:

50mM TRIS (Fa. Fluka Best.Nr. 93349); 2.5mM EDTA (Fa. Fluka Best.Nr. 03680); 5mM MgCl₂ (Fa. Merck Best.Nr. 1.05833); 0.5 mg/ml BSA (Fa. Sigma Best.Nr. A-2153)
Der pH-Wert des Puffers wird bei 4°C auf 7.4 eingestellt.

### Medium zum Einweichen der Filtermatten:

0.05% PEI (Fa. Sigma Best.Nr. P-3143)

Die Membranen (RBHCB1M, Fa.Perkin Elmer (human rekombinante Zellmembranen)) wurden in Aliquots zu je 1 ml in Trockeneis geliefert und bei - 80°C aufbewahrt. Die Proteinkonzentration der Chargen betrug um 6 mg/ml. Für die Testung wurde jeweils 1 ml rasch aufgetaut und mit 7 ml Inkubationspuffer (1:8) verdünnt. 20µl dieser Verdünnung wurden im Test eingesetzt. Dieses entsprach einem Proteingehalt von ca.15 µg im Ansatz.

### Inkubationsansatz:

Verwendet wurden MTP der Firma Costar® vom "Typ U" (Assay-MTP; Bestell-Nr.3794). Die Pipettierreihenfolge ist in der folgenden Tabelle 1. wieder gegeben.

**Tabelle 1.**

| **Substanz** | **Molarität im Ansatz** | **µl** | **Protein im Ansatz** |
|---|---|---|---|
| Inkubat.-Puffer | - | 200 | - |
| Testsubstanz oder USB* | 10⁻⁵ M | 5 | - |
| Membran | - | 20 | ca.15 µg |
| [³H]CP55,940 | 1 nM | 25 | - |

| | | | |
|---|---|---|---|
| * USB (unspezifische Bindung): WIN 55,212-Mesylate (Fa. Tocris, Best. Nr. 1038) (10⁻⁶ M im Ansatz) [³H] CP-55,940 (Fa. Perkin Elmer Best.Nr. 1051) | | | |

Nach beendetem Pipettiervorgang wurde ein Deckel auf die MTP gelegt und die Inkubation erfolgte 90 min bei 25 °C.

Anschließend wurden die Proben mit Hilfe des Brandel-Cell-Harvesters (Modell MPXRI-96T) über eine mit 0.05% PEI vorgeweichte GF/B-Unifilter-MTP (Fa. Packard Best.Nr. 6005177) abgesaugt. Gewaschen wurde zweimal mit 200 ml eisgekühltem Inkubationspuffer pro 96-er MTP. Hiernach wurde die Platte für 1 h bei + 60°C im Trockenschrank getrocknet. Anschließend wurde die Bodenseite der MTP von unten her exakt mit einem "back seal" der Firma Packard versiegelt. Danach wurden pro well 35 µl Szintillator (Packard, Typ "Ultima Gold";Bestell-Nr.6013151) hinzu pipettiert. Ferner wurde nunmehr die Plattenoberseite mit einem sog. "top seal" (Firma Packard; Best.Nr. 6005185) versiegelt. Nach 1 h Wartezeit wurde die Platte am "Trilux" der Firma Wallac ausgemessen.

### VI. Bestimmung der Affinität zum Cannabinoidrezeptor CB2 (CB2-Rezeptor):

Zur Bestimmung der Affinität der erfindungsgemäßen Verbindungen zum Cannabinoidrezeptor werden Membranen aus humanen rekombinanten HEK-293EBNA-Zellen verwendet, die stabil mit dem menschlichen CB2-Rezeptor transfiziert wurden. Als Radioligand wurde mit Tritium markiertes 5-(1,1-Dimethylheptyl)-2-(5-hydroxypropyl)cyclohexyl)-1-alpha,2-beta,5-alpha)-phenol (^{[3]}H-CP 55,940 mit 103,4 Ci/mmol, 1 m Ci/ml) verwendet. Die Bestimmung erfolgte in einem Testpuffer aus 50 mM Tris-HCl, 2.5mM EDTA, 5mM MgCl₂ und 1.0 mg/ml Fettsäure-freiem BSA. Die Prüfsubstanzen wurden jeweils in DMSO gelöst.

Die Affinität der erfindungsgemäßen Verbindungen zum CB2-Rezeptor wird durch deren Fähigkeit, ^{[3]}H-CP 55,940 aus CB2-Rezeptoren in Membranen aus HEK-293EBNA-Zellen zu verdrängen, bestimmt. Dazu werden in Vertiefungen auf einer Mikrotiterplatte jeweils 8 µg der Membranen (20 µl einer Lösung aus Membranen in einer Konzentration von 400 µg/ml) mit einer 0.33 nM Lösung von ^{[3]}H-CP 55,949 (120 Ci/mmol) in einem Gesamtvolumen aus Testpuffer von 200 µl für 90 Minuten bei 30 °C inkubiert.
Anschließend werden entweder die Prüfsubstanzen bzw. WIN 55212-2 zur Bestimmung der nicht-spezifischen Bindung jeweils gelöst in DMSO in die Vertiefungen gegeben, so dass jeweils eine Konzentration der entsprechenden Substanzen von 10 µM erhalten wird.
Es wird für weitere 40 Minuten bei 30 °C inkubiert. Die Bindungs-Reaktion wurde durch rasche Filtration über GF/C-Filterpapier, das mit 0,05% PEI behandelt worden ist, unter Verwendung eines Brandel-Zell-Harvesters mit 96 Vertiefungen beendet. Die Filter werden neun Mal mit 0.5 ml eiskaltem Wasch-Puffer (50 nM Tris-HCl, 5 mM MgCl₂, .2.5mM EDTA, 2 % BSA, pH 7.4) gewaschen, luftgetrocknet, in Szintillationsflüssigkeit gesetzt, und die Radioaktivität mit Hilfe eines Szintillationszählers bestimmt.

Die prozentuale Verdrängung des radioaktiven Liganden ^{[3]}H-CP 55,940 aus seiner Bindung zum CB2-Rezeptor wird als Prozent Hemmung der spezifischen Bindung angegeben.

Im folgenden wird die Erfindung anhand von Beispielen erläutert. Diese Erläuterungen sind lediglich beispielhaft und schränken den allgemeinen Erfindungsgedanken nicht ein.

### Beispiele:

Die Ausbeuten der hergestellten Verbindungen sind nicht optimiert.

Alle Temperaturen sind unkorrigiert.

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.
Die Mischungsverhältnisse von Lösungsmitteln, Laufmitteln oder für chromatographische Untersuchungen sind stets in Volumen/Volumen angegeben.
Die Analytik erfolgte durch Massenspektroskopie und NMR.

### 4-Hydroxymethyl-3,4-dihydro-2H-benzo[b]thiepin-5-on und 4,4-Bis-hydroxymethyl-3,4-dihydro-2H-benzo[b]thiepin-5-on

Zu einer Lösung von 3,4-Dihydro-2H-benzo[b]thiepin-5-on (16.5 g; 92.6 mmol) in 275 ml Tetrahydrofuran wurden bei Raumtemperatur Kaliumcarbonat (1.37 g) und Formalin-Lösung (55 ml; 36%-ige Lösung in Wasser) gegeben. Die Reaktionsmischung wurde 7 Tage lang bei 40°C gerührt. Dann wurde mit 300 ml Wasser verdünnt, die Phasen getrennt und mit Ethylacetat extrahiert. Das Rohprodukt wurde säulenchromatographisch an Kieselgel (Eluent: Hexan/Ethylacetat 9:1 und Hexan/Ethylacetat 4:1) gereinigt. Man erhielt 4-Hydroxymethyl-3,4-dihydro-2H-benzo[b]thiepin-5-on (farbloses Öl; 5.1 g; 26.5 %) und 4,4-Bis-hydroxymethyl-3,4-dihydro-2H-benzo[b]thiepin-5-on (farbloser Feststoff; 8.7 g; 39 %).

### 6,6-Bis-hydroxymethyl-6,7,8,9-tetrahydro-benzocyclohepten-5-on

Zu einer Lösung von 6,7,8,9-Tetrahydro-benzocyclohepten-5-on (50 g, 312 mmol) in 875 ml Tetrahydrofuran wurden bei Raumtemperatur Kaliumcarbonat (4.4 g) und Formalin-Lösung (175 ml; 36%-ige Lösung in Wasser) gegeben. Die Reaktionsmischung wurde 32 Stunden lang bei 40°C gerührt. Dann wurde mit 900 ml Wasser verdünnt, die Phasen getrennt und mit Ethylacetat extrahiert. Das Rohprodukt wurde säulenchromatographisch an Kieselgel (Eluent: Hexan/Ethylacetat 9:1 und Hexan/Ethylacetat 1:1) gereinigt. Man erhielt 6,6-Bis-hydroxymethyl-6,7,8,9-tetrahydro-benzocyclohepten-5-on (farbloses Öl; 61.3 g; 89 %).

### 4,4-Bis-hydroxymethyl-3,4-dihydro-2H-benzo[b]oxepin-5-on

Zu einer Lösung von 3,4-Dihydro-2H-benzo[b]oxepin-5-on (12.0 g; 74 mmol) in 215 ml Tetrahydrofuran wurden bei Raumtemperatur Kaliumcarbonat (1.01 g) und Formalin-Lösung (44 ml; 36%-ige Lösung in Wasser) gegeben. Die Reaktionsmischung wurde 2 Tage lang bei 40°C gerührt. Dann wurde mit 250 ml Wasser verdünnt, die Phasen getrennt und mit Ethylacetat extrahiert. Das Rohprodukt wurde säulenchromatographisch an Kieselgel (Eluent: Hexan/Ethylacetat 1:1 und Ethylacetat) gereinigt. Man erhielt 4,4-Bis-hydroxymethyl-3,4-dihydro-2H-benzo[b]oxepin-5-on (farblose Kristalle; 13.1 g; 80 %).

### Beispielverbindung 1:

### Pentyl-carbaminsäure-5-oxo-2,3,4,5-tetrahydro-benzo[b]thiepin-4-ylmethyl-ester

Zu einer Lösung von 4-Hydroxymethyl-3,4-dihydro-2H-benzo[b]thiepin-5-on (264 mg, 1.27 mmol) in 16 ml Tetrahydrofuran wurden bei 0°C Triethylamin (0.2 ml, 1.4 mmol) und n-Pentylisocyanat (492 µl, 4.9 mmol) gegeben. Es wurde 15 Stunden lang unter Rückfluss erhitzt, eingeengt und säulenchromatographisch an Kieselgel (Eluent: Diisopropylether/Hexan 4:1) gereinigt. Man erhielt das Produkt in einer Ausbeute von 145 mg (35%).

### Beispielverbindung 2:

### Phenyl-carbaminsäure-4-(phenyl-carbamoyloxymethyl)-5-oxo-2,3,4,5-tetrahydro-benzo[b]thiepin-4-ylmethyl-ester

Zu einer Lösung von 4,4-Bis-hydroxymethyl-3,4-dihydro-2H-benzo[b]thiepin-5-on (395 mg, 1.66 mmol) in 21 ml Tetrahydrofuran wurden bei 0°C Triethylamin (0.25 ml, 1.8 mmol) und Phenylisocyanat (395 µl, 3.66 mmol) gegeben. Es wurde 24 Stunden lang bei Raumtemperatur gerührt, eingeengt und säulenchromatographisch an Kieselgel (Eluent: Dichlormethan) gereinigt. Man erhielt das Produkt in einer Ausbeute von 665 mg (84%).

### Beispielverbindung 3:

### Phenyl-carbaminsäure-5-oxo-2,3,4,5-tetrahydro-benzo[b]thiepin-4-ylmethyl-ester

Zu einer Lösung von 4-Hydroxymethyl-3,4-dihydro-2H-benzo[b]thiepin-5-on (359 mg, 1.726 mmol) in 22 ml Tetrahydrofuran wurden bei 0°C Triethylamin (0.26 ml, 1.9 mmol) und Phenylisocyanat (410 µl, 3.8 mmol) gegeben. Es wurde 2 Stunden lang bei Raumtemperatur gerührt, eingeengt und säulenchromatographisch an Kieselgel (Eluent: *tert*-Butylmethylether / Cyclohexan 1:2) gereinigt. Man erhielt das Produkt in einer Ausbeute von 350 mg (62%).

### Beispielverbindung 4:

### (3-Trifluormethyl-phenyl)-carbaminsäure-5-oxo-2,3,4,5-tetrahydro-benzo[b]thiepin-4-ylmethyl-ester

Zu einer Lösung von 4-Hydroxymethyl-3,4-dihydro-2H-benzo[b]thiepin-5-on (256 mg, 1.23 mmol) in 15 ml Tetrahydrofuran wurden bei Raumtemperatur Triethylamin (0.19 ml, 1.4 mmol) und α,α,α-Trifluor-m-tolyl-isocyanat (345 µl, 2.46 mmol) gegeben. Es wurde 2 Stunden lang bei Raumtemperatur gerührt, eingeengt und säulenchromatographisch an Kieselgel (Eluent: *tert*-Butylmethylether / Cyclohexan 1:2) gereinigt. Man erhielt das Produkt in einer Ausbeute von 180 mg (37%).

### Beispielverbindung 5:

### (4-Brom-phenyl)carbaminsäure-5-oxo-2,3,4,5-tetrahydro-benzo[b]thiepin-4-ylmethyl-ester

Zu einer Lösung von 4-Hydroxymethyl-3,4-dihydro-2H-benzo[b]thiepin-5-on (224 mg, 1.08 mmol) in 14 ml Tetrahydrofuran wurden bei Raumtemperatur Triethylamin (0.16 ml, 1.18 mmol) und 4-Bromphenyl-isocyanat (0.423 g, 2.15 mmol) gegeben. Es wurde 2 Stunden lang bei Raumtemperatur gerührt, eingeengt und säulenchromatographisch an Kieselgel (Eluent: *tert*-Butylmethylether / Cyclohexan 1:2) gereinigt. Man erhielt das Produkt in einer Ausbeute von 250 mg (57%).

### Beispielverbindung 6:

### Cyclohexyl-carbaminsäure-4-hydroxymethyl-5-oxo-2,3,4,5-tetrahydro-benzo[b]thiepin-4-ylmethyl-ester

Zu einer Lösung von 4,4-Bis-hydroxymethyl-3,4-dihydro-2H-benzo[b]thiepin-5-on (424 mg, 1.78 mmol) in 22 ml Tetrahydrofuran wurden bei 0°C Triethylamin (0.27 ml, 1.96 mmol) und Cyclohexyl-isocyanat (1.0 ml, 7.84 mmol) gegeben. Es wurde 18 Stunden lang bei 80°C gerührt, eingeengt und säulenchromatographisch an Kieselgel (Eluent: *tert*-Butylmethylether / Cyclohexan 1:2) gereinigt. Man erhielt das Produkt in einer Ausbeute von 366 mg (57%).

### Beispielverbindung 7:

### Cyclohexyl-carbaminsäure-5-oxo-2,3,4,5-tetrahydro-benzo[b]thiepin-4-ylmethyl-ester

Zu einer Lösung von 4-Hydroxymethyl-3,4-dihydro-2H-benzo[b]thiepin-5-on (283 mg, 1.36 mmol) in 17 ml Tetrahydrofuran wurden bei Raumtemperatur Triethylamin (0.21 ml, 1.5 mmol) und Cyclohexyl-isocyanat (682 µl, 5.56 mmol) gegeben. Es wurde 24 Stunden lang bei 80°C gerührt, eingeengt und säulenchromatographisch an Kieselgel (Eluent: *tert*-Butylmethylether / Cyclohexan 1:2) gereinigt. Man erhielt das Produkt in einer Ausbeute von 190 mg (42%).

### Beispielverbindung 8:

### Cyclohexyl-carbaminsäure-6-(cyclohexyl-carbamoyloxymethyl)-5-oxo-6,7,8,9-tetrahydro-5H-benzocyclohepten-6-ylmethyl-ester

Zu einer Lösung von 6,6-Bis-hydroxymethyl-6,7,8,9-tetrahydro-benzocyclohepten-5-on (455 mg, 2.07 mmol) in 26 ml Tetrahydrofuran wurden bei Raumtemperatur triethylamin (0.31 ml, 2,27 mmol) und Cyclohexylisocyanat (1.76 g, 14.35 mmol) gegeben. Es wurde 24 Stunden lang bei 80°C gerührt, eingeengt und säulenchromatographisch an Kieselgel (Eluent: *tert*-Butylmethylether / Cyclohexan 1:2) gereinigt. Man erhielt das Produkt in einer Ausbeute von 130 mg (13%).

### Beispielverbindung 9:

### Phenyl-thiocarbaminsäure-O-(4-hydroxymethyl-5-oxo-2,3,4,5-tetrahydro-benzo[b]thiepin-4-ylmethyl)-ester

Zu einer Lösung von 4,4-Bis-hydroxymethyl-3,4-dihydro-2H-benzo[b]thiepin-5-on (252 mg, 1.06 mmol) in 13 ml Tetrahydrofuran wurden bei Raumtemperatur Triethylamin (0.16 ml, 1.16 mmol) und Phenyl-isothiocyanat (277 µl, 2,33 mmol) gegeben. Es wurde 36 Stunden lang bei 80°C gerührt, eingeengt und säulenchromatographisch an Kieselgel (Eluent: Diisopropylether / Hexan 4:1) gereinigt. Man erhielt das Produkt in einer Ausbeute von 48 mg (12%).

### Beispielverbindung 10:

### N-(4-methyl-phenylsulfonyl)-carbaminsäure-5-oxo-2,3,4,5-tetrahydro-benzo[b]thiepin-4-ylmethylester

Zu einer Lösung von 4-Hydroxymethyl-3,4-dihydro-2H-benzo[b]thiepin-5-on (300 mg, 1.44 mmol) in 7 ml Tetrahydrofuran wurden bei Raumtemperatur Triethylamin (0.22 ml, 1.6 mmol) und p-Toluolsulfonyl-isocyanat (312 mg, 1.586 mmol) gegeben. Es wurde 1.5 Stunden lang bei Raumtemperatur gerührt, eingeengt und säulenchromatographisch an Kieselgel (Eluent: *tert*.Butylmethylether / Dichlormethan 1:9) gereinigt. Man erhielt das Produkt in einer Ausbeute von 250 mg (43%).

### Beispielverbindung 11:

### Naphthalin-1-yl-thiocarbaminsäure-o-(4-hydroxymethyl-5-oxo-2,3,4,5-tetrahydro-benzo[b]thiepin-4-ylmethyl)-ester

Zu einer Lösung von 4,4-Bis-hydroxymethyl-3,4-dihydro-2H-benzo[b]thiepin-5-on (476 mg, 2.0 mmol) in 15 ml Tetrahydrofuran wurden bei Raumtemperatur Triethylamin (0.31 ml, 2.2 mmol) und 1-NaphthyL-isothiocyanat (407 mg, 2.2 mmol) gegeben. Es wurde 24 Stunden lang zum Rückfluß erhitzt, eingeengt und säulenchromatographisch an Kieselgel (Eluent: *tert*-Butylmethyfether / Dichlormethan 1:19) gereinigt. Man erhielt das Produkt in einer Ausbeute von 87 mg (9%).

### Beispielverbindung 12:

### Pentyl-carbaminsäure-4-hydroxymethyl-5-oxo-2,3,4,5-tetrahydro-benzo[b]oxepin-4-ylmethyl-ester

Zu einer Lösung von 4,4-Bis-hydroxymethyl-3,4-dihydro-2H-benzo[b]oxepin-5-on (260 mg, 1.17 mmol) in 15 ml Tetrahydrofuran wurden bei Raumtemperatur Triethylamin (0.18 ml, 1.3 mmol) und n-Pentyl-isocyanat (332 µl, 2.57 mmol) gegeben. Es wurde 24 Stunden lang zum Rückfluß erhitzt, eingeengt und säulenchromatographisch an Kieselgel (Eluent: Ethylacetat / Hexan 1:2) gereinigt. Man erhielt das Produkt in einer Ausbeute von 210 mg (54%).

### Beispielverbindungen 13 und 14:

### Pentyl-carbaminsäure-4-hydroxymethyl-5-oxo-2,3,4,5-tetrahydro-benzo[b]thiepin-4-ylmethyl-ester und Pentyl-carbaminsäure-4-(pentylcarbamoyloxymethyl)-5-oxo-2,3,4,5-tetrahydro-benzo[b]thiepin-4-ylmethyl-ester

Zu einer Lösung von 4,4-Bis-hydroxymethyl-3,4-dihydro-2H-benzo[b]thiepin-5-on (264 mg, 1.11 mmol) in 14 ml Tetrahydrofuran wurden bei Raumtemperatur Triethylamin (0.17 ml, 1.2 mmol) und n-Pentyl-isocyanat (314 µl, 2,44 mmol) gegeben. Es wurde 30 Stunden lang zum Rückfluß erhitzt, eingeengt und säulenchromatographisch an Kieselgel (Eluent: Ethylacetat / Hexan 1:2) gereinigt. Man erhielt Pentyl-carbaminsäure-4-hydroxymethyl-5-oxo-2,3,4,5-tetrahydro-benzo[b]thiepin-4-ylmethyl-ester (Beispiel 13) in einer Ausbeute von 200 mg (51 %) und Pentyl-carbaminsäure-4-(pentylcarbamoyloxymethyl)-5-oxo-2,3,4,5-tetrahydro-benzo[b]thiepin-4-ylmethyl-ester (Beispiel 14) in einer Ausbeute von 188 mg (37%).

### Beispielverbindung 15:

### Phenyl-thiocarbaminsäure-O-(4-hydroxymethyl-5-oxo-2,3,4,5-tetrahydro-benzo[b]oxepin-4-ylmethyl)-ester

Zu einer Lösung von 4,4-Bis-hydroxymethyl-3,4-dihydro-2H-benzo[b]oxepin-5-on (500 mg, 2.25 mmol) in 28 ml Tetrahydrofuran wurden bei Raumtemperatur Triethylamin (0.34 ml, 2.5 mmol) und Phenyl-isothiocyanat (672 µl, 5.63 mmol) gegeben. Es wurde 72 Stunden lang zum Rückfluß erhitzt, eingeengt und säulenchromatographisch an Kieselgel (Eluent: *tert*-Butylmethylether / Cyclohexan 1:1) gereinigt. Man erhielt das Produkt in einer Ausbeute von 149 mg (19%).

### Beispielverbindung 16:

### (2,4-Difluor-phenyl-carbaminsäure)-4-(2,4-difluor-phenylcarbamoyloxymethyl)-5-oxo-2,3,4,5-tetrahydro-benzo[b]oxepin-4-ylmethyl-ester

Zu einer Lösung von 4,4-Bis-hydroxymethyl-3,4-dihydro-2H-benzo[b]oxepin-5-on (399 mg, 1.795 mmol) in 22 ml Tetrahydrofuran wurden bei 0°C Triethylamin (0.2.7 ml, 1.98 mmol) und 2,4-Difluorphenyl-isocyanat (535 µl, 4.49 mmol) gegeben. Es wurde 18 Stunden lang bei Raumtemperatur gerührt, eingeengt und säulenchromatographisch an Kieselgel (Eluent: *tert*-Butylmethylether / Cyclohexan 1:2) gereinigt. Man erhielt das Produkt in einer Ausbeute von 110 mg (12%).

### Beispielverbindung 17:

### (3-Trifluormethyl-phenyl)-thiocarbaminsäure-O-(4-hydroxymethyl-5-oxo-2,3,4,5-tetrahydro-benzo[b]thiepin-4-ylmethyl)-ester

Zu einer Lösung von 4,4-Bis-hydroxymethyl-3,4-dihydro-2H-benzo[b]thiepin-5-on (555 mg, 2.33 mmol) in 29 ml Tetrahydrofuran wurden bei Raumtemperatur Triethylamin (0.35 ml, 2.56 mmol) und 3-(Trifluormethyl)phenyl-isothiocyanat (885 µl, 5.82 mmol) gegeben. Es wurde 72 Stunden lang zum Rückfluß erhitzt, eingeengt und säulenchromatographisch an Kieselgel (Eluent: Ethylacetat / Hexan 1:2) gereinigt. Man erhielt das Produkt in einer Ausbeute von 320 mg (31%).

### Beispielverbindung 18:

### (3-Trifluormethyl-phenyl)-thiocarbaminsäure-O-(4-hydroxymethyl-5-oxo-2,3,4,5-tetrahydro-benzo[b]oxepin-4-ylmethyl)-ester

Zu einer Lösung von 4,4-Bis-hydroxymethyl-3,4-dihydro-2H-benzo[b]oxepin-5-on (510 mg, 2.3 mmol) in 29 ml Tetrahydrofuran wurden bei Raumtemperatur Triethylamin (0.35 ml, 2.5 mmol) und 3-(Trifluormethyl)phenyl-isothiocyanat (872 µl, 5.74 mmol) gegeben. Es wurde 72 Stunden lang zum Rückfluß erhitzt, eingeengt und säulenchromatographisch an Kieselgel (Eluent: Ethylacetat / Hexan 1:2) gereinigt. Man erhielt das Produkt in einer Ausbeute von 483 mg (50%).

### Beispielverbindung 19:

### Benzoyl-carbaminsäure-4-benzoyl-carbamoyloxymethyl-5-oxo-2,3,4,5-tetrahydro-benzo[b]oxepin-4-ylmethyl-ester

Zu einer Lösung von 4,4-Bis-hydroxymethyl-3,4-dihydro-2H-benzo[b]oxepin-5-on (400 mg, 1.8 mmol) in 22 ml Tetrahydrofuran wurden bei Raumtemperatur Triethylamin (0.27 ml, 1.98 mmol) und Benzoyl-isothiocyanat (678 µl. 5.4 mmol) gegeben. Es wurde 7 Tage lang bei Raumtemperatur gerührt, eingeengt und säulenchromatographisch an Kieselgel (Eluent: Ethylacetat / Hexan 1:1) gereinigt. Man erhielt das Produkt in einer Ausbeute von 393 mg (42%).

### Beispielverbindung 20:

### (2,4-Difluor-phenyl)-carbaminsäure-4-hydroxymethyl-5-oxo-2,3,4,5-tetrahydro-benzo[b]thiepin-4-ylmethyl-ester

Zu einer Lösung von 4,4-Bis-hydroxymethyl-3,4-dihydro-2H-benzo[b]thiepin-5-on (100 mg, 0.42 mmol) in 5 ml Tetrahydrofuran wurde bei Raumtemperatur 2,4-Difluorphenyl-isocyanat (124 µl, 1.05 mmol) gegeben. Es wurde 72 Stunden lang bei Raumtemperatur gerührt, eingeengt und säulenchromatographisch an Kieselgel (Eluent: Ethylacetat / Hexan 1:2) gereinigt. Man erhielt das Produkt in einer Ausbeute von 50 mg (30%).

### Beispielverbindung 21:

### (2,4-Difluor-phenyl)-thlocarbaminsäure-O-(4-hydroxymethyl-5-oxo-2,3,4,5-tetrahydro-benzo[b]thiepin-4-ylmethyl)-ester

Zu einer Lösung von 4,4-Bis-hydroxymethyl-3,4-dihydro-2H-benzo[b]thiepin-5-on (720 mg, 3.02 mmol) in 38 ml Tetrahydrofuran wurden bei Raumtemperatur Triethylamin (0.46 ml, 3.32 mmol) und 2,4-Difluorphenyl-isothiocyanat (985 µl, 7.55 mmol) gegeben. Es wurde 72 Stunden lang zum Rückfluß erhitzt, eingeengt und säulenchromatographisch an Kieselgel (Eluent: Ethylacetat / Hexan 1:2) gereinigt. Man erhielt das Produkt in einer Ausbeute von 170 mg (14%).

### Beispielverbindung 22:

### (3-Trifluormethyl-phenyl)-thiocarbaminsäure-O-(6-hydroxymethyl-5-oxo-6,7,8,9-tetrahydro-5H-benzocyclohepten-6-ylmethyl)-ester

Zu einer Lösung von 6,6-Bis-hydroxymethyl-6,7,8,9-tetrahydro-benzocyclohepten-5-on (500 mg, 2.27 mmol) in 28 ml Tetrahydrofuran wurden bei Raumtemperatur Triethylamin (0.35 ml, 2.5 mmol) und 3-(Trifluormethyl)phenyl-isothiocyanat (862 µl, 5.68 mmol) gegeben. Es wurde 72 Stunden lang zum Rückfluß erhitzt, eingeengt und säulenchromatographisch an Kieselgel (Eluent: Ethylacetat / Hexan 1:2) gereinigt. Man erhielt das Produkt in einer Ausbeute von 284 mg (30%).

### Beispielverbindung 23:

### (2,4-Difluor-phenyl)-thiocarbaminsäure-O-(4-hydroxymethyl-5-oxo-2,3,4,5-tetrahydro-benzo[b]oxepin-4-ylmethyl)-ester

Zu einer Lösung von 4,4-Bis-hydroxymethyl-3,4-dihydro-2H-benzo[b]oxepin-5-on (500 mg, 2.25 mmol) in 28 ml Tetrahydrofuran wurden bei Raumtemperatur Triethylamin (0.34 ml, 2.48 mmol) und 2,4-Difluorphenyl-isothiocyanat (734 µl, 5.63 mmol) gegeben. Es wurde 72 Stunden lang zum Rückfluß erhitzt, eingeengt und säulenchromatographisch an Kieselgel (Eluent: *tert*-Butylmethylether / Cyclohexan 1:2) gereinigt. Man erhielt das Produkt in einer Ausbeute von 376 mg (43%).

### Beispielverbindung 24:

### (2,4-Difluoro-phenyl)-thiocarbaminsäure-O-(6-hydroxymethyl-5-oxo-6,7,8,9-tetrahydro-5H-benzocyclohepten-6-ylmethyl)-ester

Zu einer Lösung von 6,6-Bis-hydroxymethyl-6,7.8,9-tetrahydro-benzocyclohepten-5-on (500 mg, 2.27 mmol) in 28 ml Tetrahydrofuran wurden bei Raumtemperatur Triethylamin (0.35 ml, 2.50 mmol) und 2,4-Difluorphenyl-isothiocyanat (740 µl, 5.68 mmol) gegeben. Es wurde 72 Stunden lang zum Rückfluß erhitzt, eingeengt und säulenchromatographisch an Kieselgel (Eluent: *tert*-Butylmethylether / Cyclohexan 1:2) gereinigt. Man erhielt das Produkt in einer Ausbeute von 234 mg (26%).

### Beispielverbindung 25:

### (3-Trifluormethyl-phenyl)-carbaminsäure-4-(3-trifluormethyl-phenyl)-carbamoyloxymethyl-5-oxo-2,3,4,5-tetrahydro-benzo[b]oxepin-4-ylmethyl-ester

Zu einer Lösung von 4,4-Bis-hydroxymethyl-3,4-dihydro-2H-benzo[b]oxepin-5-on (500 mg, 2.25 mmol) in 28 ml Tetrahydrofuran wurden bei Raumtemperatur Triethylamin (0.34 ml, 2.48 mmol) und 3-(Trifluormethyl)phenyl-isocyanat (944 µl, 6.75 mmol) gegeben. Es wurde 18 Stunden lang bei Raumtemperatur gerührt, eingeengt und säulenchromatographisch an Kieselgel (Eluent: *tert*-Butylmethylether /Cyclohexan 1:2) gereinigt. Man erhielt das Produkt in einer Ausbeute von 35 mg (2,6%),

### Beispielverbindungen 26 und 27:

### Butyl-carbaminsäure-4-hydroxymethyl-5-oxo-2,3,4,5-tetrahydro-benzo[b]thiepin-4-ylmethyl-ester und Butyl-carbaminsäure-4-(butylcarbamoyloxymethyl)-5-oxo-2,3,4,5-tetrahydro-benzo[b]thiepin-4-ylmethyl-ester

Zu einer Lösung von 4,4-Bis-hydroxymethyl-3,4-dihydro-2H-benzo[b]thiepin-5-on (500 mg, 2.098 mmol) in 26 ml Tetrahydrofuran wurden bei Raumtemperatur Triethylamin (0.32 ml, 2.31 mmol) und n-Butyl-isocyanat (542 µl, 4.62 mmol) gegeben. Es wurde 24 Stunden lang zum Rückfluß erhitzt, eingeengt und säulenchromatographisch an Kieselgel (Eluent: Ethylacetat / Hexan 1:2) gereinigt. Man erhielt die beiden Produkte in einer Ausbeute von 265 mg (37%, Beispiel 26) beziehungsweise 381 mg (42%, Beispiel 27).

### Beispielverbindung 28:

### 4-Trifluoromethoxy-phenyl)-carbaminsäure-4-hydroxymethyl-5-oxo-2,3,4,5-tetrahydro-benzo[b]thiepin-4-ylmethyl-ester

Zu einer Lösung von 4,4-Bis-hydroxymethyl-3,4-dihydro-2H-benzo[b]thiepin-5-on (500 mg. 2.098 mmol) in 26 ml Tetrahydrofuran wurden bei Raumtemperatur Triethylamin (0.32 ml, 2.31 mmol) und 4-Trifluormethoxy-phenyl-isocyanat (696 µl, 4.62 mmol) gegeben. Es wurde 1 Stunde zum Rückfluß erhitzt und 72 Stunden lang bei RT gerührt, eingeengt und säulenchromatographisch an Kieselgel (Eluent: Ethylacetat / Hexan 1:4) gereinigt. Man erhielt das Produkt in einer Ausbeute von 238 mg (26%).

### Pharmakologische Daten

Die Affinität der erfindungsgemäßen substituierten benzokondensierten Cycloheptanon-Derivate zum Vanilloid-Rezeptor 1 (VR1/TRPV1-Rezeptor) bzw. zum Cannabinoid-Rezeptor CB1 und zum Cannabinoid-Rezeptor CB2 wurde wie vorstehend beschrieben bestimmt.

Die erfindungsgemäßen substituierten benzokondensierten Cycloheptanon-Derivate weisen eine hohe Affinität zu den genannten Rezeptoren auf.

| | **VR1 (Mensch) (% Hemmung im Vgl. zu 10 uM CP)** | **VR1 (Ratte) (% Stimulation im Vgl. zu 10 µM CP)** | **VR1 (Ratte (% Hemmung im Vgl. zu 10 µM CP)** | **inhibierung CB1 (10 µM, % Hemmung)** | **Inhibierung CB2 (10 µM, % Hemmung)** |
|---|---|---|---|---|---|
| **1** | | | | | 42,2 |
| **2** | | | | 42 | 74,6 |
| **5** | 53,8 | | | | 36,2 |
| **6** | 26,7 | | | | 53,6 |
| **8** | | | | 47 | 76,2 |
| **9** | 51,3 | | 52,8 | | 28,3 |
| **14** | | | | | 61,8 |
| **16** | | | | | 63,4 |
| **17** | 17,1 | | 46,6 | | 3,8 |
| **18** | 31,5 | | 52,1 | | 15,6 |
| **19** | 13,3 | | 32,2 | | 40,3 |
| **20** | 21,8 | | 17,1 | | 41,0 |
| **21** | 36,3 | | 34,6 | | |
| **22** | 67,5 | 77,9 | 59,7 | | |
| **25** | 62 | 105 | 64,7 | | |

## Patentansprüche

1. Substituierte benzokondensierte Cycloheptanon-Derivate der allgemeinen Formel I, 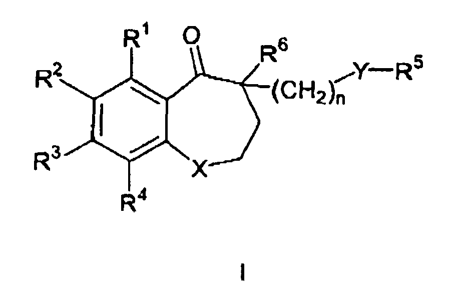 worin
n gleich 1, 2 oder 3 ist;
X für CH₂, O, S, S(=O), S(=O)₂, N(H), N(R⁷), N[C(=O)-R⁸] oder N[C(=O)- O-R⁹] steht;
Y für O, O-C(=O), O-C(=O)-O, O-S(=O)₂, O-C(=O)-N(H), O-C(=S)-N(H), N(H)-C(=O)-N(H) oder N(H)-C(=S)-N(H) steht; wobei das Atom, das an den Rest R⁵ bindet, immer zuletzt angegeben ist;
R¹, R², R³ und R⁴, unabhängig voneinander, jeweils
für H, F, Cl, Br,1, -SF₅, -CN, -NC, -NO₂, -SO₃H, -NH₂, -OH, -SH, -OR¹⁰, -SR¹¹, -NR¹²R¹³, -NH-R¹⁴, -NH-C(=O)-R¹⁵, -NR¹⁶-C(=O)-R¹⁷, - C(=O)-NH₂, -C(=O)-NH-R¹⁸, -C(=O)-NR¹⁹R²⁰, -C(=O)-H, -C(=O)-R²¹, - C(=O)-OH, -C(=O)-OR²², -O-C(=O)-R²³ oder für einen linearen oder verzweigten, gesättigten oder ungesättigten, ggf. substituierten C₁₋₁₀ aliphatischen Rest stehen;
R⁵ für eine -C(=O)-R²⁴-Gruppe steht;
für eine -S(=O)₂-R²⁵-Gruppe steht;
für einen ungesättigten oder gesättigten, ggf. substituierten 3-, 4-, 5-, 6-, 7-, 8- oder 9-gliedrigen cycloaliphatischen Rest;
für einen linearen oder verzweigten, gesättigten oder ungesättigten, ggf. substituierten C₁₋₁₀ aliphatischen Rest;
oder für einen ggf. substituierten 5- bis 14-gliedrige Aryl- oder Heteroaryl-Rest, der mit einem gesättigten oder ungesättigten, ggf. substituierten mono- oder polyzyklischen Ringsystem kondensiert und/oder über eine -(CH₂)-, -(CH₂)-(CH₂)- oder-(CH₂)-(CH₂)-(CH₂)- Gruppe gebunden sein kann, steht;
R⁶ für einen Wasserstoff-Rest;
für -(CH₂)p-Z-R²⁶ mit p = 1, 2 oder 3;
oder für -(CH₂)_{q}-OR²⁷ mit q = 1, 2 oder 3 steht;
R⁷, R⁸ und R⁹, unabhängig voneinander, jeweils
für einen linearen oder verzweigten, gesättigten oder ungesättigten, ggf. substituierten C₁₋₁₀ aliphatischen Rest;
oder für einen ggf. substituierten 5- bis 14-gliedrigen Aryl- oder Heteroaryl-Rest, der über eine -(CH₂)-. -(CH₂)-(CH₂)- oder-(CH₂)- (CH₂-(CH₂)-Gruppe ist, stehen;
R¹⁰, R¹¹, R¹², R¹³, R¹⁴, R¹⁵, R¹⁶, R¹⁷, R¹⁸, R¹⁹, R²⁰, R²¹, R²² und R²³, unabhängig voneinander, jeweils
für einen linearen oder verzweigten, gesättigten oder ungesättigten, ggf. substituierten C₁₋₁₀aliphatischen Rest
für einen ungesättigten oder gesättigten, ggf. substituierten 3-, 4-, 5-, 6-, 7-, 8- oder 9-gliedrigen cycloaliphatischen Rest;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine -(CH₂)-, -(CH₂)-(CH₂)- oder -(CH₂-(CH₂-(CH₂)- Gruppe gebunden sein kann, stehen;
Z für O, O-C(=O), O-C(=O)-O, O-S(=O)₂, O-C(=O)-N(H). O-C(=S)-N(H). N(H), N(H)-C(=O)-N(H) oder N(H)-C(=S)-N(H) steht; wobei das Atom, das an den Rest R²⁶ bindet, immer zuletzt angegeben ist;
R²⁴, R²⁵, R²⁸ und R²⁹, unabhängig voneinander, jeweils für
für einen ungesättigten oder gesättigten, ggf. substituierten 3-, 4-, 5-, 6-, 7-, 8- oder 9-gliedrigen cycloaliphatischen Rest;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
R²⁶ für eine -C(=O)-R²⁸-Gruppe steht;
für eine -S(=O)₂R²⁹-Gruppe steht;
für einen ungesättigten oder gesättigten, ggf. substituierten 3-, 4-, 5-, 6-, 7-, 8- oder 9-gliedrigen cycloaliphatischen Rest;
für einen linearen oder verzweigten, gesättigten oder ungesättigten, ggf. substituierten C₁₋₁₀ aliphatischen Rest;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine -(CH₂)-, -(CH₂)-(CH₂)- oder -(CH₂)-(CH₂-(CH₂)- Gruppe gebunden sein kann, steht;
und
R²⁷ für einen Wasserstoff-Rest steht;
wobei
die vorstehend genannten C₁₋₁₀aliphatischen Reste jeweils ggf. mit 1, 2, 3, 4, 5, 6, 7, 8 oder 9 Substituenten unabhängig voneinander ausgewählt aus der Gruppe bestehend aus F, CI, Br, 1, -CN, -NO₂, -OH, -SH und -NH₂ substituiert sein können;
die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, l, -CN, -CF₃, -SF₅, -OH, -O-C₁₋₅-Alkyl, -NH₂, -NO₂, -0-CF₃, -S-CF₃, -SH, -S-C₁₋₅-Alkyl, -C₁₋₁₀-Alkyl, -C(=O)-OH, -C(=O)-O-C₁₋₅-Alkyl. - O-C(=O)C₁₋₅-Alkyl, -NH-C₁₋₅-Alkyl, -N(C₁₋₅-Alkyl)2, -NH-C(=O)-O-C₁₋₅-Alkyl, - C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, - (CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, l, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₆-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -0-Phenyl, -O-Benzyl, Phenyl und Benzyl substituiert sein können, wobei jeweils der zyklische Teil der Reste Pyridinyl, Cyclopentyl, Cyclohexyl, Pyridazinyl, - S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklische Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
die vorstehend genannten cycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -0-C₁₋₅-Alkyl, -NH₂, -NO₂, -O-CF₃, -S-CF₃, -SH, -S-C₁₋₅-Alkyl. -C₁₋₅-Alkyl. -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, - NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)2, -S(=O)₂-C₁₋₅-Alkyl, -S(=O)₂-Pheny), -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, dass** n gleich 1 ist.

3. Verbindungen gemäß Anspruch 1 oder 2, **dadurch gekennzeichnet, dass** Y für O, O-C(=O), O-C(=O)-O, O-S(=O)₂, O-C(=S)-N(H) oder O-C(=O)-N(H) steht.

4. Verbindungen gemäß einem oder mehreren der Ansprüche 1 bis 3, **dadurch gekennzeichnet, dass**
R¹, R², R³ und R⁴, unabhängig voneinander, jeweils
für H, F, Cl, Br, I, -SF₅, -CN, -NC, -NO₂, -SO₃H, -NH₂, -OH, -SH, -OR¹⁰, -SR¹¹, -NR¹²R¹³, -NH-R¹⁴, -NH-C(=O)-R¹⁵, -C(=O)-OH, -C(=O)-OR²², - O-C(=O)-R²³
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH. -SH und -NH₂ substituiert sein kann.

5. Verbindungen gemäß einem oder mehreren der Ansprüche 1 bis 4, **dadurch gekennzeichnet, dass**
R⁵ für eine -C(=O)-R²⁴-Gruppe steht;
für eine -S(=O)₂-R²⁵-Gruppe steht;
für einen Rest ausgewählt aus der Gruppe bestehend aus Methyl, Ethyl, n-Propyl, Isopropyl, n-Butyl, sec-Butyl, Isobutyl, tert-Butyl, (1,1)~Dimethyl-propyl, n-Pentyl, sec-Pentyl, n-Hexyl und n-Heptyl steht; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht; wobei der Rest ggf. jeweils mit 1, 2, 3, 4 oder 5 Substituenten unabhängig voneinander ausgewählt aus der Gruppe bestehend aus Oxo (=O), Thioxo (=S), F, CI, Br, I, -CN, -CF₃, -SF₅, -OH, -0-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂. -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃ und -S(=O)₂-C₂H₅ substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest über eine -CH₂)-, -(CH₂)-(CH₂)- oder - (CH₂)-(CH₂)-(CH₂)-Gruppe gebunden und ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(==O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -0-Benzyl, Phenyl und Benzyl substituiert sein kann; wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann.

6. Verbindungen gemäß einem oder mehreren der Ansprüche 1 bis 5, **dadurch gekennzeichnet**, das
R⁶ für einen Wasserstoff-Rest;
für -(CH₂)ₚZ-R²⁶
oder für -(CH₂)-OR²⁷ steht.

7. Verbindungen gemäß einem oder mehreren der Ansprüche 1 bis 6, **dadurch gekennzeichnet, dass**
R⁷, R⁸ und R⁹, unabhängig voneinander, jeweils
für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen; wobei der Rest ggf. jeweils mit 1, 2, 3, 4 oder 5 Substituenten unabhängig voneinander ausgewählt aus der Gruppe bestehend aus F, Cl, Br, I, -CN, -NO₂. -OH, -SH und -NH₂ substituiert sein kann;
oder für einen Rest ausgewählt aus der Gruppe bestehend aus Phenyl, Naphthyl, Thiophenyl, Furanyl, Pyridinyl, Thiazolyl und Oxazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, - CF₃, -SF₅, -OH, -O-CH₃, -O-C₂H₅, -NH₂, -NO₂, -O-CF₃, -S-CF₃, -SH, -S-CH₃, - S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -0-C(=O)-CH₃. -O-C(=OFC₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃ substituiert sein kann und über eine -(CH₂)-, -(CH₂)-(CH₂)- oder --(CH₂)-(CH₂)-(CH₂)-Gruppe gebunden Ist.

8. Verbindungen gemäß einem oder mehreren der Ansprüche 1 bis 7, **dadurch gekennzeichnet, dass**
R¹⁰, R¹¹, R¹², R¹³, R¹⁴, R¹⁵, R¹⁶, R¹⁷, R¹⁸, R¹⁹, R²¹, R²¹, R²² und R²³, unabhängig voneinander, jeweils
für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl stehen;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl. Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl stehen, wobei der Rest über eine -(CH₂)-, -(CH₂)-(CH₂)- oder-(CH₂)-(CH₂)-(CH₂)-Gruppe gebunden und ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)_{2,} -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂ substituiert sein kann.

9. Verbindungen gemäß einem oder mehreren der Ansprüche 1 bis 8, **dadurch gekennzeichnet, dass**
R²⁴, R²⁵, R²⁸ und R²⁹, unabhängig, voneinander, jeweils
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothlophenyl, Pyrrolidinyl, Piperidinyl, Morpholinyl, Piperazinyl und Thiomorpholinyl stehen;
oder für einen Rest ausgewählt aus der Gruppe bestehend aus Phenyl, Naphthyl, (1,3)-Benzodioxolyl, (1,4)-Benzodloxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl stehen, wobei der Rest ggf. jeweils mit 1, 2, 3, 4 oder 5 Substituenten unabhängig voneinander ausgewählt aus der Gruppe bestehend aus F, Cl, Br, 1, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(-O)-O-C(CH₃)₃ -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂ substituiert sein kann.

10. Verbindungen gemäß einem oder mehreren der Ansprüche 1 bis 9, **dadurch gekennzeichnet, dass**
R²⁶ für eine -C(=O)-R²⁸-Gruppe steht;
für eine -S(=O)₂-R²⁹-Gruppe steht;
für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht; wobei der Rest ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -0-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, - C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(-O)-CH₃, -C(=O)-C₂H₅, -C(=O)-C(CH₃)₃, -C(=O)NH₂, -C(=O)-NH-CH₃, -C(=O)-NH-C₂H₅, -C(=O)-N-(CH₃)₂, -C(=O)-N-(C₂H₅)₂, -S(=O)₂-CH₃ und -S(=O)₂-C₂H₅ substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest über eine -(CH₂)-, -(CH₂)-(CH₂)- oder-(CH₂)-(CH₂)-(CH₂)-Gruppe gebunden und ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, - C(=O)-N-(C₂H₅)₂, -S(=O)₂-CH₃, -S(=O)₂-C₂H₅, -S(=O)₂-Phenyl, -NH-S(=O)₂-CH₃, -NH-S(=O)₂-C₂H₅, -S(=O)₂-NH-CH₃, -S(=O)₂-NH-C₂H₅, Cyclohexyl, Cyclopentyl, Pyridinyl, Pyridazinyl, -(CH₂)-Benzo[b]furanyl, -O-Phenyl, -0-Benzyl, Phenyl und Benzyl substituiert sein kann; wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann.

11. Verbindungen gemäß einem oder mehreren der Ansprüche 1 bis 10, **dadurch gekennzeichnet, dass**
n gleich 1 ist;
X für CH₂, O, S, S(=O), S(=O)₂, N(H), N(R⁷), N[C(=O)-R⁸] oder N[C(=O)- O-R⁹] steht;
Y für O, O-C(=O), O-C(=O)-O, O-S(=O)₂, O-C(=S)-N(H) oder O-C(=O)- N(H) steht;
R¹, R², R³ und R⁴, unabhängig voneinander, jeweils
für H, F, Cl, Br, I, -SF₅, -CN, -NC, -NO₂, -OH, -SH, -OR¹⁰, -SR¹¹, -NR¹²R¹³,
oder für einen Rest ausgewählt aus der Gruppe bestehend aus Methyl, -CF₃, -CHF₂, -CH₂F, -CCl₃, -CBr₃, -CH₂-CN, -CH₂-NO₂, Ethyl, -CF₂-CF₃, -CH₂-CF₃, -CCl₂-CCl₃, -CF₂-CH₃, -CH₂-CH₂-CN, -CH₂-CH₂-NO₂, n- Propyl, -CF₂-CF₂-CF₃, -CH₂-CH₂-CH₂-CN, -CH₂-CH₂-CH₂-NO₂, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen;
R⁵ für eine -C(=O)-R²⁴-Gruppe steht;
für eine -S(=O)₂-R²⁵-Gruppe steht;
für einen Rest ausgewählt aus der Gruppe bestehend aus Methyl, Ethyl, n-Propyl, Isopropyl, n-Butyl, sec-Butyl, Isobutyl, tert-Butyl, (1,1)- Dimethyl-propyl, n-Pentyl, sec-Pentyl, n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cyctoheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht;
oder für einen Rest ausgewählt aus der Gruppe bestehend aus Phenyl, Benzyl, Phenethyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest über eine - (CH₂)-, -(CH₂)-(CH₂)- oder -(CH₂)-(CH₂)-(CH₂)-Gruppe gebunden und ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S- C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O- C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-C(=O)-O-CH₃, -NH-C(=O)-O-C₂H₅, - NH-C(=O)-O-C(CH₃)₃, -C(=O)-H, -C(=O)-CH₃, -C(=O)-C₂H₅, -C(=O)- C(CH₃)₃, -C(=O)-NH₂, -C(=O)-NH-CH₃, -C(=O)-NH-C₂H₅, -C(=O)-N- (CH₃)₂, -C(-O)-N-(C₂H₅)2, -S(=O)₂-CH₃, -S(=O)₂-C₂H₅, -NH-S(=O)₂-CH₃ -NH-S(=O)₂-C₂H₅, -S(=O)₂-NH-CH₃ und -S(=O)₂-NH-C₂H₅ substituiert sein kann;
R⁶ für einen Wasserstoff-Rest;
für -(CH₂)-Z-R²⁶;
oder für -(CH₂)-OR²⁷ steht;
R⁷, R⁸ und R⁹, unabhängig voneinander, jeweils
für einen Rest ausgewählt aus der Gruppe bestehend aus Methyl, Ethyl, n-Propyl, Isopropyl, n-Butyl, sec-Butyl, Isobutyl, tert-Butyl, (1,1)- Dimethyl-propyl, n-Pentyl, sec-Pentyl, n-Hexyl und n-Heptyl stehen;
oder für einen Rest ausgewählt aus der Gruppe bestehend aus Benzyl und Phenethyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n- Propyl, Isopropyl, n-Butyl, sec-Butyl, Isobutyl und tert-Butyl substituiert sein kann;
R¹⁰, R¹¹, R¹² und R¹³, unabhängig voneinander, jeweils
für einen Rest ausgewählt aus der Gruppe bestehend aus Methyl, -CF₃, -CHF₂, -CH₂F, -CCl₃, -CBr₃, -CH₂-CN, -CH₂-NO₂, Ethyl, -CF₂-CF₃, -CH₂- CF₃, -CCl₂-CCl₃, -CF₂-CH₃, -CH₂-CH₂-CN, -CH₂-CH₂-NO₂, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen;
oder für einen Rest ausgewählt aus der Gruppe bestehend aus Phenyl, Benzyl und Phenethyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O- C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl und tert-Butyl substituiert sein kann;
Z für O, O-C(=O), O-C(=O)-O, O-S(=O)₂, O-C(=S)-N(H) oder O-C(=O)- N(H) steht;
R²⁴ und R²⁵, unabhängig voneinander, jeweils
für einen Rest ausgewählt aus der Gruppe bestehend aus Phenyl, Naphthyl, Thiophenyl, Furanyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S- C₂H₅, Methyl, Ethyl, n-Propyl, Isopropyl, n-Butyl, sec-Butyl, Isobutyl, tert-Butyl, -N(CH₃)₂, -N(C₂H₅)₂, -NH-CH₃ und -NH-C₂H₅ substituiert sein kann;
R²⁶ für eine -C(=O)-R²⁸-Gruppe steht;
für eine -S(=O)₂-R²⁹-Gruppe steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl und Thiomorpholinyl steht;
für einen Rest ausgewählt aus der Gruppe bestehend aus Methyl, Ethyl, n-Propyl, Isopropyl, n-Butyl, sec-Butyl, Isobutyl, tert-Butyl, (1,1)- Dimethyl-propyl, n-Pentyl, sec-Pentyl, n-Hexyl und n-Heptyl steht;
oder für einen Rest ausgewählt aus der Gruppe bestehend aus Phenyl, Benzyl, Phenethyl, Naphthyl, (1,3)-Benzodioxolyl, (1,4)-Benzodioxanyl, Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl und Benzothiazolyl steht, wobei der Rest über eine - (CH₂)-, -(CH₂)-(CH₂)- oder -(CH₂)-(CH₂)-(CH₂)-Gruppe gebunden und ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S- C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O- C(CH₃)₃, -O-C(=O)-CH₃, -O-C(=O)-C₂H₅. -O-C(=O)-C(CH₃)₃, -N(CH₃)₂, - N(C₂H₅)₂, -NH-CH₃, -NH-C₂H₅, -NH-C(=O)-O-CH₃, -NH-C(=O)-O-C₂H₅, - NH-C(=O)-O-C(CH₃)₃, -C(=O)-H, -C(=O)-CH₃, -C(=O)-C₂H₅, -C(=O)- C(CH₃)₃, -C(=O)-NH₂, -C(=O)-NH-CH₃, -C(=O)-NH-C₂H₅, -C(=O)-N- (CH₃)₂, -C(=O)-N-(C₂H₅)₂, -S(=O)₂-CH₃, -S(=O)₂-C₂H₅, -NH-S(=O)₂-CH₃, -NH-S(=O)₂-C₂H₅, -S(=O)₂-NH-CH₃ und -S(=O)₂-NH-C₂H₅ substituiert sein kann;
R²⁷ für einen Wasserstoff-Rest steht;
und
R²⁸ und R²⁹, unabhängig voneinander, jeweils
für einen Rest ausgewählt aus der Gruppe bestehend aus Phenyl, Naphthyl, Thiophenyl, Furanyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S- C₂H₅, Methyl, Ethyl, n-Propyl, Isopropyl, n-Butyl, sec-Butyl, Isobutyl, tert-Butyl, -N(CH₃)₂, -N(C₂H₅)₂, -NH-CH₃ und -NH-C₂H₅ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

12. Verbindungen gemäß einem oder mehreren der Ansprüche 1 bis 11, **dadurch gekennzeichnet, dass**
n gleich 1 ist;
X für CH₂, O, S, S(=O) oder S(=O)₂ steht;
Y für O, O-C(=O), O-C(=O)-O, O-S(=O)₂, O-C(=S)N(H) oder O-C(=O)- N(H) steht;
R¹, R², R³ und R⁴, unabhängig voneinander, jeweils
für H, F, Cl, Br, -SF₅, -OH, -OR¹⁰, -SR¹¹, -NR¹²R¹³,
oder für einen Rest ausgewählt aus der Gruppe bestehend aus Methyl, -CF₃, -CHF₂, -CH₂F, -CCl₃, -CBr₃, -CH₂-CN, -CH₂-NO₂, Ethyl, -CF₂-CF₃, -CH₂-CF₃, n-Propyl, Isopropyl, n-Butyl, sec-Butyl, Isobutyl, tert-Butyl, (1,1)-Dimethyl-propyl und n-Pentyl stehen;
R⁵ für eine -C(=O)-R²⁴-Gruppe steht;
für eine -S(=O)₂-R²⁵-Gruppe steht;
für einen Rest ausgewählt aus der Gruppe bestehend aus Methyl, Ethyl, n-Propyl, Isopropyl, n-Butyl, sec-Butyl, Isobutyl, tert-Butyl, (1,1)- Dimethyl-propyl, n-Pentyl, sec-Pentyl, n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl und Cycloheptenyl steht;
oder für einen Rest ausgewählt aus der Gruppe bestehend aus Phenyl, Naphthyl, Thiophenyl, Furanyl, Pyridinyl, Indolyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, -SF₅, -CF₃, -O-CH₃, -O-C₂H₅, -SCH₃, -SC₂H₅, -O-CF₃, -S-CF₃, Methyl, Ethyl, n-Propyl, Isopropyl, n-Butyl, sec-Butyl, Isobutyl und tert- Butyl substituiert sein kann;
R⁶ für einen Wasserstoff-Rest;
für -(CH₂)-Z-R²⁶;
oder für -(CH₂)-OR²⁷ steht;
R¹⁰, R¹¹, R¹² und R¹³, unabhängig voneinander, jeweils
für einen Rest ausgewählt aus der Gruppe bestehend aus Methyl, -CF₃, -CH₂F, -CF₂H, Ethyl, -C₂F₅, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
Z für O, O-C(=O), O-C(=O)-O, O-S(=O)₂, O-C(=S)-N(H) oder O-C(=O)- N(H) steht;
R²⁴ und R²⁵, unabhängig voneinander, jeweils
für einen Rest ausgewählt aus der Gruppe bestehend aus Phenyl, Naphthyl, Thiophenyl, Furanyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CF₃, -SF₅, -O- CH₃, -O-C₂H₅, -S-CF₃, Methyl, Ethyl, n-Propyl, Isopropyl, n-Butyl, sec- Butyl, Isobutyl und tert-Butyl substituiert sein kann;
R²⁶ für eine -C(=O)-R²⁸-Gruppe steht;
für eine -S(=O)₂-R²⁹-Gruppe steht;
für einen Rest ausgewählt aus der Gruppe bestehend aus Methyl, Ethyl, n-Propyl, Isopropyl, n-Butyl, sec-Butyl, Isobutyl, tert-Butyl, (1,1)- Dimethyl-propyl, n-Pentyl, sec-Pentyl, n-Hexyl und n-Heptyl steht;
für einen Rest ausgewählt aus der Gruppe bestehend aus Cyclopropyl, Cyclobutyl, Cyclopentyl, Cyclohexyl, Cycloheptyl, Cyclopentenyl, Cyclohexenyl und Cycloheptenyl steht;
oder für einen Rest ausgewählt aus der Gruppe bestehend aus Phenyl, Naphthyl, Thiophenyl, Furanyl, Pyridinyl, Indolyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, -SF₅, -CF₃, -O-CH₃, -O-C₂H₅, -SCH₃, -SC₂H₅, -O-CF₃, -S-CF₃, Methyl, Ethyl, n-Propyl, Isopropyl, n-Butyl, sec-Butyl, Isobutyl und tert- Butyl substituiert sein kann;
R²⁷ für einen Wasserstoff-Rest steht;
und
R²⁸ und R²⁹, unabhängig voneinander, jeweils
für einen Rest ausgewählt aus der Gruppe bestehend aus Phenyl, Naphthyl, Thiophenyl, Furanyl und Pyridinyl stehen, wobei der Rest ggf. jeweils mit 1, 2, 3, 4 oder 5 Substituenten unabhängig voneinander ausgewählt aus der Gruppe bestehend aus F, Cl, Br, I, -CF₃, -SF₅, -O- CH₃, -O-C₂H₅, -S-CF₃, Methyl, Ethyl, n-Propyl, Isopropyl, n-Butyl, sec- Butyl, Isobutyl und tert-Butyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

13. Verbindungen gemäß einem oder mehreren der Ansprüche 1 bis 12 ausgewählt aus der Gruppe bestehend aus
[1] Pentyl-carbaminsäure-5-oxo-2,3,4,5-tetrahydro-benzo[b]thiepin-4- ylmethyl-ester
[2] Phenyl-carbaminsäure-4-(phenyl-carbamoyloxymethyl)-5-oxo-2,3,4,5- tetrahydro-benzo[b]thiepin-4-ylmethyl-ester
[3] Phenyl-carbaminsäure-5-oxo-2,3,4,5-tetrahydro-benzo[b]thiepin-4- ylmethyl-ester
[4] (3-Trifluormethyl-phenyl)-carbaminsäure-5-oxo-2,3,4,5-tetrahydro- benzo[b]thiepin-4-ylmethyl-ester
[5] (4-Brom-phenyl)-carbaminsäure-5-oxo-2,3,4,5-tetrahydro- benzo[b]thiepin-4-ylmethyl-ester
[6] Cyclohexyl-carbaminsäure-4-hydroxymethyl-5-oxo-2,3,4,5-tetrahydro- benzo[b]thiepin-4-ylmethyl-ester
[7] Cyclohexyl-carbaminsäure-5-oxo-2,3,4,5-tetrahydro-benzo[b]thiepin-4- ylmethyl-ester
[8] Cyclohexyl-carbaminsäure-6-(cyclohexyl-carbamoyloxymethyl)-5-oxo- 6,7,8,9-tetrahydro-5H-benzocyclohepten-6-ylmethyl-ester
[9] Phenyl-thiocarbaminsäure-O-(4-hydroxymethyl-5-oxo-2,3,4,5- tetrahydro-benzo[b]thiepin-4-ylmethyl)-ester
[10] N-(4-methyl-phenylsulfonyl)-carbaminsäure-5-oxo-2,3,4,5-tetrahydro- benzo[b]thiepin-4-ylmethyl-ester
[11] Naphthalin-1-yl-thiocarbaminsäure-O-(4-hydroxymethyl-5-oxo-2,3,4,5- tetrahydro-benzo[b]thiepin-4-ylmethyl)-ester
[12] Pentyl-carbaminsäure-4-hydroxymethyl-5-oxo-2,3,4,5-tetrahydro- benzo[b]oxepin-4-ylmethyl-ester
[13] Pentyl-carbaminsäure-4-hydroxymethyl-5-oxo-2,3,4,5-tetrahydro- benzo[b]thiepin-4-ylmethyl-ester
[14] Pentyl-carbaminsäure-4-(pentylcarbamoyloxymethyl)-5-oxo-2,3,4,5- tetrahydro-benzo[b]thiepin-4-ylmethyl-ester
[15] Phenyl-thiocarbaminsäure-O-(4-hydroxymethyl-5-oxo-2,3,4,5- tetrahydro-benzo[b]oxepin-4-ylmethyl)-ester
[16] (2,4-Difluor-phenyl-carbaminsäure)-4-(2,4-difluor- phenylcarbamoyloxymethyl)-5-oxo-2,3,4,5-tetrahydro-benzo[b]oxepin-4- ylmethyl-ester
[17] (3-Trifluormethyl-phenyl)-thiocarbaminsäure-O-(4-hydroxymethyl-5-oxo- 2,3,4,5-tetrahydro-benzo[b]thiepin-4-ylmethyl)-ester
[18] (3-Trifluormethyl-phenyl)-thiocarbaminsäure-O-(4-hydroxymethyl-5-oxo- 2,3,4,5-tetrahydro-benzo[b]oxepin-4-ylmethyl)-ester
[19] Benzoyl-carbaminsäure-4-benzoyl-carbamoyloxymethyl-5-oxo-2,3,4,5- tet1°ahydro-berlzo[b]oxepin-äylmethyl-ester
[20] (2,4-Difluor-phenyl)-carbaminsäure-4-hydroxymethyl-5-oxo-2,3,4,5- tetrahydro-benzo[b]thiepin-4-ylmethyl-ester
[21] (2,4-Difluor-phenyl)-thiocarbaminsäure-O-(4-hydroxymethyl-5-oxo- 2,3,4,5-tetrahydro-benzo[b]thiepin-4-ylmethyl)-ester
[22] (3-Trifluormethyl-phenyl)-thiocarbaminsäure-O-(6-hydroxymethyl-5-oxo- 6,7,8,9-tetrahydro-5H-benzocyclohepten-6-ylmethyl)-ester
[23] (2,4-Difluor-phenyl)-thiocarbaminsäure-O-(4-hydroxymethyl-5-oxo- 2,3,4,5-tetrahydro-benzo[b]oxepin-4-ylmethyl)-ester
[24] (2,4-Difluoro-phenyl)-thiocarbaminsäure-O-(6-hydroxymethyl-5-oxo- 6,7,8,9-tetrahydro-5H-benzocyclohepten-6-ylmethyl)-ester
[25] (3-Trifluormethyl-phenyl)-carbaminsäure-4-(3-trifluormethyl-phenyl)- carbamoyloxymethyl-5-oxo-2,3,4,5-tetrahydro-benzo[b]oxepin-4- ylmethyl-ester
[26] Butyl-carbaminsäure-4-hydroxymethyl-5-oxo-2,3,4,5-tetrahydro- benzo[b]thiepin-4-ylmethyl-ester
[27] Butyl-carbaminsäure-4-(butylcarbamoyloxymethyl)-5-oxo-2,3,4,5- tetrahydro-benzo[b]thiepin-4-ylmethyl-ester
und
[28] (4-Trifluoromethoxy-phenyl)-carbaminsäure-4-hydroxymethyl-5-oxo- 2,3,4,5-tetrahydro-benzo[b]thiepin-4-ylmethyl-ester;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

14. Arzneimittel enthaltend wenigstens eine Verbindung gemäß einem oder mehreren der Ansprüche 1 bis 13 und ggf. einen oder mehrere physiologisch verträgliche Hilfsstoffe.

15. Arzneimittel gemäß Anspruch 14 zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

16. Arzneimittel gemäß Anspruch 14 zur Prophylaxe und/oder Behandlung von einer oder mehreren Erkrankungen ausgewählt aus der Gruppe bestehend aus Migräne; Depressionen; Harninkontinenz; Stressinkontinenz; überaktive Blase (overactive bladder, OAB); Husten; Osteoporose; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; oder zur Prophylaxe und/oder Behandlung von Alkohol- und/oder Drogen- und/oder Medikamentenmißbrauch und Alkohol- und/oder Drogen- und/oder Nikotin-und/oder Medikamentenabhängigkeit, vorzugsweise zur Prophylaxe und/oder Verminderung von Entzugserscheinungen bei Alkohol- und/oder Drogen-und/oder Nikotin- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

17. Verwendung wenigstens einer Verbindung gemäß einem oder mehreren der Ansprüche 1 bis 13 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

18. Verwendung wenigstens einer Verbindung gemäß einem oder mehreren der Ansprüche 1 bis 13 zur Herstellung eines Arzneimittels zur Prophylaxe und/oder Behandlung von einer oder mehreren Erkrankungen ausgewählt aus der Gruppe bestehend aus Migräne; Depressionen: Harninkontinenz; Stressinkontinenz; überaktive Blase (overacitve bladder, OAB); Husten; Osteoporose; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; zur Prophylaxe und/oder Behandlung von Alkohol- und/oder Drogen- und/oder Medikamentenmißbrauch und Alkohol- und/oder Drogen- und/oder Nikotin- und/oder Medikamentenabhängigkeit, vorzugsweise zur Prophylaxe und/oder Verminderung von Entzugserscheinungen bei Alkohol- und/oder Drogen- und/oder Nikotin- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

## Claims

1. Substituted benzofused cycloheptanone derivatives of the general formula I 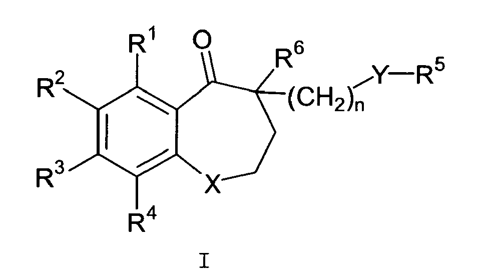 in which
n is 1, 2 or 3;
X is CH₂, O, S, S(=O), S(=O)₂, N(H), N(R⁷), N[C(=O)-R⁸] or N[C(=O)-O-R⁹];
Y is O, O-C(=O), O-C(=O)-O, O-S(=O)₂, O-C(=O)- N(H), O-C(=S)-N(H), N(H)-C(=O)-N(H) or N(H)- C(=S)-N(H); where the atom which binds to R⁵ is always stated last;
R¹, R², R³ and R⁴ are each independently
H, F, Cl, Br, I, -SF₅, -CN, -NC, -NO₂, -SO₃H, -NH₂, -OH, -SH, -OR¹⁰, -SR¹¹, -NR¹²R¹³, -NH-R¹⁴, -NH-C(=O)-R¹⁵, -NR¹⁶-C(=O)-R¹⁷, -C(=O)-NH₂, - C(=O)-NH-R¹⁸, -C(=O)-NR¹⁹R²⁰, -C(=O)-H, -C(=O)- R²¹, -C(=O)-OH, -C(=O)-OR²², -O-C(=O)-R²³ or a linear or branched, saturated or unsaturated, optionally substituted C₁₋₁₀ aliphatic radical;
R⁵ is a -C(=O)-R²⁴ group;
is a -S(=O)₂-R²⁵ group;
is an unsaturated or saturated, optionally substituted 3-, 4-, 5-, 6-, 7-, 8- or 9-membered cycloaliphatic radical;
is a linear or branched, saturated or unsaturated, optionally substituted C₁₋₁₀ aliphatic radical;
or is an optionally substituted 5- to 14-membered aryl or heteroaryl radical which may be fused to a saturated or unsaturated, optionally substituted mono- or polycyclic ring system and/or may be bonded via a -(CH₂)-, -(CH₂)-(CH₂)- or -(CH₂ -(CH₂)-(CH₂)- group;
R⁶ is a hydrogen radical;
is - (CH₂) p-Z-R²⁶ where p = 1, 2 or 3;
or is -(CH₂)_{q}-OR²⁷ where q = 1, 2 or 3;
R⁷, R⁸ and R⁹ are each independently
a linear or branched, saturated or unsaturated, optionally substituted C₁₋₁₀ aliphatic radical;
or an optionally substituted 5- to 14- membered aryl or heteroaryl radical which is bonded via a -(CH₂)-, -(CH₂)-(CH₂)- or -(CH₂)- (CH₂)-(CH₂)- group;
R¹⁰,R¹¹,R¹²,R¹³,R¹⁴,R¹⁵,R¹⁶,R¹⁷,R¹⁸,R¹⁹,R²⁰, R²¹R²² and R²³ are each independently
a linear or branched, saturated or unsaturated, optionally substituted C₁₋₁₀ aliphatic radical;
an unsaturated or saturated, optionally substituted 3-, 4-, 5-, 6-, 7-, 8- or 9-membered cycloaliphatic radical;
or an optionally substituted 5- to 14- membered aryl or heteroaryl radical which may be fused to a saturated or unsaturated, optionally substituted mono- or polycyclic ring system and/or may be bonded via a - (CH₂)-, -(CH₂)-(CH₂)- or -(CH₂)-(CH₂)-(CH₂)- group;
Z is O, O-C(=O), O-C(=O)-O, O-S(=O)₂, O-C(=O)- N(H), O-C(=S)-N(H), N(H), N(H)-C(=O)-N(H) or N(H)-C(=S)-N(H); where the atom which binds to the R²⁶ radical is always stated last;
R²⁴, R²⁵, R²⁸ and R²⁹ are each independently
an unsaturated or saturated, optionally substituted 3-, 4-, 5-, 6-, 7-, 8- or 9-membered cycloaliphatic radical;
or an optionally substituted 5- to 14- membered aryl- or heteroaryl radical which may be fused to a saturated or unsaturated, optionally substituted mono- or polycyclic ring system;
R²⁶ is a -C(=O)-R²⁸ group;
is a -S(=O)₂-R²⁹ group;
is an unsaturated or saturated, optionally substituted 3-, 4-, 5-, 6-, 7-, 8- or 9-membered cycloaliphatic radical;
is a linear or branched, saturated or unsaturated, optionally substituted C₁₋₁₀ aliphatic radical;
or an optionally substituted 5- to 14-membered aryl or heteroaryl radical, which may be fused to a saturated or unsaturated, optionally substituted mono- or polycyclic ring system and/or may be bonded via a -(CH₂)-, -(CH₂)- (-CH₂)- or -(CH₂)-(CH₂)-(CH₂)- group;
and
R²⁷ is a hydrogen radical;
where
the aforementioned C₁₋₁₀ aliphatic radicals may each optionally be substituted by 1, 2, 3, 4, 5, 6, 7, 8 or 9 substituents selected independently from the group consisting of F, Cl, Br, I, -CN, -NO₂, - OH, -SH and -NH₂;
the aforementioned aryl or heteroaryl radicals may each optionally be substituted by 1, 2, 3, 4 or 5 substituents selected independently from the group consisting of F, Cl, Br, I, -CN, -CF₃, -SF₅, -OH, - O-C₁₋₅-alkyl, -NH₂, -NO₂, -O-CF₃, -S-CF₃, -SH, -S-C₁₋₅-alkyl, -C₁₋₁₀-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, C(=O)-N-(C₁₋₅-alkyl)₂, -S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, - (CH₂)-benzo[b]furanyl, -O-phenyl, -O-benzyl, phenyl and benzyl, where the cyclic moiety of the pyridinyl, cyclopentyl, cyclohexyl, pyridazinyl, - S(=O)₂-phenyl, -O-phenyl, -O-benzyl, phenyl, - (CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and the aforementioned heteroaryl radicals may each optionally have 1, 2, 3, 4 or 5 heteroatom(s) selected independently from the group consisting of oxygen, nitrogen and sulfur as ring member(s);
the rings of the aforementioned mono- or polycyclic ring systems may each be optionally substituted by 1, 2, 3, 4 or 5 substituents selected independently from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-OH, -C(=O)-O-C₁₋₅-alkyl, -O-C(=O)-C₁₋₅-alkyl, -NH-C₁₋₅-alkyl, - N(C₁₋₅-alkyl)₂, -NH-C(=O)-O-C₁₋₅-alkyl, -C(=O)-H, - C (=O) -C₁₋₅-alkyl, -C (=O) -NH₂, -C (=O) -NH-C₁₋₅-alkyl, C (=O) -N- (C₁₋₅-alkyl)₂, -S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, - (CH₂)-benzo[b]furanyl, -O-phenyl, -O-benzyl, phenyl and benzyl, where the cyclic moiety of the pyridinyl, cyclopentyl, cyclohexyl, pyridazinyl, -S(=O)₂-phenyl, -O-phenyl, -O-benzyl, phenyl, - (CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, - NO₂, -C₁₋₅-alkyl, -O-C₁₋₅-alkyl, -O-CF₃, -S-CF₃, phenyl and -O-benzyl,
and the rings of the aforementioned mono- or polycyclic ring systems are each 5-, 6- or 7-membered and may each optionally have 1, 2, 3, 4 or 5 heteroatom(s) as ring member(s) which are independently selected from the group consisting of oxygen, nitrogen and sulfur;
the aforementioned cycloaliphatic radicals may each optionally be substituted by 1, 2, 3, 4 or 5 substituents selected independently from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-OH, -C (=O) -O-C₁₋₅-alkyl, -O-C (=O) -C₁₋₅-alkyl, -NH-C_{1- 5}-alkyl, -N(C₁₋₅-alkyl)₂, -NH-C(=O)-O-C₁₋₅-alkyl, C(=O)-H, -C(=O)-C₁₋₅-alkyl, -C(=O)-NH₂, -C(=O)-NH-C₁₋₅-alkyl, C(=O)-N-(C₁₋₅-alkyl)₂, -S(=O)₂-C₁₋₅-alkyl, -S(=0)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -0-benzyl, phenyl and benzyl, where the cyclic moiety of the pyridinyl, cyclopentyl, cyclohexyl, pyridazinyl, -S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, - NO₂, -C₁₋₅-alkyl, -O-C₁₋₅-alkyl, -O-CF₃, -S-CF₃, phenyl and -O-benzyl,
and the aforementioned cycloaliphatic radicals may each optionally have 1, 2, 3, 4 or 5 heteroatom(s) selected independently from the group consisting of oxygen, nitrogen and sulfur as ring member(s);
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

2. Compounds according to Claim 1, **characterized in that** n is 1.

3. Compounds according to Claim 1 or 2, **characterized in that** Y is O, O-C(=O), O-C(=O)-O, O-S(=O)₂, O-C(=S)-N(H) or O-C(=O)-N(H).

4. Compounds according to one or more of Claims 1 to 3, **characterized in that**
R¹, R², R³ and R⁹ are each independently
H, F, Cl, Br, I, -SF₅, -CN, -NC, -NO₂, -SO₃H, -NH₂, -H, -SH, -OR¹⁰, -SR¹¹, -NR¹²R¹³, -NH-R¹⁴, -NH-C(=O)-R¹⁵, -C(=O-OH, -C(=O)-OR²², -O-C(=O)-R²³
or a radical selected form the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂.

5. Compounds according to one or more of Claims 1 to 4, **characterized in that**
R⁵ is a -C(=O)-R²⁴ group;
is a -S(=O)₂-R²⁵ group;
is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl; where the radical may optionally be substituted by 1, 2, 3, 4 or 5 substituents selected independently from the group consisting of F, Cl, Br, I, -CN, -NO₂, - OH, -SH and -NH₂;
is a radical selected from the group consisting of cyclopropyl,
cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl and thiomorpholinyl; where the radical may in each case optionally be substituted by 1, 2, 3, 4 or 5 substituents selected independently from the group consisting of oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C (=O) -OH, -C (=O) -O-CH₃, -C(=O)-O-C₂H₅, - C (=O) -O-C (CH₃)₃, -O-C (=O) -CH₃, -O-C (=O)-C₂H₅, -O-C (=O) -C (CH₃)₃, -N (CH₃)₂, -N (C₂H₅)₂, -NH-CH₃, -NH-C₂H₅, -NH-C (=O) -O-CH₃, -NH-C (=O) -O-C₂H₅, -NH-C(=O)-O-C(CH₃)₃, -C(=O)-H, -C(=O)-CH₃, -C(=O)-C2H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃ and - S(=O)₂-C₂H₅,
or is a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, where the radical may be bonded via a -(CH₂)-, -(CH₂)-(CH₂)- or -(CH₂)-(CH₂)-(CH₂)- group and may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C (=O) -O-CH₃, -C(=O)-O-C₂H₅, -C(=O)-O-C(CH₃)₃, - O-C(=O)-CH₃, -O-C(=O)-C₂H₅, -O-C(=O)-C(CH₃)₃), - N(CH₃)₂, -N(C₂H₅)₂, -NH-CH₃, -NH-C₂H₅, -NH-C(=O)-O-CH₃, -NH-C (=O) -O-C₂H₅, -NH-C (=O) -O-C (CH₃)₃, -C (=O) - H, -C(=O)-CH₃, -C(=O)-C₂H₅, -C(=O)-C(CH₃)₃, -C(=O) - NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -0-phenyl, -O-benzyl, phenyl and benzyl where the cyclic moiety of the pyridinyl, cyclopentyl, cyclohexyl, pyridazinyl, -S(=O)₂-phenyl, -O-phenyl, -O-benzyl, phenyl, -(CH₂)-benzo[b]furanyl and benzyl radicals may in each case optionally be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C2Hs, -O-CF₃, -S-CF₃, phenyl and -O-benzyl.

6. Compounds according to one or more of Claims 1 to 5, **characterized in that**
R⁶ is a hydrogen radical;
is -(CH₂)ₚZ-R²⁶
or is - (CH₂) -OR²⁷.

7. Compounds according to one or more of Claims 1 to 6, **characterized in that**
R⁷, R⁸ and R⁹ are each independently
a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂;
or a radical from the group consisting of phenyl, naphthyl, thiophenyl, furanyl, pyridinyl, thiazolyl and oxazolyl, where the radical may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, - SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, - C(=O)-O-C (CH₃)₃, -O-C(=O)-CH₃, -O-C(=O)-C₂H₅, -O-C(=O)-C(CH₃)₃, -N(CH₃)₂, -N (C₂H₅)₂, -NH-CH₃, -NH-C₂H₅, -NH-C(=O)-O-CH₃, -NH-C(=O) -O-C₂H₅, -NH-C(=O)-O-C(CH₃)₃, -C(=O)-H, -C(=O)-CH₃, -C(=O)-C₂H₅, -C(=O)-C(CH₃)₃, and may be bonded via a -(CH₂)-, (CH₂)-(CH₂) - or - (CH₂) - (CH₂) - (CH₂) - group.

8. Compounds according to one or more of Claims 1 to 7, **characterized in that**
R¹⁰, R¹¹, R¹², R¹³, R¹⁴, R¹⁵, R¹⁶, R¹⁷, R¹⁸, R¹⁹, R²⁰, R²¹, R²² and R²³ are each independently
a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂;
a radical selected from the group consisting of cyclopropyl,
cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl and thiomorpholinyl;
or a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, where the radical may be bonded via a -(CH₂)-, -(CH₂)-(CH₂)- or -(CH₂)-(CH₂)-(CH₂)- group and may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C (=O) -O-CH₃, -C (=O) -O-C₂H₅, -C(=O)-O-C(CH₃)₃, - O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O) -N- (CH₃)₂, -C (=O) -N- (C₂H₅)₂.

9. Compounds according to one or more of Claims 1 to 8, **characterized in that**
R²⁹, R²⁵, R²⁸ and R²⁹ are each independently
a radical selected from the group consisting of cyclopropyl,
cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl and thiomorpholinyl;
or a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, where the radical may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C (=O) -O-C₂H₅, -C(=O)-O-C (CH₃)₃, -O-C(=O)-CH₃, -O-C(=O)-C₂H₅, -O-C(=O)-C(CH₃)₃, -N(CH₃)₂, -N(C₂H₅)₂, - NH-CH₃, -NH-C₂H₅, -NH-C(=O)-O-CH₃, -NH-C(=O)-O-C₂H₅, -NH-(=O)-O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, - C(=O)-NH-C₂H₅, -C(=O)-N-(CH₃)₂, -C(=O)-N-(C₂H₅)₂.

10. Compounds according to one or more of Claims 1 to 9, **characterized in that**
R²⁶ is a -C(=O)-R²⁸ group;
is a -S(=O)₂-R²⁹ group;
is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂;
is a radical selected from the group consisting of cyclopropyl,
cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl and thiomorpholinyl; where the radical may in each case optionally be substituted by 1, 2, 3, 4 or 5 substituents selected independently from the group consisting of oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, - C(=O)-O-C(CH₃)₃, -O-C(=O)-CH₃, -O-C(=O)-C₂H₅, -O-C(=O)-C(CH₃)₃, -N(CH₃)₂, -N (C₂H₅)₂, -NH-CH₃, -NH-C₂H₅, -NH-C (=O) -O-CH₃, -NH-C (=O) -O-C₂H₅, -NH-C (=O) -O-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃ and - S (=O)₂-C₂H₅;
or a radical selected from the group consisting of phenyl, naphthyl, (1,3)-benzodioxolyl, (1,4)-benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, where the radical may be bonded via a -(CH₂)-, -(CH₂)-(CH₂)-or -(CH₂)-(CH₂)-(CH₂)- group and may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, - O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O) - NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -S(=O)₂-phenyl, -NH-S(=O)₂-CH₃, -NH-S(=O)₂-C₂H₅, -S(=O)₂-NH-CH₃, -S(=O)₂-NH-C₂H₅, cyclohexyl, cyclopentyl, pyridinyl, pyridazinyl, -(CH₂)-benzo[b]furanyl, -0-phenyl, -O-benzyl, phenyl and benzyl; where the cyclic moiety of the pyridinyl, cyclopentyl, cyclohexyl, pyridazinyl, -S(=O)₂-phenyl, -0-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl.

11. Compounds according to one or more of Claims 1 to 10, **characterized in that**
n is 1;
X is CH₂, O, S, S(=O), S(=O)₂, N(H), N(R⁷), N[C(=O)-R⁸] or N[C(=O)-O-R⁹];
Y is O, O-C(=O) , O-C(=O)-O, O-S(=O)₂, O-C(=S)- N(H) or O-C(=O)-N(H);
R¹, R², R³ and R⁴ SH, -OR¹⁰, are each independently H, F, Cl, Br, I, -SF₅, -CN, -NC, -NO₂, -OH, - SR¹¹, -NR¹²R¹³,
or a radical selected from the group consisting of methyl, -CF₃, -CHF₂, -CH₂F, CCl₃, -CBr₃, -CH₂-CN, -CH₂-NO₂, ethyl, -CF₂-CF₃, -CH₂-CF₃, -CCl₂-CCl₃, -CF₂-CH₃, -CH₂-CH₂-CN, - CH₂-CH₂-NO₂, n-propyl, -CF₂-CF₂-CF₃, -CH₂-CH₂- CH₂-CN, -CH₂-CH₂-CH₂-NO₂, isopropyl, n-butyl, sec-butyl, isobutyl, tert-butyl, 1,1- dimethylpropyl, n-pentyl, sec-pentyl, n-hexyl and n-heptyl;
R⁵ is a -C(=O)-R²⁴ group;
is a -S(=O)₂-R²⁵ group;
is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl;
is a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl and thiomorpholinyl;
or is a radical selected from the group consisting of phenyl, benzyl, phenethyl, naphthyl, (1,3)-benzodioxolyl, (1,4)- benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, where the radical may be bonded via a -(CH₂)-, -(CH₂)-(CH₂)- or -(CH₂)- (CH₂)-(CH₂)- group and may in each case optionally be substituted by 1, 2, 3, 4 or 5 substituents selected independently from the group consisting of F, Cl, Br, I, -CN, -CF₃, - SF₅, -OH, -O-CH₃, -O-C₂H₅, -NH₂, -NO₂, -O-CF₃, - S-CF₃, -SH, -S-CH₃, -S-C₂H₅, methyl, ethyl, n- propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃,- C(=O)-NH₂, -C(=O)-NH-CH₃, -C(=O)-NH-C₂H₅, -C (=O) -N- (CH₃)₂, -C(=O) -N- (C₂H₅)₂, -S(=O)₂-CH₃, -S(=O)₂-C₂H₅, -NH-S(=O)₂-CH₃, -NH-S(=O)₂-C₂H₅, - S(=O)₂-NH-CH₃ and -S(=O)₂-NH-C₂H₅;
R⁶ is a hydrogen radical;
is - (CH₂)-Z-R²⁶;
or is - (CH₂)-OR²⁷;
R⁷ R⁸ and R⁹ are each independently
a radical selected from the group consisting of methyl, ethyl, n-propyl, isopropyl, n- butyl, sec-butyl, isobutyl, tert-butyl, 1,1- dimethylpropyl, n-pentyl, sec-pentyl, n-hexyl and n-heptyl;
or a radical selected from the group consisting of benzyl and phenethyl, where the radical may in each case optionally be substituted by 1, 2, 3, 4 or 5 substituents selected independently from the group consisting of F, Cl, Br, I, -CN, -CF₃, -SF₅, - OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl and tert-butyl;
R¹⁰, R¹¹, R¹² and R¹³, are each independently
a radical selected from the group consisting of methyl, -CF₃, -CHF₂, -CH₂F, -CCl₃, -CBr₃, - CH₂-CN, -CH₂-NO₂, ethyl, -CF₂-CF₃, -CH₂-CF₃, -CCl₂-CCl₃, -CF₂-CH₃, -CH₂-CH₂-CN, -CH₂-CH₂-NO₂, n-propyl, isopropyl, n-butyl, sec-butyl, isobutyl, tert-butyl, 1,1-dimethylpropyl, n-pentyl, sec-pentyl, n-hexyl and n-heptyl;
or a radical selected from the group consisting of phenyl, benzyl and phenethyl, where the radical may in each case optionally be substituted by 1, 2, 3, 4 or 5 substituents selected independently from the group consisting of F, Cl, Br, I, -CN, -CF₃, - SF₅, -OH, -O-CH₃, -O-C₂H₅, -NH₂, -NO₂, -O-CF₃, - S-CF₃, -SH, -S-CH₃, -S-C₂H₅, methyl, ethyl, n- propyl, isopropyl, n-butyl, sec-butyl, isobutyl and tert-butyl;
Z is O, O-C(=O), O-C(=O)-O, O-S(=O)₂, O-C(=S)-N(H) or O-C(=O)-N(H);
R²⁴ and R²⁵ are each independently
a radical selected from the group consisting of phenyl, naphthyl, thiophenyl, furanyl and pyridinyl, where the radical may in each case optionally be substituted by 1, 2, 3, 4 or 5 substituents selected independently from the group consisting of F, Cl, Br, I, -CN, -CF₃, - SF₅, -OH, -O-CH₃, -O-C₂H₅, -NH₂, -NO₂, -O-CF₃, - S-CF₃, -SH, -S-CH₃, -S-C₂H₅, methyl, ethyl, n- propyl, isopropyl, n-butyl, sec-butyl, isobutyl, tert-butyl, -N(CH₃)₂, -N(C₂H₅)₂, -NH- CH₃ and -NH-C₂H₅;
R²⁶ is a -C(=O)-R²⁸ group;
is a -S(=O)₂-R²⁹ group;
is a radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl and thiomorpholinyl;
is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl; or is a radical selected from the group consisting of phenyl, benzyl, phenethyl, naphthyl, (1,3)-benzodioxolyl, (1,4)- benzodioxanyl, thiophenyl, furanyl, pyrrolyl, pyrazolyl, pyranyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, isoquinolinyl, benzimidazolinyl, benzoxazolyl, benzisoxazolyl and benzothiazolyl, where the radical may be bonded via a - (CH₂) -, -(CH₂)-(CH₂)- or -(CH₂)- (CH₂)-(CH₂)- group and may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n- propyl, isopropyl, n-butyl, sec-butyl, isobutyl, tert-butyl, -C (=O) -OH, -C (=O) -O-CH₃, -C(=O)-O-C₂H₅, -C (=O) -O-C (CH₃)₃, -O-C(=O)-CH₃, -O-C(=O)-C₂H_{5,} -O-C (=O) -C (CH₃)₃, -N(CH₃)₂, - N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -C(=O)-H, -C(=O)-CH₃, -C(=O)-C₂H₅, -C(=O -C(CH₃)₃, - C(=O)-NH₂, -C(=O)-NH-CH₃, -C (=O) -NH-C₂H₅, -C (=O) -N- (CH₃) ₂, -C (=O) -N- (C₂Hₛ) ₂, -S (=O)₂-CH₃, -S (=O)₂-C₂H₅, -NH-S(=O)₂-CH₃, -NH-S (=O)₂-C₂H₅, - S(=O)₂-NH-CH₃ and -S(=O)₂-NH-C₂H₅;
R²⁷ is a hydrogen radical;
and
R^{z$} and R²⁹ are each independently
a radical selected from the group consisting of phenyl, naphthyl, thiophenyl, furanyl and pyridinyl, where the radical may in each case optionally be substituted by 1, 2, 3, 4 or 5 substituents selected independently from the group consisting of F, Cl, Br, I, -CN, -CF₃, - SF₅, -OH, -O-CH₃, -O-C₂H₅, -NH₂, -NO₂, -O-CF₃, - S-CF₃, -SH, -S-CH₃, -S-C₂H₅, methyl, ethyl, n-propyl, isopropyl, n-butyl, sec-butyl, isobutyl, tert-butyl, -N(CH₃)₂, -N(C₂H₅)_{2,} -NH-CH₃ and -NH-C₂H₅;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

12. Compounds according to one or more of Claims 1 to 11, **characterized in that**
n is 1;
X is CH₂, O, S, S (=O) or S(=O)₂;
Y is O, O-C(=O), O-C(=O)-O, O-S(=O)₂, O-C(=S)- N(H) or O-C(=O)- N(H);
R¹, R², R³ and R⁴, are each independently H, F, Cl, Br, -SF₅, -OH, -OR¹⁰, -SR¹¹, -NR¹²R¹³, or a radical selected from the group consisting of methyl, -CF₃, -CHF₂, -CH₂F, - CCl₃, -CBr₃, -CH₂-CN, -CH₂-NO₂, ethyl, -CF₂-CF₃, -CH₂-CF₃, n-propyl, isopropyl, n-butyl, sec- butyl, isobutyl, tert-butyl, 1,1- dimethylpropyl and n-pentyl;
R⁵ is a -C(=O)-R²⁴ group;
is a -S(=O)₂-R²⁵ group;
is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl;
is a radical selected from the group consisting of cyclopropyl, cyclobutyl, cyclopentyl, cyclohexyl, cycloheptyl, cyclopentenyl, cyclohexenyl and cycloheptenyl;
or is a radical selected from the group consisting of phenyl, naphthyl, thiophenyl, furanyl, pyridinyl, indolyl, thiazolyl and oxazolyl, where the radical may in each case optionally be substituted by 1, 2, 3, 4 or 5 substituents selected independently from the group consisting of F, Cl, Br, -SF₅, -CF₃, -O- CH₃, -O-C₂H₅, -SCH₃, -SC₂H₅, -O-CF₃, -S-CF₃, methyl, ethyl, n-propyl, isopropyl, n-butyl, sec-butyl, isobutyl and tert-butyl;
R⁶ is a hydrogen radical;
is -(CH₂)-Z-R²⁶;
or is - (CH₂)-OR²⁷;
R¹⁰, R¹¹, R¹² and R¹³ are each independently
a radical selected from the group consisting of methyl, -CF₃, -CH₂F, -CF₂H, ethyl, -C₂F₅, n- propyl, isopropyl, n-butyl, sec-butyl, isobutyl, tert-butyl, 1,1-dimethylpropyl, n- pentyl, sec-pentyl, n-hexyl and n-heptyl;
Z is O, O-C (=O) O-C(=O)-O, O-S(=O)₂, O-C(=S) - N(H) or O-C(=O)-N(H);
R²⁴ and R²⁵ are each independently
a radical selected from the group consisting of phenyl, naphthyl, thiophenyl, furanyl and pyridinyl, where the radical may in each case optionally be substituted by 1, 2, 3, 4 or 5 substituents selected independently from the group consisting of F, Cl, Br, I, -CF₃, -SF₅, -O-CH₃, -O-C₂H₅, -S-CF₃, methyl, ethyl, n- propyl, isopropyl, n-butyl, sec-butyl, isobutyl and tert-butyl;
R²⁶ is a -C(=O)-R²⁸ group;
is a -S(=O)₂-R²⁹ group;
is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl;
is a radical selected from the group consisting of cyclopropyl, cyclobutyl, cyclopentyl, cyclohexyl, cycloheptyl, cyclopentenyl, cyclohexenyl and cycloheptenyl;
or is a radical selected from the group consisting of phenyl, naphthyl, thiophenyl, furanyl, pyridinyl, indolyl, thiazolyl and oxazolyl, where the radical may in each case optionally be substituted by 1, 2, 3, 4 or 5 substituents selected independently from the group consisting of F, Cl, Br, -SF₅, -CF₃, -O- CH₃, -O-C₂H₅, -SCH₃, -SC₂H₅, -O-CF₃, -S-CF₃, methyl, ethyl, n-propyl, isopropyl, n-butyl, sec-butyl, isobutyl and tert-butyl;
R²⁷ is a hydrogen radical;
and
R²⁸ and R²⁹ are each independently
a radical selected from the group consisting of phenyl, naphthyl, thiophenyl, furanyl and pyridinyl, where the radical may in each case optionally be substituted by 1, 2, 3, 4 or 5 substituents selected independently from the group consisting of F, Cl, Br, I, -CF₃, -SF₅, -O-CH₃, -O-C₂H₅, -S-CF₃, methyl, ethyl, n- propyl, isopropyl, n-butyl, sec-butyl, isobutyl and tert-butyl;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

13. Compounds according to one or more of Claims 1 to 12 selected from the group consisting of
[1] pentylcarbamic acid 5-oxo-2,3,4,5- tetrahydrobenzo[b]thiepin-4-ylmethyl ester
[2] phenylcarbamic acid 4- (phenylcarbamoyloxymethyl)-5-oxo-2,3,4,5- tetrahydrobenzo[b]thiepin-4-ylmethyl ester
[3] phenylcarbamic acid 5-oxo-2,3,4,5- tetrahydrobenzo[b]thiepin-4-ylmethyl ester
[4] (3-trifluoromethylphenyl)carbamic acid 5-oxo- 2,3,4,5-tetrahydro-benzo[b]thiepin-4-ylmethyl ester
[5] (4-bromophenyl)carbamic acid 5-oxo-2,3,4,5- tetrahydro-benzo[b]thiepin-4-ylmethyl ester
[6] cyclohexylcarbamic acid 4-hydroxymethyl-5- oxo-2,3,4,5-tetrahydro-benzo[b]thiepin-4- ylmethyl ester
[7] cyclohexylcarbamic acid 5-oxo-2,3,4,5- tetrahydrobenzo[b]thiepin-4-ylmethyl ester
[8] cyclohexylcarbamic acid 6- (cyclohexylcarbamoyloxymethyl)-5-oxo-6,7,8,9- tetrahydro-5H-benzocyclohepten-6-ylmethyl ester
[9] phenylthiocarbamic acid O-(4-hydroxymethyl-.5- oxo-2,3,4,5-tetrahydro-benzo[b]thiepin-4- ylmethyl) ester
[10] N-(4-methylphenylsulfonyl)carbamic acid 5- oxo-2,3,4,5-tetrahydro-benzo[b]thiepin-4- ylmethyl ester
[11] naphthalen-1-ylthiocarbamic acid O-(4- hydroxymethyl-5-oxo-2,3,4,5- tetrahydrobenzo[b]thiepin-4-ylmethyl) ester
[12] pentylcarbamic acid 4-hydroxymethyl-5-oxo- 2,3,4,5-tetrahydro-benzo[b]oxepin-4-ylmethyl ester
[13] pentylcarbamic acid 4-hydroxymethyl-5-oxo- 2,3,4,5-tetrahydro-benzo[b]thiepin-4-ylmethyl ester
[14] pentylcarbamic acid 4- (pentylcarbamoyloxymethyl)-5-oxo-2,3,4,5- tetrahydrobenzo[b]thiepin-4-ylmethyl ester
[15] phenylthiocarbamic acid O-(4-hydroxymethyl-5- oxo-2,3,4,5-tetrahydro-benzo[b]oxepin-4- ylmethyl) ester
[16] 2,4-difluorophenylcarbamic acid 4-(2,4- difluorophenylcarbamoyloxymethyl)-5-oxo- 2,3,4,5-tetrahydrobenzo[b]oxepin-4-ylmethyl ester
[17] (3-trifluoromethylphenyl)thiocarbamic acid O- (4-hydroxymethyl-5-oxo-2,3,4,5- tetrahydrobenzo[b]thiepin-4-ylmethyl) ester
[18] (3-trifluoromethylphenyl)thiocarbamic acid O- (4-hydroxymethyl-5-oxo-2,3,4,5- tetrahydrobenzo[b]oxepin-4-ylmethyl) ester
[19] benzoylcarbamic acid 4- benzoylcarbamoyloxymethyl-5-oxo-2,3,4,5- tetrahydrobenzo[b]oxepin-4-ylmethyl ester
[20] (2,4-difluorophenyl)carbamic acid 4- hydroxymethyl-5-oxo-2,3,4,5- tetrahydrobenzo[b]thiepin-4-ylmethyl ester
[21] (2,4-difluorophenyl)thiocarbamic acid O-(4- hydroxymethyl-5-oxo-2,3,4,5- tetrahydrobenzo[b]thiepin-4-ylmethyl) ester
[22] (3-trifluoromethylphenyl)thiocarbamic acid O- (6-hydroxymethyl-5-oxo-6,7,8,9-tetrahydro-5H- benzocyclohepten-6-ylmethyl) ester
[23] (2,4-difluorophenyl)thiocarbamic acid O-(4- hydroxymethyl-5-oxo-2,3,4,5- tetrahydrobenzo[b]oxepin-4-ylmethyl) ester
[24] (2,4-difluorophenyl)thiocarbamic acid O-(6- hydroxymethyl-5-oxo-6,7,8,9-tetrahydro-5H- benzocyclohepten-6-ylmethyl) ester
[25] (3-trifluoromethylphenyl)carbamic acid 4-(3- trifluoromethylphenyl)-carbamoyloxymethyl-5- oxo-2,3,4,5-tetrahydrobenzo[b]oxepin-4- ylmethyl ester
[26] butylcarbamic acid 4-hydroxymethyl-5-oxo- 2,3,4,5-tetrahydro-benzo[b]thiepin-4-ylmethyl ester
[27] butylcarbamic acid 4- (butylcarbamoyloxymethyl)-5-oxo-2,3,4,5- tetrahydrobenzo[b]thiepin-4-ylmethyl ester
and
[28] (4-trifluoromethoxyphenyl)carbamic acid 4- hydroxymethyl-5-oxo-2,3,4,5- tetrahydrobenzo[b]thiepin-4-ylmethyl ester;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

14. Medicament comprising at least one compound according to one or more of Claims 1 to 13 and optionally one or more physiologically compatible assistants.

15. Medicament according to Claim 14 for prophylaxis and/or treatment of pain, preferably of pain selected from the group consisting of acute pain, chronic pain, neuropathic pain and visceral pain.

16. Medicament according to Claim 14 for prophylaxis and/or treatment of one or more disorders selected from the group consisting of migraine; depressions; urine incontinence; stress incontinence; overactive bladder (OAB); coughing; osteoporosis; neurodegenerative disorders, preferably selected from the group consisting of Parkinson's disease, Huntington's disease, Alzheimer's disease and multiple sclerosis; disorders of food uptake, preferably selected from the group consisting of bulimia, anorexia, obesity and cachexia; states of anxiety; cognitive dysfunctions, preferably memory impairments; cognitive deficiencies (attention deficit syndrome, ADS); epilepsy; diarrhea and pruritus; or for prophylaxis and/or treatment of alcohol abuse and/or drug abuse and/or medicament abuse and alcohol dependence and/or drug dependence and/or nicotine dependence and/or medicament dependence, preferably for prophylaxis and/or reduction of withdrawal symptoms in the case of alcohol dependence and/or drug dependence and/or nicotine dependence and/or medicament dependence; for prophylaxis and/or reduction of an evolution of tolerance toward medicaments, especially medicaments based on opioids; for regulation of food intake; for modulation of movement activity; for regulation of the cardiovascular system; for local anesthesia; for enhancing vigilence; for enhancing libido; for diuresis and/or for antinatriuresis.

17. Use of at least one compound according to one or more of Claims 1 to 13 for producing a medicament for prophylaxis and/or treatment of pain, preferably of pain selected from the group consisting of acute pain, chronic pain, neuropathic pain and visceral pain.

18. Use of at least one compound according to one or more of Claims 1 to 13 for producing a medicament for prophylaxis and/or treatment of one or more disorders selected from the group consisting of migraine; depressions; urine incontinence; stress incontinence; overactive bladder (OAB); coughing; osteoporosis; neurodegenerative disorders, preferably selected from the group consisting of Parkinson's disease, Huntington's disease, Alzheimer's disease and multiple sclerosis; disorders of food uptake, preferably selected from the group consisting of bulimia, anorexia, obesity and cachexia; states of anxiety; cognitive dysfunctions, preferably memory impairments; cognitive deficiencies (attention deficit syndrome, ADS); epilepsy; diarrhea and pruritus; or for prophylaxis and/or treatment of alcohol abuse and/or drug abuse and/or medicament abuse and alcohol dependence and/or drug dependence and/or nicotine dependence and/or medicament dependence, preferably for prophylaxis and/or reduction of withdrawal symptoms in the case of alcohol dependence and/or drug dependence and/or nicotine dependence and/or medicament dependence; for prophylaxis and/or reduction of an evolution of tolerance toward medicaments, especially medicaments based on opioids; for regulation of food intake; for modulation of movement activity; for regulation of the cardiovascular system; for local anesthesia; for enhancing vigilence; for enhancing libido; for diuresis and/or for antinatriuresis.

## Revendications

1. Dérivés substitués benzocondensés de cycloheptanone de formule générale I, 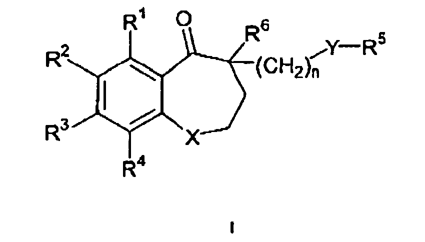 où
n vaut 1, 2 ou 3 ;
X représente CH₂, O, S, S(=O), S(=O)_{2,} N (H), N(R⁷), N[C(=O)-R⁸] ou N[C(=O)-O-R⁹];
Y représente O, O-C(=O), O-C(=O)-O, O-S(=O)₂, O- C(=O)-N(H), O-C(=S)-N(H), N(H)-C(=O)-N(H) ou N(H)- C(=S)-N(H) ; où l'atome qui se lie au radical R⁵ est toujours indiqué en dernier lieu ;
R¹ R², R³ et R⁴ représentent, indépendamment les uns des autres, à chaque fois H, F, Cl, Br, I, -SF₅, -CN, -NC, -NO₂, -SO₃H, -NH₂, -OH, -SH, -OR¹⁰, -SR¹¹, -NR¹²R¹³, -NH-R¹⁴, -NH-C(=O)-R¹⁵, -NR¹⁶-C(=O)-R¹⁷, -C(=O)-NH₂, -C (=O)-NH-R¹⁸, -C(=O)-NR¹⁹R²⁰, -C(=O)-H, -C(=O)-R²¹, -C(=O)-OH, -C(=O)-OR²², -O-C(=O)-R²³ ou un radical aliphatique en C₁₋₁₀, linéaire ou ramifié, saturé ou insaturé, le cas échéant substitué ;
R⁵ représente un groupe -C (=O) -R²⁴,
représente un groupe -S(=O)₂-R²⁵ ;
représente un radical cycloaliphatique insaturé ou saturé, le cas échéant substitué, de 3, 4, 5, 6, 7, 8 ou 9 chaînons ;
représente un radical aliphatique en C₁₋₁₀ linéaire ou ramifié, saturé ou insaturé, le cas échéant substitué ;
ou représente un radical aryle ou hétéroaryle de 5 à 14 chaînons, le cas échéant substitué, qui peut être condensé par un système monocyclique ou polycyclique, saturé ou insaturé, le cas échéant substitué et/ou lié via un groupe -(CH₂)-, -(CH₂)- (CH₂)- ou -(CH₂)-(CH₂)-(CH₂)- ;
R⁶ représente un radical hydrogène ; représente -(CH₂)ₚ-Z-R²⁶ avec p = 1, 2 ou 3 ; ou représente -(CH₂)_{q}-OR²⁷ avec q = 1, 2 ou 3 ;
R⁷, R⁸ et R⁹ représentent, indépendamment les uns des autres, à chaque fois
un radical aliphatique en C₁₋₁₀ linéaire ou ramifié, saturé ou insaturé, le cas échéant substitué ; ou un radical aryle ou hétéroaryle de 5 à 14 chaînons, le cas échéant substitué, qui est lié via un groupe -(CH₂)-, -(CH₂)-(CH₂)- ou -(CH₂)- (CH₂) - (CH₂) - ;
R¹⁰, R¹¹, R¹², R¹³, R¹⁴, R¹⁵, R¹⁶, R¹⁷, R¹⁸, R¹⁹, R²⁰, R²¹, R²² et R²³ représentent, indépendamment les uns des autres, à chaque fois
un radical aliphatique en C₁₋₁₀ linéaire ou ramifié, saturé ou insaturé, le cas échéant substitué ; un radical cycloaliphatique insaturé ou saturé, le cas échéant substitué, de 3, 4, 5, 6, 7, 8 ou 9 chaînons ;
ou un radical aryle ou hétéroaryle de 5 à 14 chaînons, le cas échéant substitué, qui peut être condensé par un système monocyclique ou polycyclique, saturé ou insaturé, le cas échéant substitué et/ou lié via un groupe -(CH₂)-, -(CH₂)- (CH₂)- ou -(CH₂)-(CH₂)-(CH₂)- ;
Z représente O, O-C(=O), O-C(=O)-O, O-S(=O)₂, O- C(=O)-N(H), O-C(=S)-N(H), N(H), N(H)-C(=O)-N(H) ou N (H) -C (=S) -N (H) ; où l'atome qui se lie au radical R²⁶ est toujours indiqué en dernier lieu ;
R²⁴, R²⁵, R²⁸ et R²⁹ représentent, indépendamment les uns des autres, à chaque fois
un radical cycloaliphatique insaturé ou saturé, le cas échéant substitué, de 3, 4, 5, 6, 7, 8 ou 9 chaînons ;
ou un radical aryle ou hétéroaryle de 5 à 14 chaînons, le cas échéant substitué, qui peut être condensé avec un système monocyclique ou polycyclique, saturé ou insaturé, le cas échéant substitué ;
R²⁶ représente un groupe -C (=O) -R²⁸,
représente un groupe -S(=O)₂-R²⁹ ;
représente un radical cycloaliphatique insaturé ou saturé, le cas échéant substitué, de 3, 4, 5, 6, 7, 8 ou 9 chaînons ;
représente un radical aliphatique en C₁₋₁₀ linéaire ou ramifié, saturé ou insaturé, le cas échéant substitué ;
ou représente un radical aryle ou hétéroaryle de 5 à 14 chaînons, le cas échéant substitué, qui peut être condensé par un système monocyclique ou polycyclique, saturé ou insaturé, le cas échéant substitué et/ou lié via un groupe -(CH₂)-, -(CH₂)- (CH₂)- ou -(CH₂)-(CH₂)-(CH₂)-
et
R²⁷ représente un radical hydrogène ;
où
les radicaux aliphatiques en C₁₋₁₀ susmentionnés peuvent, le cas échéant, à chaque fois, être substitués par 1, 2, 3, 4, 5, 6, 7, 8 ou 9 substituants, choisis indépendamment les uns des autres dans le groupe constitué par F, Cl, Br, I, -CN, -NO₂, -OH, -SH et -NH₂ ;
les radicaux aryle ou hétéroaryle susmentionnés peuvent, le cas échéant, à chaque fois, être substitués par 1, 2, 3, 4 ou 5 substituants choisis indépendamment les uns des autres dans le groupe constitué par F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₁₀-alkyle, -C (=O) -OH, -C (=O) -OC₁₋₅-alkyle, O-C (=O) -C₁₋₅-alkyle, -NH-C₁₋₅-alkyle, -N(C₁₋₅-alkyle)₂, -NH-C(=O)-O-C₁₋₅-alkyle, -C(=O)-H, -C (=O) -C₁₋₅-alkyle, -C (=O) -NH₂, -C (=O) -NH-C₁₋₅-alkyle, -C(=O)-N-(C₁₋₅-alkyle)₂, -S (=O)₂-C₁₋₅-alkyle, -S (=O) ₂-phényle, -NH-S (=O) ₂-C₁₋₅-alkyle, -S(=O)₂-NH-C₁₋₅-alkyle, cyclohexyle, cyclopentyle, pyridinyle, pyridazinyle, -(CH₂)-benzo[b]furannyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF3, -S-CF₃, phényle et -O-benzyle,
et les radicaux hétéroaryle susmentionnés peuvent présenter, le cas échéant, à chaque fois, 1, 2, 3, 4 ou 5 hétéroatome(s) comme élément(s) de cycle, qui est/sont choisi(s) indépendamment les uns des autres dans le groupe constitué par oxygène, azote et soufre ;
les cycles des systèmes monocycliques ou polycycliques susmentionnés peuvent, le cas échéant, à chaque fois, être substitués par 1, 2, 3, 4 ou 5 substituants choisis indépendamment les uns des autres dans le groupe constitué par oxo (=O), thioxo (=S) , F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₅-alkyle, -C (=O) -OH, -C(=O)-OC₁₋₅-alkyle, -O-C(=O)-C₁₋₅-alkyle, -NH-C₁₋₅-alkyle, -N(C₁₋₅-alkyle)₂, -NH-C(=O)-O-C₁₋₅-alkyle, -C(=O)-H, -C(=O)-C₁₋₅-alkyle, -C(=O)-NH₂, -C(=O)-NH-C₁₋₅-alkyle, C(=O)-N-(C₁₋₅-alkyle)₂, -S(=O)₂-C₁₋₅-alkyle, -S(=O)₂-phényle, -NH-S(=O)₂-C₁₋₅-alkyle, -S(=O)₂-NH-C₁₋₅-alkyle, cyclohexyle, cyclopentyle, pyridinyle, pyridazinyle, -(CH₂)-benzo[b]furannyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -O-benzyle,
et les cycles des systèmes monocycliques ou polycycliques susmentionnés peuvent présenter à chaque fois 5, 6 ou 7 chaînons et à chaque fois, le cas échant, 1, 2, 3, 4 ou 5 hétéroatome(s) comme élément(s) de cycle, qui sont choisis, indépendamment les uns des autres, dans le groupe constitué par oxygène, azote et soufre ;
les radicaux cycloaliphatiques susmentionnés peuvent, le cas échéant, à chaque fois, être substitués par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₅-alkyle, -C(=O)-OH, -C(=O)-O-C₁₋₅-alkyle, -O-C(=O)-C₁₋₅-alkyle, -NH-C₁₋₅-alkyle, -N(C₁₋₅-alkyle)₂, -NH-C(=O)-O-C₁₋₅-alkyle, -C(=O)-H, -C(=O)-C₁₋₅-alkyle, -C(=O)-NH₂, -C(=O)-NH-C₁₋₅-alkyle, C(=O)-N-(C₁₋₅-alkyle)₂, -S(=O)₂-C₁₋₅-alkyle, -S(=O)₂-phényle, -NH-S(=O)₂-C₁₋₅-alkyle, -S(=O)₂-NH-C₁₋₅-alkyle, cyclohexyle, cyclopentyle, pyridinyle, pyridazinyle, -(CH₂)-benzo[b]furannyle, -0-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -O-benzyle,
et les radicaux cycloaliphatiques susmentionnés peuvent présenter, le cas échéant, à chaque fois, 1, 2, 3, 4 ou 5 hétéroatome(s) comme élément(s) de cycle, qui est/sont choisi(s) indépendamment les uns des autres dans le groupe constitué par oxygène, azote et soufre ;
le cas échéant, à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

2. Composés selon la revendication 1, **caractérisés en ce que** n vaut 1.

3. Composés selon la revendication 1 ou 2, **caractérisés en ce que** Y représente O, O-C(=O), 0-C(=O)-O, O-S (=O) ₂, O-C (=S) -N (H) ou O-C (=O) -N (H) .

4. Composés selon l'une ou plusieurs des revendications 1 à 3, **caractérisés en ce que**
R¹, R ² , R³ et R⁴ représentent, indépendamment les uns des autres, à chaque fois
H, F, Cl, Br, I, -SF₅, -CN, -NC, -NO₂, -SO₃H, -NH₂, -OH, -SH, -OR¹⁰, -SR¹¹, -NR¹²R¹³, -NH-R¹⁴, -NH-C(=O)-R¹⁵, -C(=O)-OH, -C(=O)-OR²², -O-C(=O)-R²³ ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ; le radical pouvant, le cas échéant, à chaque fois, être substitué par 1, 2, 3, 4 ou 5 substituants choisis indépendamment les uns des autres dans le groupe constitué par F, Cl, Br, I, -CN, -NO₂, -OH, -SH et -NH₂.

5. Composés selon l'une ou plusieurs des revendications 1 à 4, **caractérisés en ce que**
R⁵ représente un groupe -C (=O) -R²⁴,
représente un groupe -S (=O) ₂-R²⁵ ;
représente un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ; le radical pouvant, le cas échéant, à chaque fois, être substitué par 1, 2, 3, 4 ou 5 substituants choisis indépendamment les uns des autres dans le groupe constitué par F, Cl, Br, I, -CN, -NO₂, -OH, -SH et -NH₂ ;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle et thiomorpholinyle ; le radical pouvant, le cas échéant, à chaque fois, être substitué par 1, 2, 3, 4 ou 5 substituants choisis indépendamment les uns des autres dans le groupe constitué par oxo (=O) , thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃) ₃, -O-C(=O)-CH₃, -O-C(=O)-C₂H₅, -OC(=O)-C(CH₃)₃, -N(CH₃)₂, -N(C₂H₅) ₂, -NH-CH₃, -NH-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃ et -S(=O)₂-C₂H₅ ;
ou représente un radical choisi dans le groupe constitué par phényle, naphtyle, (1,3)-benzodioxolyle, (1,4)-benzodioxanyle, thiophényle, furannyle, pyrrolyle, pyrazolyle, pyrannyle, pyridinyle, imidazolyle, indolyle, iso-indolyle, benzo[b]furannyle, benzo[b]thiophényle, thiazolyle, oxazolyle, isoxazolyle, pyridazinyle, pyrazinyle, pyrimidinyle, indazolyle, quinazolinyle, quinoléinyle, isoquinoléinyle, benzimidazolinyle, benzoxazolyle, benzisoxazolyle et benzothiazolyle, le radical pouvant être lié via un groupe - (CH₂) -, - (CH₂) - (CH₂) - ou -(CH₂)-(CH₂)-(CH₂)- et le cas échéant, à chaque fois, être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃) ₃, -O-C(=O)-CH₃, -O-C(=O)-C₂H₅, -0-C(=O)-C(CH₃)₃, -N(CH₃)₂, -N(C₂H₅)₂, -NH-CH₃, -NH-C₂H₅, -NH-C(=O)-O-CH₃, -NH-C(=O) -O-C₂H₅, -NH-C(=O)-OC(CH₃)₃, -C(=O)-H, -C(=O)-CH₃, -C(=O)-C₂H₅, -C(=O) - C(CH₃)₃, -C(=O)-NH₂, -C(=O) -NH-CH₃, -C(=O) -NH-C₂H₅, -C(=O)-N-(CH₃)₂, -C(=O)-N-(C₂H₅)₂, -S(=O)₂-CH₃. -S(=O)₂-C₂H₅, -S(=O)₂-phényle, -NH-S(=O)₂-CH₃, -NH-S(=O)₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furannyle, -O-phényle, -0-benzyle, phényle et benzyle ; la partie cyclique des radicaux pyridinyle, cyclopentyle, cyclohexyle, pyridazinyle, -S(=O)₂-phényle, -O-phényle, -0-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -0-CH₃, -O-C₂H₅, -O-CF₃, -S-CF₃, phényle et -O-benzyle.

6. Composés selon l'une ou plusieurs des revendications 1 à 5, **caractérisés en ce que**
R⁶ représente un radical hydrogène ; représente - (CH₂) ₚZ-R²⁶ ou représente - (CH₂) -OR²⁷.

7. Composés selon l'une ou plusieurs des revendications 1 à 6, **caractérisés en ce que**
R⁷, R⁸ et R⁹ représentent, indépendamment les uns des autres, à chaque fois
un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ; le radical pouvant, le cas échéant, à chaque fois, être substitué par 1, 2, 3, 4 ou 5 substituants choisis indépendamment les uns des autres dans le groupe constitué par F, Cl, Br, I, -CN, -NO₂, -OH, -SH et -NH₂ ;
ou un radical choisi dans le groupe constitué par phényle, naphtyle, thiophényle, furannyle, pyridinyle, thiazolyle et oxazolyle, le radical pouvant, le cas échéant, à chaque fois, être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃) ₃, -O-C(=O) -CH₃, -O-C(=O)-C₂H₅, -OC(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C(=O) - C(CH₃) ₃ et lié via un groupe -(CH₂)-, -(CH₂)-(CH₂)-ou - (CH₂) - (CH₂) - (CH₂) - .

8. Composés selon l'une ou plusieurs des revendications 1 à 7, **caractérisés en ce que**
R¹⁰, R¹¹, R¹², R¹³, R¹⁴, R¹⁵, R¹⁶, R¹⁷, R¹⁸, R¹⁹, R²⁰, R²¹, R²² et R²³ représentent, indépendamment les uns des autres, à chaque fois
un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ; le radical pouvant, le cas échéant, à chaque fois, être substitué par 1, 2, 3, 4 ou 5 substituants choisis indépendamment les uns des autres dans le groupe constitué par F, Cl, Br, I, -CN, -NO₂, -OH, -SH et -NH₂ ;
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle et thiomorpholinyle ;
ou un radical choisi dans le groupe constitué par phényle, naphtyle, (1,3)-benzodioxolyle, (1,4)-benzodioxanyle, thiophényle, furannyle, pyrrolyle, pyrazolyle, pyrannyle, pyridinyle, imidazolyle, indolyle, iso-indolyle, benzo[b]furannyle, benzo[b]thiophényle, thiazolyle, oxazolyle, isoxazolyle, pyridazinyle, pyrazinyle, pyrimidinyle, indazolyle, quinazolinyle, quinoléinyle, isoquinoléinyle, benzimidazolinyle, benzoxazolyle, benzisoxazolyle et benzothiazolyle, le radical pouvant être lié via un groupe - (CH₂) -, -(CH₂)-(CH₂)- ou - (CH₂) - (CH₂) - (CH₂) - et pouvant, le cas échéant, à chaque fois, être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -OC(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃) ₃, -C(=O)-NH₂, -C(=O)-NH-CH₃, -C(=O)-NH-C₂H₅, -C(=O)-N-(CH₃)₂, -C(=O)-N-(C₂H₅)₂.

9. Composés selon l'une ou plusieurs des revendications 1 à 8, **caractérisés en ce que**
R²⁴, R²⁵, R²⁸ et R²⁹ représentent, indépendamment les uns des autres, à chaque fois
un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle et thiomorpholinyle ;
ou un radical choisi dans le groupe constitué par phényle, naphtyle, (1,3)-benzodioxolyle, (1,4)-benzodioxanyle, thiophényle, furannyle, pyrrolyle, pyrazolyle, pyrannyle, pyridinyle, imidazolyle, indolyle, iso-indolyle, benzo[b]furannyle, benzo[b]thiophényle, thiazolyle, oxazolyle, isoxazolyle, pyridazinyle, pyrazinyle, pyrimidinyle, indazolyle, quinazolinyle, quinoléinyle, isoquinoléinyle, benzimidazolinyle, benzoxazolyle, benzisoxazolyle et benzothiazolyle, le radical pouvant, le cas échéant, à chaque fois, être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -OC(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C (=O) - C(CH₃)₃, -C(=O)-NH₂, -C(=O)-NH-CH₃, -C(=O)-NH-C₂H₅, -C(=O) -N-(CH₃)₂, -C(=O)-N-(C₂H₅)₂.

10. Composés selon l'une ou plusieurs des revendications 1 à 9, **caractérisés en ce que**
R²⁶ représente un groupe -C(=O) -R²⁸ ;
représente un groupe -S(=O)₂-R²⁹ ;
représente un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ; le radical pouvant, le cas échéant, à chaque fois, être substitué par 1, 2, 3, 4 ou 5 substituants choisis indépendamment les uns des autres dans le groupe constitué par F, Cl, Br, I, -CN, -NO₂, -OH, -SH et -NH₂ ;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle et thiomorpholinyle ; le radical pouvant, le cas échéant, à chaque fois, être substitué par 1, 2, 3, 4 ou 5 substituants choisis indépendamment les uns des autres dans le groupe constitué par oxo (=O) , thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -0-C(=O)-C(CH₃) ₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-OC(CH₃)₃, -C(=O)-H. -C(=O)-CH₃, -C(=O)-C₂H₅, -C(=O) C(CH₃)₃, -C(=O)-NH₂, -C(=O)-NH-CH₃, -C(=O)-NH-C₂H₅, -C(=O)-N-(CH₃), -C(=O)-N-(C₂H₅)₂, -S(=O)₂-CH₃ et -S(=O)₂-C₂H₅ ;
ou représente un radical choisi dans le groupe constitué par phényle, naphtyle, (1,3)-benzodioxolyle, (1,4)-benzodioxanyle, thiophényle, furannyle, pyrrolyle, pyrazolyle, pyrannyle, pyridinyle, imidazolyle, indolyle, iso-indolyle, benzo[b]furannyle, benzo[b]thiophényle, thiazolyle, oxazolyle, isoxazolyle, pyridazinyle, pyrazinyle, pyrimidinyle, indazolyle, quinazolinyle, quinoléinyle, isoquinoléinyle, benzimidazolinyle, benzoxazolyle, benzisoxazolyle et benzothiazolyle, le radical pouvant être lié via un groupe -(CH₂)-, -(CH₂)-(CH₂)- ou -(CH₂)-(CH₂)-(CH₂)- et le cas échéant, à chaque fois, être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O) -O-CH₃, -C(=O)-O-C₂H₅, -C(=O)-O-C(CH₃)₃, -O-C(=O)-CH₃, -O-C(=O)-C₂H₅, -OC(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O -OC(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃. -S(=O)₂-C₂H₅, -S(=O)₂-phényle, -NH-S(=O)₂-CH₃, -NH-S(=O) ₂-C₂H₅, -S(=O)₂-NH-CH₃, -S(=O)₂-NH-C₂H₅, cyclohexyle, cyclopentyle, pyridinyle, pyridazinyle, -(CH₂)-benzo[b]furannyle, -O-phényle, -0-benzyle, phényle et benzyle ; la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -0-CH₃, -O-C₂H₅, -O-CF₃, -S-CF₃, phényle et -O-benzyle.

11. Composés selon l'une ou plusieurs des revendications 1 à 10, **caractérisés en ce que**
n vaut 1 ;
X représente CH₂, O, S, S(=O), S(=O)₂, N (H) , N (R⁷) , N[C(=O)-R⁸] ou N[C(=O)-O-R⁹] ;
Y représente O, O-C(=O), O-C(=O)-O, O-S(=O)₂, O- C(=S)-N(H) ou O-C(=O)-N(H) ;
R¹, R², R³ et R⁴ représentent, indépendamment les uns des autres, à chaque fois
H, F, Cl, Br, I, -SF₅, -CN, -NC, -NO₂, -OH, -SH, -OR¹⁰ -SR¹¹, -NR¹²R¹³,
ou un radical choisi dans le groupe constitué par méthyle, -CF₃, -CHF₂, -CH₂F, -CCl₃, -CBr₃, -CH₂-CN, -CH₂-NO₂, éthyle, -CF₂-CF₃, -CH₂-CF₃, -CCl₂-CCl₃, -CF₂-CH₃, -CH₂-CH₂-CN, -CH₂-CH₂-NO₂, n-propyle, -CF₂- CF₂-CF₃, -CH₂-CH₂-CH₂-CN, -CH₂-CH₂-CH₂-NO₂, isopropyle, n-butyle, sec-butyle, isobutyle, tert- butyle, (1,1)-diméthylpropyle, n-pentyle, sec- pentyle, n-hexyle et n-heptyle ;
R⁵ représente un groupe -C(=O)-R²⁴,
représente un groupe -S(=O)₂-R²⁵;
représente un radical choisi dans le groupe constitué par méthyle, éthyle, n-propyle, isopropyle, n-butyle, sec-butyle, isobutyle, tert- butyle, (1,1)-diméthylpropyle, n-pentyle, sec- pentyle, n-hexyle et n-heptyle ;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle et thiomorpholinyle ;
ou représente un radical choisi dans le groupe constitué par phényle, benzyle, phénéthyle, naphtyle, (1,3)-benzodioxolyle, (1,4)- benzodioxanyle, thiophényle, furannyle, pyrrolyle, pyrazolyle, pyrannyle, pyridinyle, imidazolyle, indolyle, iso-indolyle, benzo[b]furannyle, benzo[b]thiophényle, thiazolyle, oxazolyle, isoxazolyle, pyridazinyle, pyrazinyle, pyrimidinyle, indazolyle, quinazolinyle, quinoléinyle, isoquinoléinyle, benzimidazolinyle, benzoxazolyle, benzisoxazolyle et benzothiazolyle, le radical pouvant être lié via un groupe -(CH₂)-, -(CH₂)-(CH₂)- ou -(CH₂)-(CH₂)-(CH₂)- et être, le cas échéant, à chaque fois substitué, par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec- butyle, isobutyle, tert-butyle, -C(=O)-OH, -C(=O)- O-CH₃, -C(=O)-O-C₂H₅, -C(=O)-O-C(CH₃) ₃, -O-C(=O)- CH₃, -O-C(=O) -C₂H₅, -O-C(=O)-C(CH₃) ₃, -N(CH₃)₂, -N(C₂H₅)₂, -NH-CH₃, -NH-C₂H₅, -NH-C(=O) -O-CH₃, -NH- C(=O)-O-C₂H₅, -NH-C(=O)-O-C(CH₃)₃, -C(=O)-H, -C(=O)- CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅) ₂, -S(=O)₂-CH₃, -S(=O)₂-C₂H₅, -NH- S(=O)₂-CH₃, -NH-S(=O)₂-C₂H₅, -S(=O)₂-NH-CH₃ et -S(=O)₂-NH-C₂H₅ ;
R⁶ représente un radical hydrogène ;
représente -(CH₂)-Z-R²⁶;
ou représente - (CH₂) -OR²⁷ ;
R⁷, R⁸ et R⁹ représentent, indépendamment les uns des autres, à chaque fois
un radical choisi dans le groupe constitué par méthyle, éthyle, n-propyle, isopropyle, n-butyle, sec-butyle, isobutyle, tert-butyle, (1,1)- diméthylpropyle, n-pentyle, sec-pentyle, n-hexyle et n-heptyle ;
ou un radical choisi dans le groupe constitué par benzyle et phénéthyle, le radical pouvant, le cas échéant, à chaque fois, être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec- butyle, isobutyle et tert-butyle ;
R¹⁰, R¹¹, R¹² et R¹³ représentent, indépendamment les uns des autres, à chaque fois
un radical choisi dans le groupe constitué par méthyle, -CF₃, -CHF₂, -CH₂F, -CCl₃, -CBr₃, -CH₂-CN, -CH₂-NO₂, éthyle, -CF₂-CF₃, -CH₂-CF₃, -CCl₂-CCl₃, -CF₂-CH₃, -CH₂-CH₂-CN, -CH₂-CH₂-NO₂, n-propyle, isopropyle, n-butyle, sec-butyle, isobutyle, tert- butyle, (1,1)-diméthylpropyle, n-pentyle, sec- pentyle, n-hexyle et n-heptyle ;
ou un radical choisi dans le groupe constitué par phényle, benzyle et phénéthyle, le radical pouvant, le cas échéant, à chaque fois, être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S- CH₃, -S-C₂H₅, méthyle, éthyle, n-propyle, isopropyle, n-butyle, sec-butyle, isobutyle et tert-butyle ;
Z représente O, O-C(=O), O-C(=O)-O, O-S(=O)2, O- C (=S) -N (H) ou O-C (=O) -N (H) ;
R²⁴ et R²⁵ représentent, indépendamment l'un de l'autre, à chaque fois
un radical choisi dans le groupe constitué par phényle, naphtyle, thiophényle, furannyle et pyridinyle, le radical pouvant, le cas échéant, à chaque fois, être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec- butyle, isobutyle, tert-butyle, -N(CH₃)₂, -N(C₂H₅)₂, -NH-CH₃ et -NH-C₂H₅ ;
R²⁶ représente un groupe -C (=O) -R²⁸ ;
représente un groupe -S(=O)₂-R²⁹ ;
représente un radical choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle et thiomorpholinyle ;
représente un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ;
ou représente un radical choisi dans le groupe constitué par phényle, benzyle, phénéthyle, naphtyle, (1,3)-benzodioxolyle, (1,4)-benzodioxanyle, thiophényle, furannyle, pyrrolyle, pyrazolyle, pyrannyle, pyridinyle, imidazolyle, indolyle, iso-indolyle, benzo[b]furannyle, benzo[b]thiophényle, thiazolyle, oxazolyle, isoxazolyle, pyridazinyle, pyrazinyle, pyrimidinyle, indazolyle, quinazolinyle, quinoléinyle, isoquinoléinyle, benzimidazolinyle, benzoxazolyle, benzisoxazolyle et benzothiazolyle, le radical pouvant être lié via un groupe -(CH₂)-, -(CH₂)-(CH₂)- ou -(CH₂)-(CH₂)-(CH₂)- et être substitué, le cas échéant, à chaque fois, par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O) -O-C₂H₅, -C(=O)-O-C(CH₃)₃, -OC(=O)-CH₃, -O-C(=O)-C₂H₅, -O-C(=O)-C(CH₃)₃, -N (CH₃) ₂, -N(C_{2H5})₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃, -C(=O) -NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -NH-S(=O)₂-CH₃, -NH-S(=O)₂-C₂H₅, -S(=O)2-NH-CH₃ et -S(=O)₂-NH-C₂H₅;
R²⁷ représente un radical hydrogène ;
et
R²⁸ et R²⁹ représentent, indépendamment l'un de l'autre, à chaque fois
un radical choisi dans le groupe constitué par phényle, naphtyle, thiophényle, furannyle et pyridinyle, le radical pouvant, le cas échéant, à chaque fois, être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec- butyle, isobutyle, tert-butyle, -N(CH₃)₂, -N(C₂H₅)₂, -NH-CH₃ et -NH-C₂H₅ ;
le cas échéant, à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

12. Composés selon l'une ou plusieurs des revendications 1 à 11, **caractérisés en ce que**
n vaut 1 ;
X représente CH₂, O, S, S(=O) ou S(=O) ₂ ;
Y représente O, O-C(=O), O-C(=O)-O, O-S(=O)₂, O- C(=S)-N(H) ou O-C(=O)-N(H) ;
R¹, R², R³ et R⁴ représentent, indépendamment les uns des autres, à chaque fois
H, F, Cl, Br, -SF₅, -OH, -OR¹⁰, -SR¹¹, -NR¹²R¹³, ou un radical choisi dans le groupe constitué par méthyle, -CF₃, -CHF₂, -CH₂F, -CCl₃, -CBr₃, -CH₂-CN, -CH₂-NO₂, éthyle, -CF₂-CF₃, -CH₂-CF₃, n-propyle, isopropyle, n-butyle, sec-butyle, isobutyle, tert- butyle, (1,1)-diméthylpropyle et n-pentyle ;
R⁵ représente un groupe -C (=O) -R²⁴,
représente un groupe -S(=O)₂-R²⁵ ;
représente un radical choisi dans le groupe constitué par méthyle, éthyle, n-propyle, isopropyle, n-butyle, sec-butyle, isobutyle, tert- butyle, (1, 1) -diméthylpropyle, n-pentyle, sec- pentyle, n-hexyle et n-heptyle ;
représente un radical choisi dans le groupe constitué par cyclopropyle, cyclobutyle, cyclopentyle, cyclohexyle, cycloheptyle, cyclopentényle, cyclohexényle et cycloheptényle ; représente un radical choisi dans le groupe constitué par phényle, naphtyle, thiophényle, furannyle, pyridinyle, indolyle, thiazolyle et oxazolyle, le radical pouvant, le cas échéant, à chaque fois, être substitué par 1, 2, 3, 4 ou 5 substituants choisis indépendamment les uns des autres dans le groupe constitué par F, Cl, Br, -SF₅, -CF₃, -O-CH₃, -O-C₂H₅, -SCH₃, -SC₂H₅, -O-CF₃, -S-CF₃, méthyle, éthyle, n-propyle, isopropyle, n- butyle, sec-butyle, isobutyle et tert-butyle ;
R⁶ représente un radical hydrogène ;
représente - (CH₂)-Z-R²⁶ ;
ou représente - (CH₂)-OR²⁷ ;
R¹⁰, R¹¹, R¹² et R¹³ représentent, indépendamment les uns des autres, à chaque fois un radical choisi dans le groupe constitué par méthyle, -CF₃, -CH₂F, -CF₂H, éthyle, -C₂F₅, n- propyle, isopropyle, n-butyle, sec-butyle, isobutyle, tert-butyle, (1, 1) -diméthylpropyle, n- pentyle, sec-pentyle, n-hexyle et n-heptyle ;
Z représente O, O-C(=O), O-C(=O)-O, O-S(=O)₂, O- C(=S)-N(H) ou O-C(=O)-N(H) ;
R²⁴ et R²⁵ représentent, indépendamment l'un de l'autre, à chaque fois
un radical choisi dans le groupe constitué par phényle, naphtyle, thiophényle, furannyle et pyridinyle, le radical pouvant, le cas échéant, à chaque fois, être substitué par 1, 2, 3, 4 ou 5 substituants choisis indépendamment les uns des autres dans le groupe constitué par F, Cl, Br, I, -CF₃, -SF₅, -O-CH₃, -O-C₂H₅, -S-CF₃, méthyle, éthyle, n-propyle, isopropyle, n-butyle, sec- butyle, isobutyle et tert-butyle ;
R²⁶ représente un groupe -C(=O)-R²⁸ ;
représente un groupe -S(=O)₂-R²⁹ ;
représente un radical choisi dans le groupe constitué par méthyle, éthyle, n-propyle, isopropyle, n-butyle, sec-butyle, isobutyle, tert- butyle, (1,1)-diméthylpropyle, n-pentyle, sec- pentyle, n-hexyle et n-heptyle ; représente un radical choisi dans le groupe constitué par cyclopropyle, cyclobutyle, cyclopentyle, cyclohexyle, cycloheptyle, cyclopentényle, cyclohexényle et cycloheptényle ;
ou représente un radical choisi dans le groupe constitué par phényle, naphtyle, thiophényle, furannyle, pyridinyle, indolyle, thiazolyle et oxazolyle, le radical pouvant, le cas échéant, à chaque fois, être substitué par 1, 2, 3, 4 ou 5 substituants choisis indépendamment les uns des autres dans le groupe constitué par F, Cl, Br, -SF₅, -CF₃, -O-CH₃, -O-C₂H₅, -S-CH₃, -S-C₂H₅, -O-CF₃, -S-CF₃, méthyle, éthyle, n-propyle, isopropyle, n- butyle, sec-butyle, isobutyle et tert-butyle ;
R²⁷ représente un radical hydrogène ; et
R²⁸ et R²⁹ représentent, indépendamment l'un de l'autre, à chaque fois
un radical choisi dans le groupe constitué par phényle, naphtyle, thiophényle, furannyle et pyridinyle, le radical pouvant, le cas échéant, à chaque fois, être substitué par 1, 2, 3, 4 ou 5 substituants choisis indépendamment les uns des autres dans le groupe constitué par F, Cl, Br, I, -CF₃, -SF₅, -O-CH₃, -O-C₂H₅, -S-CF₃, méthyle, éthyle, n-propyle, isopropyle, n-butyle, sec-butyle, isobutyle et tert-butyle ;
le cas échéant, à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

13. Composés selon l'une ou plusieurs des revendications 1 à 12 choisis dans le groupe constitué par
1. l'ester 5-oxo-2,3,4,5-tétrahydrobenzo[b]thiépin-4- ylméthylique de l'acide pentylcarbamique
2. l'ester 4-(phénylcarbamoyloxyméthyl)-5-oxo- 2,3,4,5-tétrahydrobenzo[b]thiépin-4-ylméthylique de l'acide phénylcarbamique
3. l'ester 5-oxo-2,3,4,5-tétrahydrobenzo[b]thiépin-4- ylméthylique de l'acide phénylcarbamique
4. l'ester 5-oxo-2,3,4,5-tétrahydrobenzo[b]thiépin-4- ylméthylique de l'acide (3-trifluorométhylphényl)- carbamique
5. l'ester 5-oxo-2,3,4,5-tétrahydrobenzo[b]thiépin-4- ylméthylique de l'acide (4-bromophényl)-carbamique
6. l'ester 4-hydroxyméthyl-5-oxo-2,3,4,5- tétrahydrobenzo[b]thiépin-4-ylméthylique de l'acide cyclohexylcarbamique
7. l'ester 5-oxo-2,3,4,5-tétrahydrobenzo[b]thiépin-4- ylméthylique de l'acide cyclohexylcarbamique
8. l'ester 6-(cyclohexylcarbamoyloxyméthyl)-5-oxo- 6,7,8,9-tétrahydro-5H-benzocycloheptén-6- ylméthylique de l'acide cyclohexylcarbamique
9. l'ester 0-(4-hydroxyméthyl-5-oxo-2,3,4,5- tétrahydrobenzo[b]thiépin-4-ylméthylique) de l'acide phénylthiocarbamique
10. l'ester 5-oxo-2,3,4,5-tétrahydrobenzo[b]thiépin-4- ylméthylique de l'acide N-(4- méthylphénylsulfonyl)-carbamique
11. l'ester 0-(4-hydroxyméthyl-5-oxo-2,3,4,5- tétrahydrobenzo[b]thiépin-4-ylméthylique) de l'acide naphtalén-1-yl-thiocarbamique
12. l'ester 4-hydroxyméthyl-5-oxo-2,3,4,5- tétrahydrobenzo[b]oxépin-4-ylméthylique de l'acide pentylcarbamique
13. l'ester 4-hydroxyméthyl-5-oxo-2,3,4,5- tétrahydrobenzo[b]thiépin-4-ylméthylique de l'acide pentylcarbamique
14. l'ester 4-(pentylcarbamoyloxyméthyl)-5-oxo- 2,3,4,5-tétrahydrobenzo[b]thiépin-4-ylméthylique de l'acide pentylcarbamique
15. l'ester 0-(4-hydroxyméthyl-5-oxo-2,3,4,5- tétrahydrobenzo[b]oxépin-4-ylméthylique) de l'acide phénylthiocarbamique
16. l'ester 4-(2,4-difluorophénylcarbamoyloxyméthyl)- 5-oxo-2,3,4,5-tétrahydrobenzo[b]oxépin-4- ylméthylique de l'acide 2,4- difluorophénylcarbamique
17. l'ester O-(4-hydroxyméthyl-5-oxo-2,3,4,5- tétrahydrobenzo[b]thiépin-4-ylméthylique) de l'acide (3-trifluorométhylphényl)-thiocarbamique
18. l'ester 0-(4-hydroxyméthyl-5-oxo-2,3,4,5- tétrahydrobenzo[b]oxépin-4-ylméthylique) de l'acide (3-trifluorométhylphényl)-thiocarbamique
19. l'ester 4-benzoylcarbamoyloxyméthyl-5-oxo-2,3,4,5- tétrahydrobenzo[b]oxépin-4-ylméthylique de l'acide benzoylcarbamique
20. l'ester 4-hydroxyméthyl-5-oxo-2,3,4,5- tétrahydrobenzo[b]thiépin-4-ylméthylique de l'acide (2,4-difluorophényl)-carbamique
21. l'ester 0-(4-hydroxyméthyl-5-oxo-2,3,4,5- tétrahydrobenzo[b]thiépin-4-ylméthylique) de l'acide (2,4-difluorophényl)-thiocarbamique
22. l'ester O-(6-hydroxyméthyl-5-oxo-6,7,8,9- tétrahydro-5H-benzocycloheptén-6-ylméthylique) de l'acide (3-trifluorométhylphényl)-thiocarbamique
23. l'ester O-(4-hydroxyméthyl-5-oxo-2,3,4,5- tétrahydrobenzo[b]oxépin-4-ylméthylique) de l'acide (2,4-difluorophényl)-thiocarbamique
24. l'ester 0-(6-hydroxyméthyl-5-oxo-6,7,8,9- tétrahydro-5H-benzocycloheptén-6-ylméthylique) de l'acide (2,4-difluorophényl)-thiocarbamique
25. l'ester 4-(3-trifluorométhylphényl)- carbamoyloxyméthyl-5-oxo-2,3,4,5- tétrahydrobenzo[b]oxépin-4-ylméthylique de l'acide (3-trifluorométhylphényl)-carbamique
26. l'ester 4-hydroxyméthyl-5-oxo-2,3,4,5- tétrahydrobenzo[b]thiépin-4-ylméthylique de l'acide butylcarbamique
27. l'ester 4-(butylcarbamoyloxyméthyl)-5-oxo-2,3,4,5- tétrahydrobenzo[b]thiépin-4-ylméthylique de l'acide butylcarbamique
et
28. l'ester 4-hydroxyméthyl-5-oxo-2,3,4,5- tétrahydrobenzo[b]thiépin-4-ylméthylique de l'acide (4-trifluorométhoxyphényl)-carbamique ;
le cas échéant, à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

14. Médicament contenant au moins un composé selon l'une ou plusieurs des revendications 1 à 13 et le cas échéant un ou plusieurs adjuvants physiologiquement acceptables.

15. Médicament selon la revendication 14 pour la prophylaxie et/ou le traitement de la douleur, de préférence de la douleur choisie dans le groupe constitué par la douleur aiguë, la douleur chronique, la douleur neuropathique et la douleur viscérale.

16. Médicament selon la revendication 14 destiné à la prophylaxie et/ou au traitement d'une ou de plusieurs maladies choisies dans le groupe constitué par la migraine ; les dépressions ; l'incontinence urinaire ; l'incontinence de stress ; une vessie hyperactive (overactive bladder, OAB) ; la toux ; l'ostéoporose ; les maladies de neurodégénérescence, de préférence choisies dans le groupe constitué par la maladie de Parkinson, de Huntington, d'Alzheimer et la sclérose en plaques ; les troubles de l'alimentation, de préférence choisis dans le groupe constitué par la boulimie, l'anorexie, l'obésité et la cachexie ; les états anxieux ; les dysfonctionnements cognitifs, de préférence les troubles de la mémoire ; les états déficients cognitifs (trouble du déficit de l'attention - attention deficit syndrom - ADS) ; l'épilepsie ; la diarrhée et le prurit ; ou à la prophylaxie et/ou au traitement de l'abus d'alcool et/ou de drogues et/ou de médicaments et de la dépendance à l'alcool et/ou aux drogues et/ou à la nicotine et/ou aux médicaments, de préférence à la prophylaxie et/ou à la diminution des symptômes de sevrage en cas de dépendance à l'alcool et/ou aux drogues et/ou à la nicotine et/ou aux médicaments ; à la prophylaxie et/ou à la diminution d'un développement d'une tolérance aux médicaments, en particulier aux médicaments à base d'opioïdes ; à la régulation de la prise d'aliments ; à la modulation de l'activité mobile ; à la régulation du système cardiovasculaire ; à l'anesthésie locale ; à l'augmentation de la vigilance ; à l'augmentation de la libido ; à la diurèse et/ou à l'antinatriurèse.

17. Utilisation d'au moins un composé selon l'une ou plusieurs des revendications 1 à 13 pour la préparation d'un médicament destiné à la prophylaxie et/ou au traitement de la douleur, de préférence de la douleur choisie dans le groupe constitué par la douleur aiguë, la douleur chronique, la douleur neuropathique et la douleur viscérale.

18. Utilisation d'au moins un composé selon l'une ou plusieurs des revendications 1 à 13 pour la préparation d'un médicament destiné à la prophylaxie et/ou au traitement d'une ou de plusieurs maladies choisies dans le groupe constitué par la migraine ; les dépressions ; l'incontinence urinaire ; l'incontinence de stress ; une vessie hyperactive (overactive bladder, OAB) ; la toux ; l'ostéoporose ; les maladies de neurodégénérescence, de préférence choisies dans le groupe constitué par la maladie de Parkinson, de Huntington, d'Alzheimer et la sclérose en plaques ; les troubles de l'alimentation, de préférence choisis dans le groupe constitué par la boulimie, l'anorexie, l'obésité et la cachexie ; les états anxieux ; les dysfonctionnements cognitifs, de préférence les troubles de la mémoire ; les états déficients cognitifs (trouble du déficit de l'attention - attention deficit syndrom - ADS) ; l'épilepsie ; la diarrhée et le prurit ; ou à la prophylaxie et/ou au traitement de l'abus d'alcool et/ou de drogues et/ou de médicaments et de la dépendance à l'alcool et/ou aux drogues et/ou à la nicotine et/ou aux médicaments, de préférence à la prophylaxie et/ou à la diminution des symptômes de sevrage en cas de dépendance à l'alcool et/ou aux drogues et/ou à la nicotine et/ou aux médicaments ; à la prophylaxie et/ou à la diminution d'un développement d'une tolérance aux médicaments, en particulier aux médicaments à base d'opioïdes ; à la régulation de la prise d'aliments ; à la modulation de l'activité mobile ; à la régulation du système cardiovasculaire ; à l'anesthésie locale ; à l'augmentation de la vigilance ; à l'augmentation de la libido ; à la diurèse et/ou à l'antinatriurèse.
